# EUROPÄISCHE PATENTSCHRIFT

(11) **EP 1 185 869 B1**
(45) Veröffentlichungstag und Bekanntmachung des Hinweises auf die Patenterteilung: **26.07.2006**
(21) Anmeldenummer: 00949060.8
(22) Anmeldetag: 26.05.2000
(51) Int. Cl.: G01N 33/553, G01N 33/543

(54) **LIGAND-ANKER-KONJUGATE ZUR GENERIERUNG EINER BIOSENSOR-SCHICHT**
LIGAND-ANCHOR CONJUGATES FOR PRODUCING A BIOSENSOR LAYER
CONJUGAISONS DES LIGANDS ET DES ANCRES POUR PRODUIRE UNE COUCHE BIOACTIVE POUR UN DETECTEUR

(30) Priorität: 28.05.1999 DE 19924606
(43) Veröffentlichungstag der Anmeldung: 13.03.2002
(73) Patentinhaber: Graffinity Pharmaceuticals Aktiengesellschaft, 69120 Heidelberg (DE)
(72) Erfinder: EICHLER, Jutta, D-69221 Dossenheim (DE); FRANK, Michael, D-69117 Heidelberg (DE); OTTLEBEN, Holger, D-69120 Heidelberg (DE); RAU, Harald, D-69221 Dossenheim (DE); SEKUL, Renate, D-68526 Ladenburg (DE); VETTER, Dirk, D-69120 Heidelberg (DE)
(74) Vertreter: Vossius & Partner
(86) Internationale Anmeldenummer: PCT/DE2000/001705
(87) Internationale Veröffentlichungsnummer: WO 2000/073796

(56) Entgegenhaltungen:
- EP-A- 0 574 000
- EP-A- 0 872 735
- WO-A-92/10757
- WO-A-98/31839
- US-A- 5 514 501
- US-A- 6 127 129
- HUISMAN B -H ET AL: "Self-Assembled Monolayers of Calix[4]arene derivatives on Gold" TETRAHEDRON LETTERS,NL,ELSEVIER SCIENCE PUBLISHERS, AMSTERDAM, Bd. 36, Nr. 18, 1. Mai 1995 (1995-05-01), Seiten 3273-3276, XP004028245 ISSN: 0040-4039
- J SPINKE, M LILEY, F-J SCHMITT, H-J GUDER, L ANGERMAIER, W KNOLL: "Molecular recognition at self-assembled monolayers: Optimization of surface functionalization" JOURNAL OF CHEMICAL PHYSICS, Bd. 99, Nr. 9, 1. November 1993 (1993-11-01), Seiten 7012-7019, XP000972391
- BROCKMAN ET AL: "A Multistep Chemical Modification Procedure To Create DNA Arrays on Gold Surfaces for the Study of Protein-DNA Interactions with Surface Plasmon Resonance Imaging" JOURNAL OF THE AMERICAN CHEMICAL SOCIETY,AMERICAN CHEMICAL SOCIETY, WASHINGTON, DC,US, Bd. 121, Nr. 35, 1999, Seiten 8044-8051, XP002148625 ISSN: 0002-7863

## Beschreibung

Die vorliegende Erfindung betrifft neuartige Ligand-Anker-Konjugate (LAK) die eine hochspezifische Wechselwirkung mit geeigneten Interaktionspartnern erlauben. Weiterhin betrifft die Erfindung Verfahren zur Synthese der Ligand-Anker-Konjugate. Derartige Ligand-Anker-Konjugate werden erfindungsgemäß eingesetzt zur Bereitstellung von Sensoren zur Detektion molekularer Interaktionen zwischen immobilisierten Liganden und nicht immobilisierten Interaktionspartnern (Rezeptoren).

Die Modifizierung organischer oder anorganischer Oberflächen kann nicht nur zur Aufreinigung von Biomolekülen (z.B. die Adsorption von Nukleinsäuren an Träger, wie von Qiagen, Hilden, Deutschland in WO 95/01359 offenbart, oder die Vernetzung einer Dextranpolymennatrix für die Affinitätschromatographie oder. Gelfiltration, Sephadex® von Pharmacia, Uppsala, Schweden) eingesetzt werden, sondern auch für die biomolekulare Interaktionsanalyse.

Biomolekulare Interaktionen werden in bekannten Verfahren der Interaktionsanalyse an Rezeptor-Ligandsystemen studiert, wobei der Rezeptor gewöhnlich ein Biomakromolekül (z.B. ein Protein oder eine Einzelstrang-DNA) ist und der Ligand eine "Sonde", ein Molekül meist geringeren Molekulargewichts biologischer oder synthetischer Herkunft darstellt (Peptide, Oligonukleodde oder sog. kleine organische Moleküle). Solche Liganden weisen hochspezifische Strukturmerkmale auf, die bei Vorliegen korrespondierender Strukturen am Rezeptor mit diesem in Wechselwirkung treten können. Die Anbindung an den Rezeptor kann durch einen oder mehrere Liganden erfolgen. Die Interaktionsanalyse wird in der pharmazeutischen und agrochemischen Industrie zur Wirkstoffsuche eingesetzt. Hierbei gilt es, eine möglichst große Anzahl an verschiedenen Proben in möglichst kurzem Zeitraum zu analysieren (High-Throughput Screening, HTS). Weiterhin findet die Interaktionsanalyse für das Genomstudium (Polymorphismen- (SNP) oder Expressionsmusteranalyse) oder auch für die Lebensmittelanalytik Verwendung.

Es ist von praktischem Vorteil, einen der Bindungspartner, Rezeptor oder Liganden, kovalent oder durch Adsorption an eine organische oder anorganische Oberfläche zu binden. Durch diese Generierung einer spezifischen Grenzschicht auf der Oberfläche (Immobilisierung von Liganden oder Rezeptor) erhält diese eine Bioaktivität. Die Immobilisierung eines Bindungspartners erleichtert die Prozeßführung, wie z.B. das Durchführen von Waschschritten und vermag in Verbindung mit einem geeigneten, meist optischen Detektionsverfahren (z.B. Fluoreszenzmessung) Auskunft über das Vorhandensein und die Stärke der Wechselwirkungen zwischen Rezeptor und Ligand auf molekularer Ebene zu geben.

Bioaktive Oberflächen lassen sich üblicherweise in mehreren Schritten erzeugen. Besondere Vorteile (physikochemische Stabilität, strukturelle Einheitlichkeit) bieten Filme aus organischen Monolagen (Bain und Whitesides, Angew. Chem. 101 (1989) 522-8; Zhong und Porter, Anal. Chem. (1995) 709A-715A). Hierbei werden in einem ersten Schritt Thiole auf Gold chenüsorbiert. Langkettige Alkylthiole packen sich als hochgeordnete Monoschicht (self assembled monolayer, SAM) auf die Festphase, wobei die Goldatome von den Schwefelfunktionen komplexiert werden. Solche SAMs sind aus der Literatur vorbekannt und mit vielerlei physikalischen Methoden gut charakterisiert worden. In Poirier und Pylant, Science, 272 (1996) 1145-8 finden sich "scanning tunnel"-mikroskopische Aufnahmen solcher Monolagen auf Gold.

Sind die SAM-Alkanketten endständig mit beispielsweise einer Hydroxygruppe versehen ("omega-funktionalisiert"), so lassen sich in nachfolgenden Reaktionen sog. hydrophile Spacer (z.B. Dextran) anknüpfen. Das Dextran wirkt als proteinadsorptionsresistentes Hydrogel und vermindert die unspezifische Bindung (passive Adsorption) der zu untersuchenden Biomoleküle. Die Modifikation (Carboxymethylierung) oder Oxidation des Dextrans führt zu statistisch verteilten Carboxyfunktionen, welche sich für Biokonjugationsreaktionen eignen; die Carboxylate werden anschließend durch Ausbildung sog. Aktivester chemisch aktiviert. In einem zweiten Schritt der auch als "Konjugationsschritt" bezeichnet wird, werden diese Aktivester mit einem eine primäre Aminofunktion enthaltenden Liganden oder Rezeptor kovalent verknüpft (Biacore®-Verfahren). Durch diesen letzten Schritt wird anschließend eine synthetische, bioaktive Oberfläche erhalten. Oberflächen, bei denen die Aktivierungs- und Könjugationsschritte nicht durchgeführt werden oder bei denen Konjugate erzeugt werden, von denen erwartet werden kann, daß sie keine Bioaktivität zeigen durch Verknüpfung mit sogenannten "Nichtliganden", dienen üblicherweise als wichtige sog. negative Kontrollen in den Bindungsstudien. Als Nichtligandew werden dabei gewöhnlich sehr kleine organischen Gruppen, z.B. Acetyl-, Methyl- oder Aminoethylreste aufgebracht.

Andere Verfahren zur Generierung bioaktiver Oberflächen nutzen folgende molekulare Schichtaufbauten:
- Silanisierung von Glas oder Silizium mit reaktiven Epoxid- oder Aminogruppen-haltigen Silanen, anschließende Acylierung der Aminogruppen, beispielsweise mit Nukleosidderivaten (Maskos und Southern, Nucl. Acids Res. 20 (1992) 1679-84).
- Passive Adsorption von Polylysin auf Glas, anschließende Deponierung von DNA durch nicht-kovalent elektrostatische Bindung (Schena et al., Science, 270 (1995) 467-70).
- Passive Adsorption von Protein auf Polystyrol (allgemein gebräuchliche ELISA-Technik).
- Passive Adsorption von Vesikeln oder Micellen an SAMs oder hydrophoben Silanschichten (Sackmann, Science, 271 (1996) 43-47).
- Passive Adsorption von gespriteten Lipiden auf Glas (Langmuir-Blodgett Technik).
- Passive Adsorption von Proteinen oder Peptiden auf Nitrocellulose oder anderen Membranmaterialien (allgemein gebräuchliche "dot blot" Technik).

Auf Grundlage dieser Methoden können relativ komplexe Mehrlagensysteme realisiert werden, z.B. kann ein Aminopolysiloxan auf Glas oder ein SAM auf Gold, biotinyliert werden, darauf läßt sich eine Avidinschicht aufziehen, welche wiederum biotinylierte Liganden oder Rezeptoren zu binden vermag (Müller et al., Science, 262 (1993) 1706-8). Ein anderes Beispiel ist die sog. "His-tag" oder Nickel-NTA-Oberfläche, welche durch Metallkomplexierung Liganden oder Rezeptoren bindet, die ein Histidin-Oligomermotiv tragen.

Alle oben beschriebenen Verfahren bergen den enormen Nachteil in sich, die bioaktive Komponente (Rezeptor od. Ligand) erst in einem abschließenden, späten Schritt innerhalb eines mehrstufigen Oberflächenmodifikationsprozesses einzubringen. Dadurch entsteht auf der Oberfläche ein schlecht charakterisierbarer, komplexer Aufbau, dessen Eigenschaften wie Belegungsdichte und Konstitution schwer zu erfassen sind. Ein großes Problem bei der Gestaltung bioaktiver Oberflächen liegt darin, genaue Kenntnis über die molekularen Bestandteile der Oberflächen zu erlangen. Ohne diese Information können gefundene Aktivitäten nur schlecht mit den dafür verantwortlichen chemischen oder biologischen Strukturen korreliert werden. Die detaillierte chemische Analyse von in mehrstufigen Verfahren modifizierten Oberflächen ist aufgrund der naturgemäß sehr geringen Substanzmengen äußerst schwierig. Bislang konnte nur gezeigt werden, daß die Anwesenheit von an die bioaktive Oberfläche nicht-kovalent gebundenen Rezeptoren mittels Laserdesoiptionsmassenspektrometrie nachweisbar ist (Nelson et al., Anal. Chem., 69 (1997) 4363-8). Zum vergleichenden Studium von Bindungsphänomenen an bioaktiven Grenzschichten ist eine genaue Kenntnis der diese Schicht konstituierenenden chemischen Substanzen unumgänglich, da bekannt ist, daß schon kleine strukturelle Unterschiede drastische Effekte auf die molekulare Wechselwirkung haben können. Die derzeit für die direkte physikochemische Charakterisierung von Monolagen zur Verfügung stehenden Techniken (z.B. XPS, FT-IR) sind nicht in der Lage, zur detaillierten Strukturaufklärung beizutragen. Der nicht-destruktiven Desorption und Analyse mit hochauflösenden Verfahren, wie MS, steht die hohe Beständigkeit der SAM- oder Silan-Filme entgegen.

Die derzeit bestcharakterisierten bioaktiven Schichten werden, wie vorstehend ausgeführt, erhalten, indem gelöste Alkanthiole mit einer Goldoberfläche kontaktiert werden. Die dabei gewonnenen selbst assemblierten Monoschichten (SAM) sind mit zahlreichen physikalischen Meßmethoden eingehend charakterisiert worden, und das Verständnis der strukturellen Eigenschaften dieser Oberflächen ist hoch. Das Aufbringen makromolekularer Schichten auf eine SAM, wie sie im Biacore®-Verfahren angewendet werden, macht diesen Vorteil jedoch wieder zunichte. Die Knüpfung neuer chemischer Bindungen an einer heterogenen, strukturell uneinheitlichen Dextranmatrix erfolgt gewissermaßen "blind" und kann nur durch indirekte Meßverfahren überprüft werden. Dies ist nicht nur für die Optimierung der Reaktionsparameter ungünstig, sondern auch unter dem Gesichtspunkt der Immobilisierung einer sehr großen Probenvielfalt von großem erheblichen Nachteil. Von einer "Kontrolle" der Oberflächenstrukturen auf molekularem Niveau kann hierbei nicht ausgegangen werden.

Bei dem vorgenannten Biacore® System (Biacore AB, Uppsala, Schweden) wird die bioaktive Oberfläche in Form eines MeBchips verwendet, der eine dünne Goldoberfläche mit einer darauf immobilisierten Einfachschicht (Monolage) organischer Moleküle enthält, an die wiederum eine organische Matrix, insbesondere eine Dextranmatrix, gekoppelt ist. Dieser Aufbau wird in WO 90/05303 beschrieben. Der Meßchip wird in der Regel in das Meßgerät eingelegt und anschließend "aktiviert", d.h. in die Matrix werden chemisch reaktive Gruppen eingefügt, die eine weitere Funktionalisierung der Oberfläche mit Hilfe von Liganden ermöglichen. Diese zu immobilisierenden Liganden werden anschließend mit der Oberfläche in Kontakt gebracht, was zu ihrer kovalenten Bindung an die Matrix führt. Dann werden überschüssige reaktive Gruppen mit einer anderen, meist niedermolekularen, Substanz abgesättigt, die nicht zur Interaktion mit den Testsubstanzen fähig sind. Erst jetzt ist der Meßchip prinzipiell für die Detektion von Substanzen, die mit dem auf der Oberfläche immobilisierten Liganden interagieren, vorbereitet. Die eigentliche Messung erfolgt dabei mittels Oberflächen-Plasmonen-Resonanz (Surface Plasmon Resonance, SPR).

Nachteilig bei diesem Verfahren ist jedoch die Tatsache, daß die Meßoberfläche, d.h. die Bindematrix, zunächst wie oben erläutert in einem oder mehreren Schritten aktiviert bzw. vorbereitet werden muß, wobei diese Schritte in einem Flußsystem innerhalb des Meßgerätes vorgenommen werden. Ferner bildet die verzweigte organische Matrix eine gelärtige Schicht über der Goldoberfläche und enthält nach der Vorbereitung der Bindematrix und der Immobilisierung die Liganden in statistischer Verteilung nicht nur an der Oberfläche der Matrix sondern auch in ihrem Inneren. Die Reaktionsbedingungen lassen sich während der Bereitstellung der Meßoberfläche nicht derart steuern, daß eine genau definierte Oberflächenstruktur gebildet wird. Somit werden häufig Diffusionseffekte des Analyten in die stark hydratisierte organische Matrix relevant so daß eine Diffusionslimitierung der Interaktion zwischen Analyt und immobilisiertem Liganden vorliegen kann. In solchen Fällen können keine verläßlichen Aussagen mehr über kinetische bzw. thermodynamische Konstanten der Interaktion mehr getroffen werden. In der Vergangenheit haben Schuck und Minton bereits auf dieses durch eine undefinierte Oberfläche erzeugte Problem hingewiesen (Schuck & Minton, Trends Biochem. Sci. (1996) 21 (12): 458-460).

Speziell bei der Immobilisierung von Lipiden auf der modifizierten Goldoberfläche, was z.B. häufig unter Zuhilfenahme micellärer Lösungen oder Lipidvesikel mit darin befindlichen Membranproteinen geschieht, sind die zusätzliche Belegung der Chipoberfläche (bzw. der Bindematrix) und die Schichtdicke der entstehenden Lipidschicht nicht mehr genau definiert. Dies liegt daran; daß eine genaue Kontrolle des Membranverschmelzungs- und Aufbauprozesses, wie z.B. bei der Ausspreitung einer Monoschicht unter Verwendung einer Filmwaage, nicht möglich ist, da ein Meßchip mit bereits immobilisierter Dextran- oder Lipidschicht und den eventuell daran gebundenen Biomolekülen vielen Messungen, wie z.B. genauen Bestimmung des Oberflächenbelegungsgrädes und der Schichtdicke, nicht mehr zugänglich ist. Da die auf SPR basierenden Verfahren in der Regel in Zyklen eingesetzt werden, d.h. es werden mehrere Meßreihen nacheinander auf derselben Meßoberfläche (bzw. demselben Meßchip) durchgeführt, treten auch Akkumulations- und. Abnutztingseffekte an der Oberfläche auf, was zusätzliche Schwierigkeiten bei der Messung und Auswertung verursacht.

EP-A-0 574 000 beschreibt ein Verfahren zur Herstellung einer Bindematrix, die ein Trägermaterial, z.B. Gold oder Silber, und einen daran gebundenen "Affinitätsträger" wie z.B. Biotin, enthält, der mit mindestens einem freien Reaktionspartner, z.B. Streptavidin oder Avidin, bindefähig ist. Dieser Affinitätsträger bildet eine durch nicht wechselwirkende Gruppen verdünnte und im wesentlichen lateral homogene Bindeschicht auf der Oberfläche des Trägermaterials. Das Trägermaterial wird dabei mit einer wässrigen Reaktionslösung inkubiert, die den über ein kurzkettiges Spacermolekül mit dem schichtbildenden Teil des Moleküls verknüpften Affinitätsträger und mindestens ein hydrophiles Verdünnungsmolekül enthält, wodurch sich auf dem Trägermaterial eine sogenannte "gemischte" selbst assemblierende Monoschicht ausbildet.

Der Affinitätsträger wirkt in dieser Bindematrix jedoch nicht selbst als Ligand oder Rezeptor sondern dient nur einem weiteren Schichtaufbau durch nicht-kovalente Bindung an Avidin oder Streptavidin, welches dann wiederum biotinylierte Liganden oder Rezeptoren zu binden vermag.

Nachteilig bei diesem Verfahren ist weiterhin die Tatsache, daß die Synthese der Konjugate aus dem Affinitätsträger und den Ankerverbindungen mittels derer er an die Oberfläche gebunden ist, in homogener Lösung in relativ großen Volumina erfolgt. Dazu müssen vergleichsweise große Mengen an Substanz eingesetzt werden, gleichzeitig werden aufwendige Arbeitsschritte wie Säulenchromatographie oder Extraktion erforderlich. Dies macht insbesondere den Einsatz dieses Verfahrens zur Erzeugung einer Vielzahl verschiedener Liganden, etwa bei Screening-Verfahren (HTS) bezüglich neuer Arzneistoffe etc., problematisch. Bei solchen Screening-Verfahren sind besonders miniaturisierbare Verfahren mit hohem Probendurchsatz interessant, die die parallele Messung von Interaktionen zwischen einer Vielzahl verschiedener Liganden mit einem oder mehreren interessierenden Biomolekülen erlauben. Häufig sind dabei chemische Modifikationen einer Ligandengrundstruktur notwendig bzw. wünschenswert, wie sie z.B. mit Methoden der kombinatorischen Chemie erzeugt werden können, um mit hoher Effizienz den Einfluß solcher Modifikationen auf Affinität oder Spezifität zu messen, etwa im Hinblick auf Bindung, Hemmung oder Aktivierung eines Enzyms. Insbesondere in diesem Zusammenhang ist das vorstehend genannte Verfahren jedoch ungeeignet.

Ein weiteres Beispiel für eine bekannte Bindematrix, die auch als Bindefilm bezeichnet wird, findet sich in der WO 92/10757. Hier wird ebenfalls ein durch Ankergruppen an ein Trägermaterial adsorbierter Affinitätsträger beschrieben.

US-A-5,514,501 beschreibt ein photochemisches Verfahren zu Strukturierung von selbstassemblierenden Monolagen auf Gold- oder Silbersubstraten.

EP-A-872 735 offenbart ein Verfahren zum Aufbringen von räumlich definierten Reagenzflächen auf eine Festphase in Form einer selbst-assemblierenden Monolage aufgebracht wird.

B.-H. Huisman et al. beschreiben in Tetrahedron Letters 36 (18), 3273-3276 (1995) selbstassemblierende Monolagen mit Calix[4]-Arenen als Kopfgruppen, die als Bausteine für den weiteren Aufbau von Erkennungsmotiven geeignet sind.

In WO 98/31839 wird die Immobilisierung von Nucleinsäuren an für Elektronentransfermessungen geeigneten Oberflächen beschrieben, wobei ein komplexierendes Agens verwendet wird.

Die Funktionsweise des "Biacore®" wurde vorstehend diskutiert und verwendet das für die Biowissenschaften bis vor kurzem wenig gebräuchlichen SPR-MeBprinzip, welches Schichtdickenänderungen an Oberflächen registriert und daher massensensitiv ist. SPR ermöglicht es, die Assoziation von Biomolekülen in Echtzeit, ohne chemische, radiochemische oder immunchemische Markierung und unter nur sehr geringem Substanzverbrauch zu beobachten.

Hierbei wird das an einer dünnen Goldschicht reflektierte Licht detektiert. Bei geeigneter Resonanzbedingung (Einfallswinkel und Wellenlänge des Lichtes und Schichtdicke der Göldschicht) nimmt die Intensität des reflektierten Lichtes ab. Die Lichtenergie wird dann in Ladungsdichtewellen des Elektronengases in der Goldschicht umgewandelt. Diese Ladungsdichtewellen nennt man Plasmonen. Um die Resonanz zu beobachten, benutzt man entweder monochromatisches Licht und zeichnet die Intensität des reflektierten Lichtes in Abhängigkeit des Einfallswinkels auf, oder man hält den Einfallswinkel konstant und variiert die Wellenlänge des Lichtes. Die Lage der Resonanz kann durch die Beschichtung auf der dem Lichteinfall abgewandten Seite der Goldschicht verändert werden. Der Rezeptor oder Ligand wird auf der Goldoberfläche immobilisiert. Nach der Zugabe des Ligandes oder Rezeptors wird, wenn diese Moleküle sich anlagern, die Resonanzbedingung geändert.

1989 brachte Pharmacia (Uppsala, Schweden) den ersten Biosensor auf den Markt, welcher auf der Messung von SPR beruhte.

Vorteile der SPR-MeBmethode liegen in der hohen Meßgenauigkeit der Bestimmung von Brechzahl und Schichtdicke dünner dielektrischer Schichten. Die SPR-Spektroskopie findet daher in den letzten Jahren verstärkt in der biochemischen Analytik Anwendung, da mit ihr die direkte Untersuchung der Wechselwirkung zwischen Biomolekülen möglich ist. Dazu wird ein Reaktionspartner (Ligand) auf der Carboxydextran-SAM-Goldoberfläche immobilisiert, der andere Reaktionspartner (Analyt, Rezeptor) wird in Lösung über die Sensoroberfläche geleitet. Die Wechselwirkung ist als Schichtdickenzuwachs direkt nachweisbar.

Nachdem sich die SPR-Meßmethode in vielen Bereichen als sehr schlagkräftig erwiesen hat und als etabliert gilt, sollten sich neue Anwendungsbereiche wie dem Hoch-Durchsatz-Screening (High-Throughput Screening = HTS) für SPR-Sensoren erschließen lassen.

Zu alternativen Biosensorverfahren, die ebenfalls ein "labeling", ein Markieren des Zielmoleküls mit Fluoreszenzfarbstoffen, hochaffinen Gruppen (Biotin) oder radioaktiven Elementen nicht erforderlich machen und sehr schonend im Umgang und sparsam im Verbrauch der oft sehr kostbaren Biomakromoleküle sind, zählen
- die Schwingquarz-Mikrowaage und
- die reflektometrische Interferenz Spektroskopie (RIFS).

Bei Biosensoren auf der Basis von Schwingquarz-Mikrowaagen wird die Bindung von Rezeptoren an Liganden mittels der die Schwingungsfrequenz des Schwingquarzes beeinflussenden Massenzunahme gemessen (Ebara und Okahata, JACS 116 (1994) 11209-12). Diese Meßmethode befindet sich noch in der Entwicklungsphase, kommerzielle Geräte sind nicht erhältlich, und die Verwendung für bioanalytische Fragestellungen ist noch wenig dokumentiert.

Mit Hilfe der reflektometrischen Interferenzmikröskopie kann die Teilreflexion von Licht an Phasenübergängen für die Detektion von Schichtdickenänderungen aüsgenutzt werden. Die Anlagerung von Biomolekülen an auf einer transparenten Oberfläche vorliegende Bindungspartner (Liganden) verursacht dabei eine Verschiebung eines Intensitätsprofils in Abhängigkeit der Wellenlänge. Die Verschiebung der ermittelten Kurven ist proportional der Schichtdickenänderung. Im RIFS können jedoch keine Gold/SAM Oberflächen eingesetzt werden.

Aufgabe der vorliegenden Erfindung ist es, wohldefinierte, SAMbildende Molekülstrukturen bereitzustellen. Auf Basis solcher Molekülstrukturen lassen sich Sensoren, insbesondere zum Einsatz im HTS, erzeugen. Zur Vermeidung der oben beschriebenen Nachteile des Standes der Technik sollen die für die Bioaktivität relevanten Strukturmotive (Liganden) in vorgelagerten Schritten mit einem SAM-bildenden Anker verknüpft werden und sind danach einer demnach vollständigen analytischen Charakterisierung zugänglich. Erst nach erfolgter, vollständiger Synthese werden diese Konjugate aus Liganden und Ankern (Ligand-Anker-Konjugate, LAK) auf einer geeigneten Oberfläche immobilisiert um so eine biospezifische Grenzschicht in Form einer Monoschicht bioaktiver LAK zu bilden. Zur Synthese der LAK haben sich bekannte Methoden der Festphasensynthese als vorteilhaft erwiesen. In solchen Verfahren wird die Zielstruktur ausgehend von einer festen Oberfläche aufgebaut. So können zunächst der Anker und nachfolgend der daran gebundene Ligand in mehreren Einzelschritten synthetisiert werden. Wahlweise kann ein vorgefertigter Ligand aber auch in einem einzigen Schritt mit dem Anker verknüpft werden. Die Anwendung solcher Syntheseverfahren erlaubt es, Ligand-Anker-Konjugate bereitzustellen, deren Struktur für ihren Einsatz in Form von SAMs in Screeningverfahren optimiert ist. Die Vorteile einer Verknüpfung von kombinatorischer oder hochparalleler Synthese und SAM-Oberflächen sind aus dem derzeit vorliegenden Stand der Technik nicht bekannt.

Als Liganden werden im Kontext der vorliegenden Erfindung allgemein Strukturelemente bezeichnet, die aufgrund ihrer strukturellen Eigenarten spezifische Wechselwirkungen mit Testsubstanzen oder ihren Untereinheiten eingehen können. Mit Hilfe der Liganden können z.B. im Rahmen von Screening-Verfahren Rezeptoren an die Meßoberfläche gebunden werden, die entsprechend kompatible Struktureinheiten aufweisen. Durch die Kenntnis der Struktur des Liganden lassen sich so unter anderem Rückschlüsse auf die Struktur des Rezeptors ziehen.

In der Literatur werden die Begriffe "Ligand" bzw. "Rezeptor" oft nicht einheitlich verwendet. Daher sollte betont werden, daß im Rahmen der vorliegenden Erfindung die Bezeichnung "Ligand" für solche Moleküle verwendet wird, die, vorzugsweise kovalent, endständig mit dem Anker verknüpft sind. Für die Zwecke der Interaktionsanalyse werden Liganden mit Hilfe solcher Anker auf den einzusetzenden Meßoberflächen immobilisiert und sorgen damit für die Biospezifität der entstehenden Grenzschicht. Als Beispiele für solche Liganden können Peptide, Oligonukleotide oder auch kleine organische Moleküle angeführt werden.

Als "Rezeptoren" werden Moleküle, vorzugsweise Biomoleküle, bezeichnet, die im Meßmedium vorliegen und deren Fähigkeit zur Wechselwirkung mit der vorgenannten Grenzschicht bzw. mit den darauf präsentierten Liganden der Interaktionsanalyse zugrunde liegt.

Eine Bindung der Rezeptormoleküle an die Liganden im Verlauf der Messung erfolgt bevorzugt aufgrund spezifischer korrespondierender sterischer oder elektronischer Strukturen in beiden Molekülen. Dafür kommen z.B. ionische bzw. polare, van der Waals oder weitere hydrophobe Wechselwirkungen oder Wasserstoffbrückenbindungen in Frage. Eine kovalente Bindung des Rezeptors an den Liganden ist aufgrund der im allgemeinen erheblichen Aktivierungsenergie und der damit verbundenen geringen Spezifität eher nachteilig.

Die Liganden werden im Rahmen der Erfindung mit Hilfe von Ankern auf der. Meßoberfläche des Sensors immobilisiert. Ein Ankermolekül im Sinne der Erfindung umfaßt damit zumindest zwei funktionelle Einheiten, die an entgegengesetzten Enden des Ankers vorliegen, und die zum einen die Verknüpfung mit der Meßoberfläche, zum anderen die Anbindung des Liganden ermöglichen. Zusätzlich sollte bei Anwendung der Festphasensynthese die Möglichkeit bestehen, Grundbausteine des Ankers mit dem zur Synthese verwendeten Feststoff zu verknüpfen und diese Verbindung nach erfolgreicher Synthese des LAK schonend wieder zu lösen. Als schonend können Verfahren gelten, die das LAK in seinen für die Bereitstellung der biospezifischen Grenzschicht wesentlichen Eigenschaften nicht beeinträchtigen. Die Bindung des Ankers an den Feststoff während der Synthese der LAK ist bevorzugt kovalent.

Besonderes Augenmerk liegt bei der Aufgabenstellung darin, Syntheseverfahren z.B. der kombinatorischen Chemie zur Erzeugung von bioaktiven Oberflächen auf der Basis von organischen Monolagen einzusetzen, wobei die Synthese jedoch aus oben genannten Gründen nicht direkt an der Monolage erfolgen soll. Die kombinatorische Chemie umfaßt eine ganze Bandbreite von Techniken, die in wenigen, oft automatisierten Reaktionsfolgen eine Vielzahl verschiedener Substanzen (sog. Substanzbibliotheken) zu erzeugen vermögen (siehe z.B. M.A. Gallop et al, J. Med. Chem. 37 (1994); 1233-1251, E.M. Gorden et al, J. Med. Chem. 37 (1994), 1385-1401). Auch hierbei werden die Reaktionen aus praktischen Gründen bevorzugt an fester Phase durchgeführt. Die Trägermaterialien sind üblicherweise quervernetzte Polymere in Partikelform (sog. "beads" oder Perlen aus Polystyrol- oder Polyethylenglykol/Polystyrolhari). Ausgehend von einer funktionalisierten Oberfläche werden in mehreren Syntheseschritten die gewünschten Strukturen aufgebaut. Einen Überblick über die Synthese von Substanzbibliotheken an fester Phase sowie in Lösung geben L.A. Thompson und J.A. Ellman, Chem. Rev. 96 (1996), 555-600. Nach Abschluß einer kombinatorischen Festphasensynthese werden die Produkte allgemein von der festen Phase abgespalten, d.h. durch Trennen einer labilen Bindung zwischen Endprodukt und Trägerharz freigesetzt und zum Zwecke des HTS gereinigt oder direkt in biologische Assaymedien transferiert. Es wurde auch versucht, die Produkte auf den Perlen zu belassen und die biomolekularen Interaktionsstudien unmittelbar an dem Trägermaterial durchzuführen. Dies hat jedoch den massiven Nachteil, daß die für die organisch-chemische Synthese geeigneten Substratmaterialien aufgrund ihrer hohen unspezifischen Bindungskapazität für Interaktionsanalysen ungeeignet sind.

Die Synthese einer Bibliothek von LAK, die sich nur in ihren Liganden unterscheiden, wird durch den Einsatz einer vorgefertigten, den Anker bereits enthaltenden Festphase enorm erleichtert. Für die Anker-Konjugat-Synthese vormodifizierte Partikel tragen in diesem Fall bereits alle für den Aufbau oder die Immobilisierung der Liganden erforderlichen molekularen Elemente, einschließlich des gesamten Ankermoleküls. Der Anker ist durch einen Linker an die feste Phase gekoppelt, der die Freisetzung der LAK nach erfolgter Synthese vorzugsweise unter milden Bedingungen erlaubt.

In einfachen Kupplungsreaktionen können Aliquote der vormodifizierten festen Phase dann mit sehr vielen unterschiedlichen Liganden versehen werden. Bei der Abspaltung vom Trägermaterial werden die konjugierten Beschichtungsstrukturen (LAK) freigesetzt, und die Bildung der Monolagen tritt selbsttätig bei Aufbringen der LAK auf die Oberfläche des Sensors (Kontaktierung) ein.

Mit Hilfe der vorliegenden Erfindung wird somit eine Bindematrix mit definierter Oberfläche durch einfache chemische Synthese bereitgestellt, die eine hohe Flexibilität hinsichtlich Auswahl und Möglichkeiten chemischer Modifikationen der immobilisierten Liganden bietet und sich für den Einsatz in Verfahren mit hohem Probendurchsatz (HTS) eignet.

Als Trägermaterialien, auf die Anker-Ligand-Konjugate oder Gemische davon zur Bereitstellung des Sensors aufgebracht werden, eignen sich bevorzugt Metalle, Edelmetalle oder Metalloxide bzw. Verbundmaterialien, auf deren Oberflächen Edelmetalle, Metalle wie z.B. Kupfer oder Metalloxide aufgebracht sind. Besonders bevorzugt werden Edelmetalle wie Silber, Gold, Palladium oder Platin eingesetzt, insbesondere eignet sich Gold.

In einer weiteren Ausfühnmgsform der Erfindung können biospezifische Grenzschichten auch auf Kunststoffen bereitgestellt werden. Hierbei können die Oberflächen handelsüblicher Polymermaterialien, wie Polyalkylene (z.B. PP oder PE), PTFE, PMMA oder Polycarbonate verwendet werden, aber auch Polymermischungen die eines oder mehrere dieser Polymere enthalten. Weiterhin können auch Copolymere aus solchen Monomeren eingesetzt werden, die die genannten Kunststoffe bilden.

Die in der einsatzbereiten Messanordnung mit dem Träger verbundenen Anker sollten strukturelle Untereinheiten aufweisen, die die folgenden Aufgaben ermöglichen
a) Anbindung des Ankers an die Meßoberfläche
b) Kopplung des Liganden L oder dessen Aufbau an den bzw. dem der Meßoberfläche entgegengesetzten Teil des Ankers (ω-ständig);
c) Ausbildung einer Monoschicht (selbstassemblierte Monolage, SAM) bei Inkontaktbringen der LAK mit der Meßoberfläche.

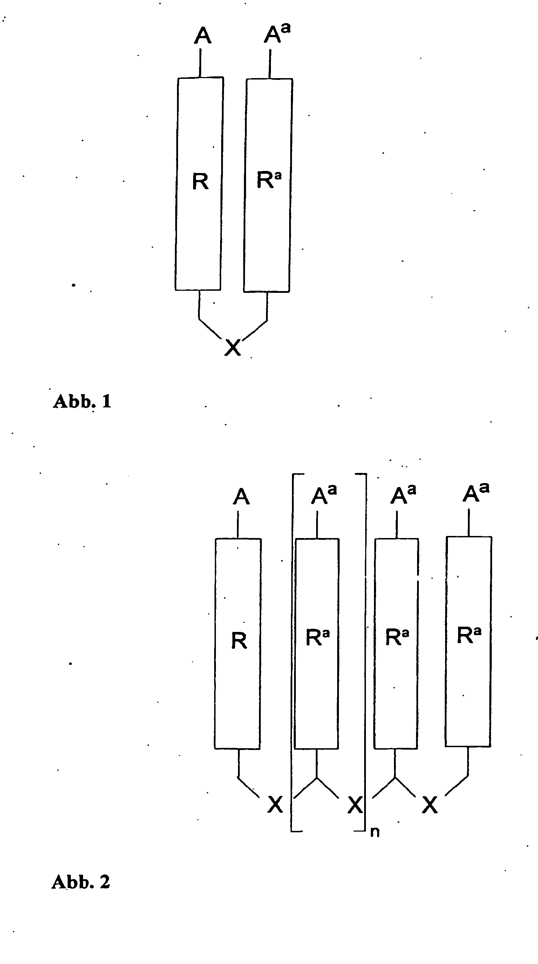

Eine schematische Darstellung des Aufbaus eines erfindungsgemäßen Ankermoleküls ist in Abb. 1 wiedergegeben.

Die Struktureinheit X erlaubt dabei die Anbindung des fertigen LAK an der Meßoberfläche, die Gruppierungen R bzw. R^{a} umfassen Reste, die die Ausbildung einer SAM ermöglichen. Die endständigen Gruppen A bzw. A^{a} dienen zur Anbindung von Liganden oder Nichtliganden. R^{a} und A^{a} gemeinsam oder A^{a} alleine können gegebenenfalls durch ein Wasserstoffatom ersetzt sein. Die angeführten strukturellen Untereinheiten des Ankers sind direkt oder durch kurzkettige, bivalente Kupplungsgruppen wie C₁-C₄ Alkylen, insbesondere Methylen oder Ethylen, kovalent verknüpft (in der schematischen Darstellung durch Striche symbolisiert). Zusätzlich kann der Anker eine Struktureinheit Y enthalten, die vom Linker zur Anbindung an die feste Phase während der Synthese herrührt.

Die Gruppierung X dient zur Anbindung des LAK an die Meßoberfläche und enthält bevorzugt ein Element der V. oder VI. Hauptgruppe, wobei auch Kombinationen von identischen oder verschiedenen Elementen verwendet werden können. Vorteilhaft sind hier Kombinationen wie -S-Se- oder -Se-Se-. Ebenfalls vorteilhaft ist, je nach Oberflächenbeschaffenheit die Verwendung von bei neutralem pH ionisiert vorliegenden Gruppen, wie z.B. Sulfonat. Bevorzugt eingesetzt wird Schwefel, z.B. in Form der Disulfidfunktion (-S-S-), der Thiolfunktion (-SH) oder der Sulfidfunktion (-S-). Die verwendeten Elemente zeichnen sich dadurch aus, daß sie entweder eine hohe Affinität zu Metallen, insbesondere Edelmetallen (Gold, Silber etc.), aufweisen und somit eine Immobilisierung der erfindungsgemäßen Ligand-Anker-Konjugate z.B. auf einer Gold-, Silber oder Platinoberfläche ermöglichen, oder aber, wenn es sich um eine ionische Gruppe handelt; an eine Metalloxidoberfläche wie z.B. Al₂O₃ binden können.

Es ist bekannt, daß neben Thiolen auch Sulfide besonders zur Ausbildung von SAMs geeignet sind (Troughton et al, Langmuir 4 (1988) 365-85; Schierbaüm et al, Science 265 (1994) 1413-5; Huismann et al, JACS 118 (1996) 3523-4). Sulfide zeigen bei der chemischen Synthese, besonders der Festphasensynthese, bezüglich ihrer Stabilität Vorteile gegenüber Thiolen oder Disulfiden.

Eine besonders bevorzugte Ausführungsform der vorliegenden Erfindung besteht daher in Ankermolekülen, die auf Sulfidbasis mit der Meßoberfläche verbunden werden. Die "Sulfid-Anker" können auf einer dem Schwefel abgewandten, omega-ständigen Seite der Kette mit Molekülstrukturen unterschiedlicher Funktionalitäten ausgestattet werden. Es ist besonders vorteilhaft, diese Verknüpfung dem Adsorptionsprozeß voranzustellen und das Konjugat somit vor der Immobilisierung vollständig analytisch zu charakterisieren und auf strukturelle Integrität zu prüfen. Da die Schwefel-Gold Komplexierung eine der wenigen Methoden der nicht-kovalenten Oberflächenmodifizierung darstellt, kann davon ausgegangen werden, daß die Konjugate durch den Adsorptionsprozeß keine Veränderung ihrer chemischen Struktur erfahren. Durch die Verwendung solcher funktionalisierter Konjugate kann jegliche weitere sich der chemischen Analytik entziehende Oberflächenmodifizierung vermieden werden. Zur Erzeugung auch strukturell komplexer biospezifischer Grenzschichten auf Oberflächen ist daher nur ein einzelner Beschichtungsschritt erforderlich.

Von der chemischen Natur der eingesetzten Gruppe X hängt gleichzeitig die chemische Grundstruktur des Ankers ab. Durch den Einsatz von Thiolen werden LAKs erzeugt, die lediglich eine einzelne Kette, die gegebenenfalls verzweigt sein kann, aufweisen. Demgegenüber sind durch Verwendung von Sulfiden und Disulfiden Anker zugänglich, wie sie beispielhaft in Abb. 1 gezeigt sind; sie beinhalten zwei durch die Gruppe X voneinander getrennte Kettenshukturen.

Werden die erfindungsgemäßen LAK auf Kunststoffoberflächen aufgebracht, so dienen die oben aufgeführten Gruppen X vor allem zur Strukturierung des Ankermoleküls, während sich eine attraktive Wechselwirkung zwischen der Gruppe R bzw. den Gruppen R und R^{a} und der Polymeroberfläche ausbildet.

Die Reste R und R^{a} können gleich oder verschieden sein und stellen eine verzweigte oder unverzweigte, gegebenenfalls substituierte, gesättigte oder ungesättigte Kohlenwasserstoffkette dar, die durch Heteroatome, Aromaten und heterozyklische Verbindungen unterbrochen sein kann und 2-2000 Atome, einschließlich Heteroatome, umfaßt. Sind Struktureinheiten des Typs X, z.B. durch Einsatz mehrvalenter Reste R oder R^{a}, miteinander verknüpft, ist auch der Aufbau von oligomeren LAK möglich, die schematisch in Abbildung 2 dargestellt sind, wobei n eine natürliche Zahl ≥ 0 darstellt. Die Reste R^{a} und A^{a} sind dabei wie vorstehend definiert und können ebenfalls jeweils gleich oder verschieden sein.

Die erfindungsgemäßen Anker sind ω-ständig bezüglich der Gruppe X funktionalisiert, um so die Anbindung der Liganden zu ermöglichen. Funktionelle Gruppen A bzw. A^{a}, die zu diesem Zweck eingesetzt werden können, sind u.a.: Acetale, Ketale, Acylale, Acylhalogenide, Alkohole (Hydroxylgruppen), Aldehyde, Alkene, Halogenide, Alkine, Allene, Amide, Amidine, Aminaie, Amine, Anhydride, Azide, Azine, Aziridine, Azoverbindungen, Borane, Carbamate, Carbodiimide. Carbonsäuren, Carbonsäureester, Cyanamide, Cyanate, Diazoverbindungen, Diazoniumsalze, Epoxide, Ether, Hydrazide, Hydrazine, Hydrazone, Hydroxamsäuren, Hydroxamsäureester, Hydroxylamine, "Imide, Imine, Anorganische Ester, Isocyanate, Isocyanide, Isothiocyanate, Ketene, Ketone, Nitrile, Nitroverbindungen, Nitrosoverbindungen, Oxime, Phenole, Phosphine, Phosphonate, Ammoniumsalze, Phosphoniumsalze, Sulfonamide, Sulfone, Sulfonsäuren, Sulfonester, Sulfoniumsalze, Sulfonylazide, Sulfonylhalogenide, Sulfbxide, Thioamide, Thiocarbamate, Thiocyanate, Triazene, Harnstoffe oder Isohamstoffe. Die Reste A und A^{a} können gleich oder verschieden sein, A^{a} kann durch ein Wasserstoffatom ersetzt sein. Sie sollten bevorzugt weniger als 10, besonders bevorzugt weniger als 4 C-Atome umfassen. Besonders bevorzugt als funktionelle Gruppen A und A^{a} sind Hydroxylgruppen, primäre oder sekundäre Amine, bevorzugt C₁-C₄ N-alkyliert, und Carbonsäuren die direkt als Substituenten mit R bzw. R^{a} verbunden sind. Sie können gegebenenfalls aktiviert werden (z.B. als Aktivester), um so die Verknüpfung mit den Liganden zu erleichtern.

Reaktionen zur Bindung des Liganden an den Anker können z.B. Substitutions- oder Additionsreaktionen, Eliminierungsreaktionen (Additions- Eliminierungsreaktionen wie Kondensationsreaktionen) sein, es können Reaktionen zur Ausbildung einer Doppelbindung, wie z.B. Wittig-Reaktion, oder einer C-C-Einfachbindung, z. B. Aldol, Heck- oder Suzuki-Reaktion, oder elektrozyklische Reaktionen sein. Diese Aufzählung ist nicht erschöpfend oder einschränkend und kann leicht von einem Fachmann ergänzt werden.

Ein Vorteil des erfindungsgemäßen Verfahrens besteht darin, daß für die Anbindung des Liganden an den Anker und die Anbindung an die feste Phase (SP) die gleiche funktionelle Gruppe verwendet werden kann, da letztere während der Anbindung des Liganden durch die feste Phase regioselektiv "blockiert" ist.

Funktionelle Reste (Y), die die Ankerstruktur aufgrund ihrer Anbindung an eine feste Phase während ihrer Synthese auch nach der Trennung der LAK vom Linker enthalten kann, umfassen: Acetale, Ketale, Acylale, Acylhalogenide, Alkohole, Aldehyde, Alkene, Halogenide, Alkine, Allene, Amide, Amidine, Aminale, Amine, Anhydride, Azide, Azine, Aziridine, Azoverbindungen, Borane, Carbamate, Carbodiimide, Carbonsäuren, Carbonsäureester, Cyanamide, Cyanate, Diazoverbindungen, Diazoniumsalze, Epoxide, Ether, Hydrazide, Hydrazine, Hydrazone, Hydroxamsäuren, Hydroxamsäureester, Hydroxylamine, Imide, Imine, Anorganische Ester, Isocyanate, Isocyanide, Isothiocyanate, Ketene, Ketone, Nitrile, Nitroverbindungen, Nitrosoverbindungen, Oxime, Phenole, Phosphine, Phosphonate, Ammoniumsalze, Phosphoniumsalze, Sulfonamide, Sulfone; Sulfonsäuren, Sulfonester, Sulfoniumsalze, Sulfonylazide, Sulfönylhalogenide, Sulfoxide, Thioamide, Thiocarbamate, Thiocyanate, Triazene, Harnstoffe oder Isoharnstoffe. Diese Reste sind bevorzugt direkt oder über eine gegebenenfalls in Y enthaltene Seitenkette mit dem Rest des Ankers verknüpft, wobei die Seitenkette 1-8, bevorzugt 1-4 C-Atome aufweist. Sie kann durch weitere funktionelle Einheiten, insbesondere -0- oder -CONHunterbrochen sein. Der Rest Y in seiner Gesamtheit umfaßt nicht mehr als 20, bevorzugt nicht mehr als 10, besonders bevorzugt nicht mehr als 5 C-Atome.

Bei Verwendung spezieller Linkerverbindungen zur Verknüpfung des Ankers mit der festen Phase ("traceless linker") kann das LAK auch ohne Zurückbleiben einer funktionellen Gruppe an der Ankerstruktur gelöst werden. Der Linker wird in diesem Fall bei der Abspaltung durch ein Wasserstoffatom ersetzt. Überraschenderweise wurde jedoch gefunden, daß trotz des Vorhandenseins einer funktionellen Gruppe Y in einem Seitenarm des Ankers die Ausbildung von SAMs nicht oder nur geringfügig gestört wird, so daß auf konventionelle, meist auch kostengünstigere Linker zurückgegriffen werden kann, die solche Gruppen hinterlassen.

Linker, die vorteilhaft in der Festphasensynthese einzusetzen sind, wie auch Reste, die nach der Trennung des Zielmoleküls von der festen Phase an diesem verbleiben, sind z.B. in Novabiochem® Combinatorial Chemistry Catalog & Solid Phase Organic Chemistry Handbook, März '98, Callbiochem-Novobiochem AG, Schweiz beschrieben. Bei der Verwendung identischer Linker kann die Struktur der resultierenden Gruppen Y je nach eingesetztem Spaltungsreagenz variieren. Bevorzugte funktionelle Gruppen, die nach der Abspaltung der LAK von der festen Phase am Anker verbleiben, sind in der folgenden Tabelle aufgeführt.

| | |
|---|---|
| Y an SP gebunden | Y nach Abspaltung von fester Phase (SP) |
| -CO- | -COOH |
| | -COOR' |
| | -CHO |
| | -CH₂OH |
| | -CO-NR'₂ |
| | -CO-NH-OH |
| | -CO-NH-NH₂ |
| | -*cyclo*[CO-NH-CH(R')-CO-NH-CH] |
| -O- | -OH |

wobei jeder der Reste R' unabhängig voneinander ein Wasserstoffatom oder ein Alkylrest, bevorzugt ein Wasserstoffatom oder ein C₁-C₄- Alkylrest sein kann. Liegt die Gruppierung Y vor, so sind besonders bevorzugt -CONR'₂, -COOH oder -OH.

Die oben als bevorzugt und besonders bevorzugt aufgeführten Gruppen Y können zusätzlich an der freien Valenz eine Kupplungsgruppe umfassen, mittels derer sie mit dem Rest der Ankerstruktur, bevorzugt mit R oder R^{a} verknüpft sind. Bei der Kupplungsgruppe handelt es sich bevorzugt um einen mindestens bivalenten organischen Rest, der geradkettig oder verzweigt sein kann und bevorzugt 1-8, besonders bevorzugt 1-4 Kohlenstoffatome umfaßt. Er kann durch zusätzliche funktionelle Einheiten, insbesondere -O-, -CONR'-, unterbrochen sein, wobei R' wie oben definiert ist. Besonders bevorzugt sind C₁-C₄₋Alkylenreste, wie Methylen, Ethylen oder Propylen.

In einer weiteren bevorzugten Ausführungsform ist die genannte freie Valenz direkt mit R bzw. R^{a} verbunden.

Bevorzugt sollte der Anker Strukturen aufweisen, die eine passive Adsorption des Rezeptors sowohl an der Ankerstruktur als auch auf der Meßoberfläche erschweren oder vermeiden. Weiterhin ist es von Vorteil, wenn der Anker eine Spacergruppierung enthält, die die Anpassung der Gesamtkettenlänge und der Flexibilität des LAK ermöglicht.

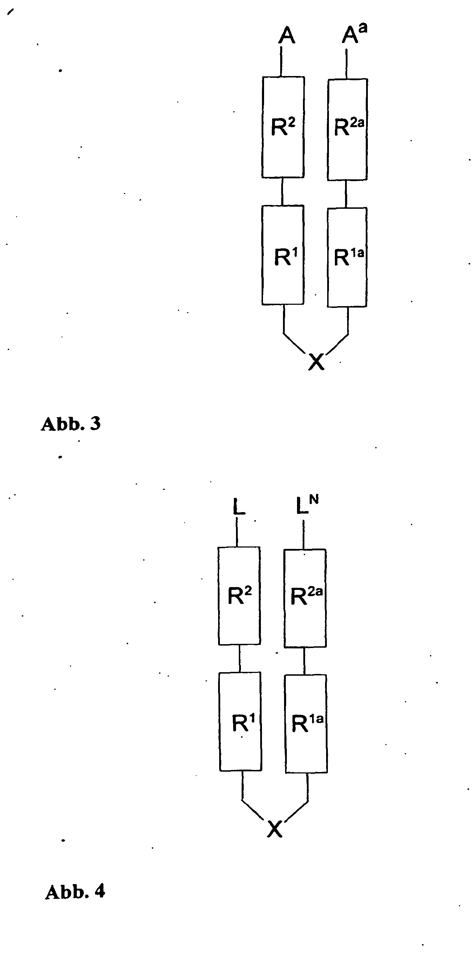

Eine bevorzugte Ausführungsform der erfindungsgemäßen Anker stellt damit die in Abbildung 3 schematisch wiedergegebene Struktur dar,
wobei A, A^{a} und X wie vorstehend definiert sind, und R¹ und R² bzw. R^{1a} mit R^{2a} jeweils die Reste R bzw. R^{a} bilden. Der Rest Y, soweit vorhanden, kann bevorzugt entweder an R¹ bzw. R^{1a} oder an R² bzw. R^{2a} als Seitenkette gebunden vorliegen. Besonders bevorzugt befindet er sich nahe oder an der Verknüpfungsstelle zwischen R¹ bzw. R^{1a} und R² bzw. R^{2a}.

Bevorzugt dienen R¹ und R^{1a} zur Ausbildung eines SAMs und sollen zu diesem Zweck weitgehend hydrophob sein. Sie umfassen unabhängig voneinander eine verzweigte oder unverzweigte Kohlenwasserstoffkette mit 1 bis 50 C-Atomen, die vollständig gesättigt oder teilweise ungesättigt und dabei durch Aromaten, Heterozyklen oder Heteroatome unterbrochen sein kann, wobei eine vollständig gesättigte Kohlenwasserstoffkette ohne Heteroatome bevorzugt ist. Sie hat in einer bevorzugten Form die allgemeine Formel - (CH₂)ₙ-, wobei n eine natürliche Zahl von 1 bis 50, vorzugsweise 3 bis 25, besonders bevorzugt 4 bis 18 und am meisten bevorzugt 8 bis 12 ist.

Für die Einführung von R¹ und/oder R^{1a} in einer besonders bevorzugten Form eignen sich kommerziell erhältliche Verbindungen, insbesonders funktionalisierte Alkane, die an beiden Endgruppen funktionelle Einheiten, wie z.B. Hydroxylgruppen, Halogenatöme, Carbonsäuregruppen oder Mercaptogruppen tragen. Diese terminalen funktionellen Einheiten erleichtern z.B. die Verbindung zu den benachbarten Struktureinheiten während der Synthese des Ankers. Wahlweise können mit ihrer Hilfe notwendige Bestandteile des Ankers, z.B. X, eingeführt werden. Beispielhaft sollen 11-Brom-1-undecanol, 1,10-Decandiol oder 11-Mercaptoundecansäure sowie deren Derivate, angeführt werden, wobei bei letzteren gleichzeitig die Einführung der Schwefelfunktion (X) gewährleistet ist.

R² und R^{2a} stellen bevorzugt Spacer dar, die die Anpassung der Gesamtkettenlänge und der Flexibilität des Ligand-Anker-Konjugates ermöglichen. Sie stellen unabhängig voneinander bevorzugt Kohlenwasserstoffketten dar, die durch Heteroatome unterbrochen und dadurch hydrophil sind und bevorzugt eine passive Adsorption des Rezeptors erschweren. Die Kette umfaßt 2 bis 1000 Atome, einschließlich Heteroatome, bevorzugt sind Kettenlängen von 5 bis 500, ganz besonders bevorzugt sind Kettenlängen zwischen 10 und 100 Atomen.

In einer bevorzugten Ausführungsform ist R² und/oder R^{2a} ein Oligoether der allgemeinen Formel -(OAlk)_{y}-, bei der y eine natürliche Zahl ist und Alk einen Alkylenrest darstellt Bevorzugt ist eine Struktur, bei der y zwischen 1 und 100, bevorzugt zwischen 1 und 20 und am meisten bevorzugt zwischen 2 und 10 liegt. Der Rest Alk weist bevorzugt 1-20, besonders bevorzugt 2-10 und insbesondere bevorzugt 2-5 C-Atome auf. Ganz besonders bevorzugt ist -(OC₂H₄)_{y}-

In einer zweiten bevorzugten Ausführungsform ist R² und/oder R^{2a} ein Oligoamid, aufgebaut aus Dicarbonsäuren und Diaminen und/oder Aminocarbonsäuren, wobei die Amine unabhängig voneinander bevorzugt 1 bis 20, besonders bevorzugt 1 bis 10 Kohlenstoffatome aufweisen und auch durch weitere Heteroatonie, insbesondere Sauerstoffatome, unterbrochen sein können. Die Carbonsäuremonomere besitzen unabhängig voneinander bevorzugt 1 bis 20, besonders bevorzugt 1 bis 10 Kohlenstoffatome und können ebenfalls durch weitere Heteroatome, insbesondere Sauerstoffatome, unterbrochen sein.

Für die Einführung von R² und/oder R^{2a} in einer besonders bevorzugten Form eignen sich kommerziell erhältliche Verbindungen, wie insbesondere Glycolether wie z.B. Triethylenglycol, Triethylenglycolmonomethylether, Tetraethylenglycol, aber auch Dicarbonsäuren wie Bernsteinsäure, 1,13-Diamino-4,7,10-trioxatridecan, 3,6,9-Trioxaundecandisäure, 8-Amino-3,6-dioxaoctansäure oder 4-Aminobenzoesäure sowie deren Derivate oder Kombinationen aus gleichen Bausteinen (wie. z.B. bei 8-Amino-3,6-dioxaoctansäure oder 4-Amminobenzoesäure) oder Kombinationen aus verschiedenen Bausteinen (wie z.B. 1,13-Diamino-4,7,10-trioxatridecan und 3,6,9-Trioxaundecandisäure in alternierender Folge). Ein Vorteil bei der Verwendung von 4-Aminobenzoesäure liegt in ihrer guten spektroskopischen Detektierbarkeit z.B. mittels UV-Spektroskopie.

Während R¹ und R² in der in Abbildung 3 angegebenen Strukturformel vorhanden sein müssen, können eine oder zwei beliebige Struktureinheiten, ausgewählt aus R^{1a}, R^{2a} und F^{a} gegebenenfalls fehlen. Auch die Kombination aus R^{1a}, R^{2a} und A^{a} kann gegebenenfalls vollständig durch ein Wasserstoffatom ersetzt sein.

In einer besonders bevorzugten Ausführungsform der vorliegenden Erfindung werden die Ankerstrukturen 1 bis 16, wie unten illustriert, bereitgestellt.

In der bevorzugten Form des Ankers, die zwei Äste aufweist, stehen zwei dieser Endgruppen A und A^{a} zur Verfügung. Sie können zur Bereitstellung der LAK beide durch Liganden (L) besetzt sein, die mit dem Rezeptor in Interaktion treten können. Bevorzugt ist nur eine Endgruppe mit einem Liganden besetzt, während die andere mit einer niedermolekularen Verbindung (L^{N}) abgesättigt ist, die nicht zu einer solchen Interaktion fähig ist (Nichtligand). Die entsprechende Struktur ist in Abb. 4 gezeigt.

Solche SAM-bildenden Unterstrukhiren der LAK, die mit einer Gruppierung L^{N} für Detektionszwecke nicht mehr zur Verfügung stehen, ermöglichen eine genaue Strukturierung der interaktionsfähigen Liganden auf-der Meßoberfläche, während gleichzeitig in den entstehenden Zwischenräumen eine passive, d.h. unspezifische Adsorption des Rezeptors vermieden wird. Sie werden als "Verdünnerkomponenten" bezeichnet. Gegebenenfalls können bei der Bereitstellung der Meßoberfläche zusätzlich Ankerstrukturen eingesetzt werden, die ausschließlich Gruppierungen L^{N} tragen, um diesen Effekt noch zu verstärken.

Der Ligand L dient zur Bereitstellung spezifischer Strukturmerkmale in der erzeugten Grenzschicht, die damit für eine Wechselwirkung mit dem Rezeptor zur Verfügung steht. Im Rahmen der Festphasensynthese kann Ligand L in einem einzelnen Schritt mit dem Anker verknüpft oder in mehreren Syntheseschritten am Anker aufgebaut werden. Letzteres Verfahren ist insbesondere beim Einsatz kombinatorischer Synthesemethoden von Vorteil, bei denen unter Verwendung von Gemischen aus zwei oder mehr Synthesebausteinen eine große Anzahl strukturell diversifizierter Liganden in wenigen Schritten erzeugt werden kann. Liganden, die typischerweise verwendet werden, um der Meßoberfläche eine Bioaktivität zu verleihen, umfassen: Proteine, Peptide, Oligtinucleotide, Kohlenhydrate ; (Glycoside), Isoprenoide, Enzyme, Lipidstrukttiren, sowie organische Moleküle, die ein Molekulargewicht ≥ 50 g/mol besitzen und charakteristische räumliche oder elektronische Strukturen aufweisen, wie z.B. eine Aminosäure, ein Nukleosid, eine heterocyclische Verbindung, eine alicyclische Verbindung, eine aromatische Verbindung, ein Terpen, eine phosphororganische Verbindung, ein Chelatkomplex, Neurotransmitter, ein substituiertes Amin, ein Alkohol, ein Ester, ein Ether oder eine Carbonsäure und deren Derivate. Sie können unter Verwendung literaturbekannter Reaktionen (siehe z.B. J.S. Früchtel, G. Jung, Angew. Chem. Int. Ed. 35, (1996) 17-42) synthetisiert werden. Diese Aufzählung ist weder erschöpfend noch beschränkend und kann vom Fachmann mühelos ergänzt werden.

In WO-A2-8903041 und WO-A1-8903042 werden Moleküle mit Molekülmassen von bis 7000 g/mol als kleine Moleküle beschrieben. Üblicherweise werden jedoch Molekülmassen zwischen 50 und 3000 g/mol, häufiger aber zwischen 75 und 2000 g/mol und meistens im Bereich zwischen 100-1000 g/mol angegeben. Solche kleinen Moleküle sind z.B. in WO-A1-8602736, WO-A1-9731269, US-A-5928868, US-A-5242902, US-A-5468651, US-A-5547853, US-A-5616562, US-A-5641690, US-A-4956303 und US-A-5928643 offenbart.

Im Rahmen der vorliegenden Erfindung soll das Molekulargewicht eines Liganden/kleinen Moleküls (ohne Anker) zwischen 50 und 1500 g/mol, bevorzugt zwischen 75 und 1500 g/mol liegen. Beispiele für kleine Moleküle, die im Rahmen der vorliegenden Erfindung als Liganden eingesetzt werden können, umfassen die folgenden Verbindungen:
Propargylamin, Cyclopropylamin, Propylamin, Ethylendiamin, Ethanolamin, Imidazol, 3-Aminopropionitril, Pyrrolidin, Glyoxylsäure-Monohydrat, Essigsäurehydrazid, L-Glycin, Glykolsäure, Pyridin, 1-Methylimidazol, Cyanessigsäure, Cyclopropancarbonsäure, (S)-(+)-3-Methyl-2-butylamin, Brenztraubensäure, N,N-Dimethylethylendiamin, N,N'-Dimethylethylendiamin, L-Alanin, Beta-L-Alanin, D-Alanin, beta-Alanin, Sarcosin, (R)-2-Amino-1-butanol, 2-Amino-1,3-propandiol, Anilin, 3-Aminopyridin, 4-Pentinsäure, 4-Pentensäure, alpha-beta-Dehydro-2-aminobuttersäure, Aminocyclopröpylcarbonsäure, 3-Amino-1-propanol-vinylether, (R)-(-)-Tetrahydrofurfurylamin, (S)-(+)-Prolinol, (R)-3,3-Dimethyl-2-butylamin, 1,5-Diaminopentan, gamma-Aminobuttersäure, 2-Aminobuttersäure, 2-Aminoisobuttersäure, 3-Amino-2,2-dimethyl-1-propanol, Thiomorpholin, L-2,3-Diaminopropionsäure, D-Serin, L-Serin, 2-(2-Aminoethoxy)ethanol, (Methylthio)essigsäure, Benzylamin, 3-Chlorpropionsäure, 4-Aminophenol, Histamin, Chinuclidin, Exo-2-aminonorbornan, Cyclopentancarbonsäure, trans-1,4-Diaminocyclohexan, L-Prolin, D-Prolin, L-Allylglycin, 1-Amino-1-cyclopentanmethanol, Tetrahydro-2-furoesäure, 3,3-Dimethylbuttersäure, Succinamidsäure, L-Valin, L-Leucinol, Hydantoinsäure, L-Threonin, D-Threonin, (S)-(-)-alpha-Methylbenzylamin, 2-(2-Aminoethyl)pyridin, 5-Amino-o-cresol, p-Anisidin, Pyrazincarbonsäure, 1-(3-Aminopropyl)-imidazol, Tropan, Cyclooctylamin, L-alpha-Aminocaprolactam, 5-Oxo-L-prolin, Isonipecotinsäure. L-Pipecolinsäure, 1,4,7-Triazacyclononan, Octylamin, Dibutylamin, 4-Methyl-2-oxovaleriansäure, L-Asparaginsäure, L-Asparagin, L-Leucin, 6-Aminticaprorisäure; L-Isoleucin, L-alpha-t-Butylglycin, D-Leucin, Z-beta-Alanin, L-Ornithin, 5-Aminoindol, D-Asparaginsäure, L-Thiazolidin-4-carbonsäure, 4-Aminobenzoesäure, 3-(2-Furyl)acrylsäure, 3-Thiophenessigsäure, Cycloheptancarbonsäure, 3,5-Difluorbenzylamin, 1,4-Dioxa-8-azaspiro[4,5]-decan, N-Cyclohexylethanölamin, Caprylsäure, L-Glutamin, D-Glutamin, L-Lysin, D-Glutaminsäure, L-Glutaminsäure, 4-Cyanobenzoesäure, (S)-1,2,3,4-Tetrahydro-1-naphthylamin, 2,2,3,3,3-Pentafluörpropylamin, (1S,2R)-(-)-cis-1-Amino-2-indanol, L-Methionin, D-Methionin, 4-Carboxybenzaldehyd, 3-Phenylpropionsäure, 4'-Aminoacetanilid, Piperonylamin, L-Phenylglycin, D-Phenylglycin, 4-(Aminomethyl)benzoesäure, 1-Adamantanamin, 4-(Hydroxymethyl)benzoesäure, (-)-cis-Myrtanylamin, (1R,2R,3R,5S)-(-)-Isopinocampheylamin, (R)-(+)-Bornylamin, 1,3,3-Trimethyl-6-Azabicyclo[3,2,1]octan, 3,5-Dihydroxybenzoesäure, 2-Norbomanessigsäure, L-2-Furylalanin, L-Histidin, D-Histidin, L-Cyclohexylglycin, Ethylpipecolinat, 5-Amino-1-naphthol, Tryptamin, 4-Aminobutyraldehyddiethylacetal, 2-Benzofurancarbonsäure, L-Indolin-2-carbonsäure, D-Phenylalanin, L-Phenylalanin, 4-Dimethylaminobenzoesäure, L-Methioninsulfoxid, 3-(4-Hydroxyphenyl)propionsäure, DL-Atrolactinsäure-Hemihydrat, 4-Sulfamoylbuttersäure, Vanillinsäure, 4-Aminobiphenyl, (R)-(+)-Citronellsäure, 4-Chlorphenylessigsäure, L-3-Thienylalanin, L-Cyclohexylalanin, D-Cyclohexylalanin, (S)-(-)-1-(1-Naphthyl)-ethylamin, 2-Chlor-6-methylniaotinsäure, L-Arginin, D-Arginin, L-4-Thiazolylalanin, 3-Pyridylessigsäure-Hydrochlorid, 3-Indolylessigsäure, 7-Amino-4-methylcumarin, L-Citrullin, 4-Benzylpiperidin, 2,4-Dichlorbenzylamin, 4-Amino-N-methylphthalimid, (-)-Cotinin, L-Tetrahydroisochinolincarbonsäure, 4-Acetamidobenzoesäure, (R)-(-)-2-Benzylamino-1-butanol, 4-Pentyloxyanilin, o-Acetylsalicylsäure, 4-Nitrophenylessigsäure, 2-Nitrophenylessigsäure, 2-Methyl-6-nitrobenzoesäure, L-Tyrosin, D-Tyrosin, L-Methionin(O2), 3-(Diethylamino)propionsäure-Hydrochlorid, 4-Nitroanthranilsäure, 2,6-Dimethoxybenzoesäure, 3,5-Dimethoxybenzoesäure, 3,4-Dihydroxyhydrozimtsäure, 2=(4-Hydroxyphenoxy)-propionsäure, 2-Methoxyphenoxyessigsäure, 4-Hydroxy-3-methoxyphenylessigsäure, 4-(Ethylthio)benzoesäure, S-Benzylthioglykolsäure, 4-(Methylthio)phenylessigsäure,- 2-Chlorzimtsäure, 3-Chlorzimtsäure, gamma-Maleimidobuttersäure, 2,6-Dimethoxynicotinsäure, L-4-Fluörphenylalanin, L-2-Fluorphenylalanin, (R)-(-)-Epinephrin, Cyclododecylamin, trans-2,5-Difluorzimtsäure, DL-3,4-Dihydroxymandelsäure, Thymin-1-essigsäure, cis-Pinonsäure, 1,2-Bis(4-pyridyl)ethan, 4-tert-Butylcyclohexancarbonsäure, N,N-Diethylnipecotamid, 3,4-Difluorhydrozimtsäure, 2-Naphthylessigsäure, 3-Carboxyproxyl, 4-Chlor-o-anissäure, 4-Chlorphenoxyessigsäure, 3-Chlor-4-hydroxyphenylessigsäure, 5-Chlor-2-methoxybenzoesäure, 4-Chlor-DL-mandelsäure, 4-(Pyrrol-1-yl)benzoesäure, 4-(Difluormethoxy)benzoesäure, Gallussäure-Monohydrat, 2,4,6-Tnhydroxybenzoesäure-Monohydrat, 6-Hydroxy-2-naphthoe-Säure, Suberinsäure-Monomethylester; 2-Hydroxydecansäure, 2-Chlor-6-fluorphenylessigsäure, alpha-Cyano-3-hydroxyzimtsäure, Indol-3-glyoxylsäure, 8-Hydroxychinolin-2-carbonsäure, 2-Methyl-3-indolessigsäure, 4-(Trifluormethyl)benzoesäure, Cumarin-3-carbonsäure, 3-Hydroxy-2-chinoxalincarbonsäure, 4-Fluor-1-naphthoesäure, 1-Phenyl-1-cyclopentancarbonsäure, p-Toluolsulfonylchlorid, 5-Brom-2-furoesäure, 2,5-Dichlorbenzoesäure, 3,4-Dichlorbenzoesäure, 5-Methoxyindol-2-carbonsäure, Isochinolin-3-carbonsäure-Hydrat, L-Styrylalanin, 4-(Dimethylamino)zimtsäure, 4-Oxo-2-thiöxo-3-thiazolidinylessigsäure, 1-Ethyl- 3-(3-dimethylaminopropyl)carbodiimid- Hydrochlorid. 5,6-Dichloraicotinsäure, 2,6-Dichltimicotinsäure, 2,6-Dichlorpyridin-4-carbonsäure, Trimellith-Anhydrid, D-(-)-Chininsäure, trans-3,4-Methylendioxyzimtsäure, 7-Methoxybenzofuran-2-carbonsäure, trans-5-Acetoxy-1,3-oxathiolan-2-carbonsäure, 4-Benzoylbuttersäure, 4-Pentylbenzoesäure, 6-Phenylhexansäure, 2-Clilor-4,5-difluorbenzoesäure, 4-Chlor-2,5-difluorbenzoesäure, 5-Fluorindol-3-essigsäure, N-Formyl-DL-phenylalanin, 4-Diethylaminobenzoesäure, 2-Aminoanthracen, D-Glucuronsäure, trans-Ferulasäure; (S)-(+)-o-Acetylmandelsäure, 4-Aminohippursäure, 1-Adamantanessigsäure, 6-Bromhexansäure, alpha-Hydroxyhippursäure, N-[3-(2-Furylacryloyl)]-glycin, 1-Methyl-2-aminoterephthalat, L-Serin(Bzl), 3,3,3-Trifluor-2-(Trifluormethyl)propionsäure, Diethylphosphonoessigsäure, D-Gluconsäure, 3-(4-Fluorbenzoyl)-propionsäure, 2,5-Dimethoxyphenylessigsäure, Monomethyl-cis-5-norbornen-endo-2,3-dicarboxylat, 4-Hydroxy-3-nitrophenylessigsäure, 3-Methoxy-4-nitrobenzoesäure, 5-Methoxy-2-nitrobenzoesäure, - 3,4,5-Trimethoxybenzylamin, DL-4-Hydroxy-3-methoxymandelsäure, (-)-Camphansäure, (1R)-(+)-Camphansäure, 2-Methoxy-4-(methylthio)benzoesäure, cis-5-Dodecansäure, 4-Amino-5-carboxy-2-ethyl-mercaptopyrimidin, 4-Aminozimtsäure-Hydrochlorid, DL-3-(4-Hydroxyphenyl)milchsäure-Hydrat, 4-(Methylsulfonyl)benzoesäure, 4-Carboxy-2,2,6,6-tetramethylpiperidin-1-oxyl, 2-Butyloctansäure, trans-2-Chlor-6-fluorzimtsäure, 4-Chlor-o-tolyloxyessigsäure, 2-Brombenzoesäure, 4-Carboxybenzolsulfonamid, 2-(2-Aminothiazol-4-yl)-2-methoxyiminoessigsäure, 1-(N-t-Amino)cyclopropancarbonsäure, 2-Chlor-3-nitrobenzoesäure, -4-Chlor-3-nitrobenzoesäure, 2-Chlor-4-nitrobenzoesäure, 4-Chlor-2-nitrobenzoesäure, 4-Amino-5-chlor-2-methoxybenzoesäure, 5-Bromnicotinsäure, 6-Brompicolinsäure, 2-Methyl-5-phenylfuran-3-carbonsäure, Tributylphosphin, 2-Chlor-5-(methylthio)benzoesäure, 4,5-Difluor-2-nitrobenzoesäure, 2-Hydroxy-5-(pyrrol-1-yl)benzoesäure, Indol-3-buttersäure, 2-(Trifluormethyl)phenylessigsäure, 3-(Trifluormethyl)phenylessigsäure, 4-(Trifluormethyl)phenylessigsäure, 3,7-Dihydroxy-2-naphthoesäure, 6-methylchromon-2-carbonsäure, L-Tryptophan, D-Tryptophan, 2,6-Dichlorphenylessigsäure, 3,4-Dichlorphenylessigsäure, 3-(Trifluormethyl)anthranilsäure, alpha-Acetamidozimtsäure, 5-Methoxyindol-3-essigsäure, DL-Indol-3-milchsäure, (1S,2S)-(-)-2-Benzyloxycyclohexylamin, 3,5-Dichloranthrahilsäure, Chlorämben, S-(+)-Ibuprofen, DL-Thioctansäure, 3,5-Dichlor-4-hydroxybenzoesäure, 5-Bromthiophen-2-carbonsäure, 2,3,5,6-Tetrafluor-p-toluylsäure, 2-Fluor-3-(trifluormethyl)benzoesäure, 3-Fluor-4-(trifluormethyl)benzoesäure, 5-Azido-2-nitrobenzoesäure, trans-2,3-Dimethoxyzimtsäure, N-(4-Aminobenzoyl)-beta-alanin, 4-Butoxyphenylessigsäure, 2-(2-Aminophenyl)indol, 2-Amino-3,4,5,6-tetrafluorbenzoesäure, 2-Nitrophenylbenistemsäure, Z-Glycin, 4-(4-Nitrophenyl)buttersäure, S-(-)-2-[(Phenylamino)carbonyloxy]propionsäure, L-Threonin(Bzl), 2,6-Dichlor-S-flüor-3-pyridincarbonsäure, Trimesinsäure, (4-Formyl-3-methoxy-phenoxy)essigsätire, (E)-5-(2-Carboxyvinyl)-2,4-dimethoxypyrimidin, L-Phenylalanin(4-N02), 2-OXo-6-pentyl-2H-pyran-3-carbonsäure, N,N-Bis(2-hydroxyethyl)isonicotinamid, (+/-)-Jasmonsäure, epsilon-Maleimidocapronsäure, (S)-(-)-N-Benzyl-1-phenylethylamin, 2,4-Dinitrobenzoesäure, 2,4,5-Trimethoxybenzoesäure, . 3,4,5-Trunethoxybenzoesäure, S-(Thiobenzoyl)thioglykolsäure, 4-Iodbuttersäure, 3-Phenoxybenzoesäure, 4-(4-Hydroxyphenyl)benzoesäure, D-Desthiobiotin, (-)-Methoxyessigsäure, 2-(o-Chlorphenoxy)-2-methylpropionsäure, 4-Bromphenylessigsäure, 3-Brom-4-methylbenzoesäure, 3-Bromphenylessigsäure, [1R-(1alpha,2beta,3alpha)]-(+)-3-Methyl-2-(nitromethyl)-5-oxocyclopentanessigsäure, L-Asparaginsäure(OCHX), L-1-Naphthylalanin, 2-(Trifluormethyl)zimtsäure, Monomethylsebacat, 5-Aminovaleriansäure, o-Carboxyphenylphosphat, 4-(Trifluormethyl)hydrozimtsäure, Monoethyl-(R)-3-acetoxyglutarat, beta-(Naphthylmercapto)-essigsäure, 3-Brom-4-fluorbenzoesäure, 3-Phthalimidopropionsäure, L-Arginin(NO2), cis-(1S,2R)-(-)-2-Benzylaminocyclohexanmethanol, 7-Hydroxycumarin-4-essigsäure, 2-Sulfobenzoesäure-Hydrat, 5-Methoxy-1-indanon-3-essigsäure, 4,7,10-Trioxa-1,13-tridecandiamin, 2,4-Dichlorphenoxyessigsäure, (S)-(+)-2-Oxo-4-phenyl-3-oxazolidinessigsäure, (S)-(-)-N-(1-Phenylethyl)succinamidsäure, 3-(Trifluormethylthio)benzoesäure, 5-(4-Chlorphenyl)-2-furoesäure, 8-Bromoctansäure, L-Asparaginsäure(OBzl), N-Acetyl-L-tyrosin, 2-Nitro-5-thiocyanatobenzoesäure, 9-Fluorenon-4-carbonsäure, Fluoren-9-essigsäure, 2-Chlor-5-(trifluormethyl)benzoesäure, 1-(4-Chlorphenyl)-1-cyclopentancarbonsäure, 3,5-Diaminobenzoesäurethyl)benzoesäure, 1-(4-Chlorphenyl)-1-cyclopentancarbonsäure, 3,5-Diaminobenzoesäure-Dihydrochlorid, N-Acetyl-4-fluor-DL-phenylalanin, 2,4,6-Trichlorbenzoesäure, 2,3,4,5,6-Pentafluorphenylessigsäure, 2,4-Dinitrophenylessigsäure, 3,4,5-Trimethoxyphenylessigsäure, Xanthen-9-carbonsäure, (R)-(+)-3-Hydroxy-5-oxo-1-cyclopenten-1-heptansäure, 2-Bibenzylcärbonsäure, 2,2-Diphenylpropionsäüre, 4-Bromzimtsäure, 4-Carboxybenzolsulfonazid, 3-Benzoyl-2-pyridincarbonsäure, trans-4-Chlor-3-nitrozimtsäure, 2,3,5,6-Tetrafluor-4-hydroxybenzoesäure-Hydrat, 3,5-Dinitrosalicylsäure, (Z)-(2-(Formamido)thiazol-4-yl)(methoxyimino)essigsäure, L-Glutaminsäure-gamma-cyclohexylester, Mono-2-(methacryloyloxy)ethylsuccinat, Naproxen, L-Lysin-(ALLOC)-OH, 4-Brommandelsäure, 2-Brom-5-methoxybenzoesäure, L-Hydroxyprolin, 6-(Amino)hexansäure, N-tert-Butoxycarbonyl-L-leucin, 4-Brom-3,5-dihydroxybenzoesäure, N-(4-Carboxy-3-hydroxyphenyl)maleimid, 5-(2-Nitrophenyl)-2-furoesäure, - 5-(3-Nitrophenyl)-2-furoesäure, N-Phthaloyl-DL-alpha-aminobuttersäure, L-Thiazolidin-4-carbonsäure, (S)-(-)-alpha-Methoxy-alpha-(trifluormethyl)phenylessigsäure, 7-Carboxymethoxy-4-methylcumarin, 3,5-Di-tert-butylbenzoesäure, 2-(2-Chloracetamido)-4-thiazolessigsäure, 5-Bromorotsäure, 2-Nitro-alpha,alpha,alpha-trifluor-p-toluylsäure, Benzoyl-DL-leucin, L-Glutaminsäure(OBzl), N,N'-Dibenzylethylendiamin, L-Biphenylalanin, Diphensäure, L-4-Bromphenylalanin, Pindolol, L-Leucin-4-nitroanilid, alpha,alpha-Diphenyl-L-prolinol, L-Pentafluorphenylalanin, L-Phosphotyrosin, 4-Iodphenylessigsäure, L-Benzoylphenylalanin, Methylrot, L-Tyrosin(Bzl), Pentafluorphenyltrifluoracetat, L-Lysin(Z), R-(+)-1,1'-Binaphtyl-2,2'-diamin, (+)-Dehydroabietylamin, N-(4-Amino-2-methylphenyl)-4-chlorphthalimid, 1-Pyrenbuttersäure, Atropin, L-Phenylalanin(4-I), 4-(2,4-Di-tert-amylphenoxy)butylamin, L-Diaminopropionsäure(IVDDE), L-Lysin(DDE), L-Lysin(2-Cl-Z)-OH. L-Tyrosin(2,6-Cl2-Bzl), 4,4'-(9-Fluorenyliden)dianilin, L-Hydroxyprolin, 4'-Carboxybenzo-18-krone-6, Cholsäure sowie Verbindungen mit folgender Struktur: 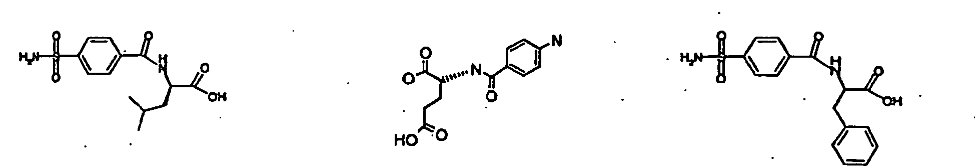 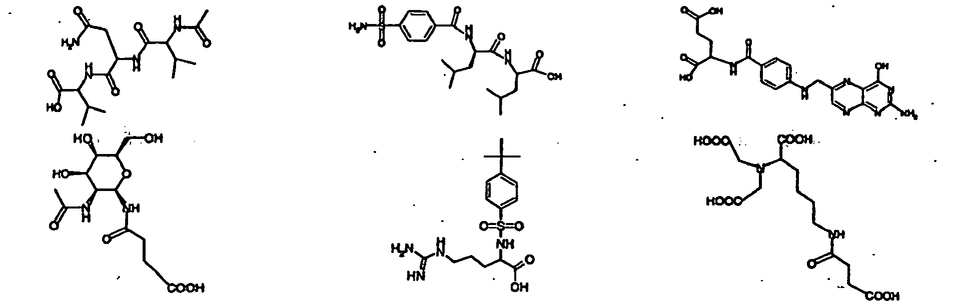 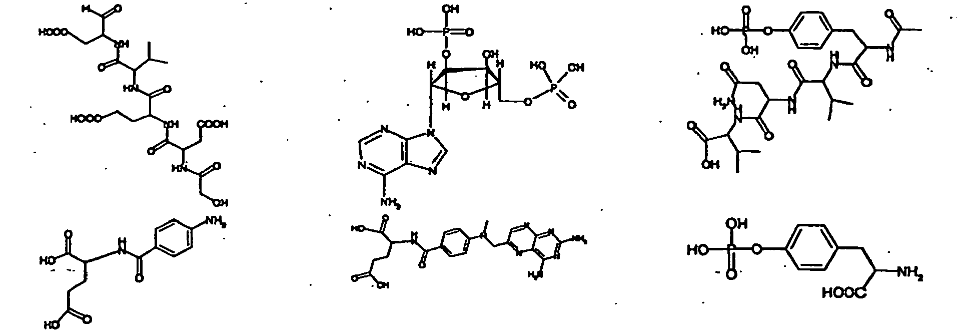 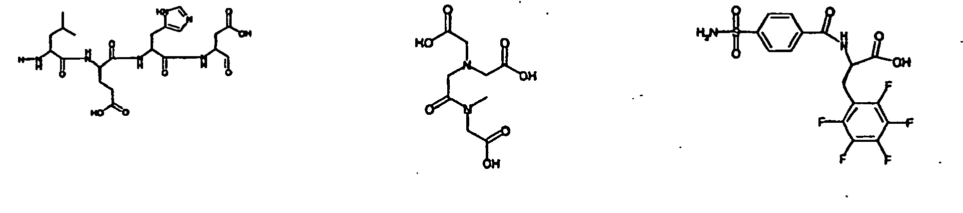 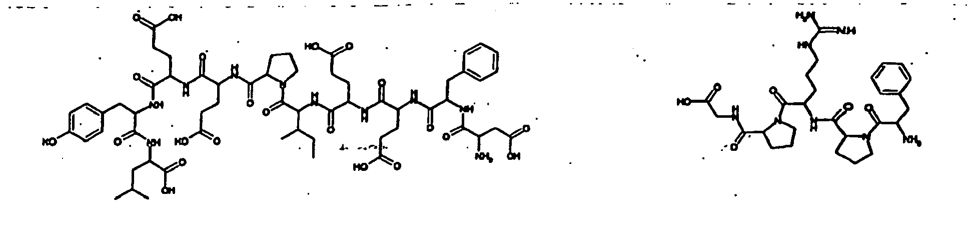 (BZL = Benzyl, OBZL = Benzyloxy, 2-CL-Z = 2-Chlorbenzyloxycarbonyl, 2,6-CL2-BZL = 2,6-Dichlorbenzyloxycärbonyl, DDE = 1-(4,4-dimethyl-2,6-dioxocyclohexyliden)ethyl, ivDDE = 1-(4,4-dimethyl-2,6-dioxocyclohexyliden)3-methylbutyl.)

Diese Aufzählung ist weder erschöpfend noch beschränkend und kann vom Fachmann mühelos ergänzt werden.

Trägt das LAK einen Rest L^{N}, so stellt dieser eine niedermolekulare Verbindung mit einem Molekulargewicht < 75, bevorzugt < 50 g/mol dar, z.B. einen C₁-C₄ Alkoxyrest, bevorzugt Methoxy, oder Acyloxy, bevorzugt Acetyloxy, oder Aminoethyl dar. Die Möglichkeit einer Wechselwirkung des Rezeptors mit solchen Nichtliganden L^{N} ist zwar nicht auszuschließen, jedoch aufgrund der einfachen Struktur solcher Gruppierungen gering. In Fällen, wo ein zweiarmiger Anker verwendet wird, der sowohl einen Rest L als auch einen Rest L^{N} trägt, ist bevorzugt der mit dem Liganden endende Ast länger als der nicht funktionell abgesättigte. Besonders gute Ergebnisse zeigen sich bei einem Längenverhältnis von etwa 2:1.

Das erfindungsgemäße Verfahren zur Bereitstellung der Ligand-Anker-Konjugate umfaßt die folgenden Schritte:
a) Immobilisation oder Synthese eines Ankers an einer für die chemische Synthese geeigneten festen Phase SP
b) Bindung' oder Synthese eines Liganden L an den Anker zur Bildung eines Ligand Anker-Konjugates an der festen Phase SP;
c) Abspalten des Ligand-Anker-Konjugates von der festen Phase SP.

Im Anschluß an die Abspaltung kann das LAK an ein geeignetes Trägermaterial gebunden werden, um so eine einsatzbereite Meßoberfläche zu erhalten.

Gemäß dem erfindungsgemäßen Verfahren wird zunächst eine Verbindung, die in der Regel einen Teil des Ankers darstellt und eine funktionelle Gruppe trägt, mit Hilfe dieser Gruppe an einer für die Festphasensynthese geeignete feste Phase immobilisiert. Zur Anbindung des Ankers an das Harz geht die Festphasensynthese von Verbindungen aus, die beispielsweise folgende funktionelle Gruppen enthalten: Acetale, Ketale, Acylale, Acylhalogenide, Alkohole, Aldehyde, Alkene, Halogenide, Alkine, Allene, Amide, Amidine, Aminale, Amine, Anhydride, Azide, Azine, Aziridine, Azoverbindungen, Borane, Carbamate, Carbodiimide, Carbonsäuren, Carbonsäureester, Cyanamide, Cyanate, Diazoverbindungen, Diazoniumsalze, Epoxide, Ether, Hydrazide, Hydrazine, Hydrazone, Hydroxamsäuren, Hydroxamsäureester, Hydroxylamine, Imide, Imine, Anorganische Ester, Isocyanate, Isocyanide, Isöthiocyanate, Ketene, Ketone, Nitrile, Nitroverbindungen, Nitrosoverbindungen, Oxime, Phenole, Phosphine, Phosphonate, Ammoniumsalze, Phosphoniumsalze, Sulfonamide, Sulfone, Sulfonsäuren, Sulfonester, Sulfoniumsalze, Sulfonylazide, Sulfonylhalogenide, Sulfoxide, Thioamide, Thiocarbamate, Thiocyanate, Triazene, Harnstoffe, Isohamstoffe.

Besonders bevorzugt ist die Ausbildung von Amid- oder Esterbindungen. Hierbei können an der festen Phase Amino-, Hydroxyl, oder Carboxylfunktionen vorliegen. Der Anker bzw. ein Teil des Ankers enthält dann entsprechend eine komplementäre funktionelle Gruppe.

Bei der Abspaltung können diese funktionellen Gruppen am LAK unverändert oder in veränderter Form (z.B. durch Reaktion mit dem Spaltungsreagenz, dem Lösemittel, durch Aktivierung, durch Blockierung oder indem Teile des Linkers am Anker verbleiben) vorliegen. Ebenso können diese Gruppen oder Teile des Ankers am Linker und somit an der festen Phase verbleiben.

Die feste Phase kann ein Syntheseharz, ein Synthesepolymerfilm oder eine zur Synthese geeignete Silizium- oder Silikatffäche sein. Im Falle der Syntheseharze können prinzipiell verschiedene Typen von Harzen eingesetzt werden. Partikelförmige Polymerharze auf Polystyrol- oder Polystyroµl-Polyethylenglykolbasis sind in der Festphasenpeptidsynthese und der kombinatorischen Chemie fest etabliert und auBerordentlich hilfreich bei der Durchführung mehrstufiger Reaktionsfolgen. Als Syntheseharze können kommerziell erhältliche Harze verwendet werden, die direkt oder nach ihrer Modifikation, bevorzugt durch Umsetzung mit Glycolsäure, zum Einsatz kommen. Zur Bindungsvermittlung sind kommerzielle Harze mit sogenannten Linkern versehen, d.h. Verbindungen, die mindestens zwei funktionelle Gruppen bereitstellen und während der Synthese sowohl an die feste Phase als auch an den Anker gebunden sind. Insbesondere können Halomethylharze eingesetzt werden, z.B. Merrifield-Harz. Bei Verwendung dieser Harze ist die funktionelle Gruppe am Ausgangsmolekül für die LAK-Synthese bevorzugt -COOH oder -OH. Im Falle einer Carboxylgruppe (-COOH) liegt diese nach der Abspaltung vom Harz häufig als -COOH, - CH₂OH oder -COOCH₃ vor. Bei einer Hydroxylgruppe (-OH) liegt diese nach der Abspaltung bevorzugt unverändert vor. Für den Fall einer Aminogruppe (-NH₂), liegt nach der Abspaltung bevorzugt -NHSO₂- vor. Eine weitere vorteilhafte Ausführungsform basiert auf der Verwendung von Hydroxyhaizen, wobei eine Cyano-, Carboxyl- oder Hydroxylgruppe im genannten Ausgangsmolekül vorhanden sein kann. Liegt eine Isocyanogruppe (-NCO) vor, so ergibt die Abspaltung bevorzugt ein Harnstoffderivat - NHCONH-. Im Fall der Carboxylgruppe entstehen nach der Abspaltung vom Harz bevorzugt folgende funktionellen Gruppen: -COOH, -CON<, -CH₂OH, -COOCH₃, -CONH₂, -CONH-,-CONHNH₂. Angegebene freie Valenzen dieser Gruppen sind gegebenenfalls bevorzugt mit einem C₁-C₄ Alkylrest abgesättigt. Bei einer Hydroxygruppe entsteht bevorzugt wiederum eine Hydroxy-Funktion. In einer bevorzugten Ausführungsform wird als feste Phase das Harz NovaSyn® TGA (Calbiochem-Novabiochem AG, Schweiz) verwendet. Die Verwendung von Aminoharzen hat sich ebenfalls als vorteilhaft erwiesen, wobei bevorzugt eine Carboxylfunktion zur Anbindung verwendet wird und nach Abspaltung des LAK von der festen Phase als -CONH-,-CHO oder -CO- vorliegen kann. In einer besonders bevorzugten Ausführungsform wird als Aminoharz Tentagel RAM@ (Rapp Polymere, Tübingen) verwendet.

In einer weiteren vorteilhaften Ausführungsform werden als feste Phase Tritylharze verwendet, wobei hier die Anbindung über -COOH, -NH₂, -OH, -CONHNH₂ erfolgen kann. Die nach Abspaltung von der festen Phase entstehende funktionelle Gruppe liegt in diesem Fall anschließend wieder in der ursprünglichen Form vor. In einer bevorzugten Ausführungsform wird das 2-Chlortritylchloridharz (Calbiochem-Novabiochem AG, Schweiz) verwendet. Der Einsatz von Dihydropyran- oder Carboxyharzen als feste Phase hat sich ebenfalls als vorteilhaft erwiesen, wobei in einer bevorzugten Ausführungsform eine Hydroxygruppe im Ausgangsmolekül der LAK Synthese vorliegt und nach Abspaltung unverändert ist. In einer bevorzugten Ausführungsform wird das Carboxyharz NovaSyn® TG Carboxy (Calbiochem-Novabiochem AG, Schweiz) oder das Dihydropyranharz DHP HM-Harz (Calbiochem-Novabiochem AG, Schweiz) verwendet. Weitere vorteilhafte Ausführungsformen umfassen Arylsiloxyharze, wobei die funktionelle Gruppe ein Halögenatom sein kann. Hier tritt nun der Fall auf, daß nach der Abspaltung des LAK von der festen Phase (dem Harz) am Ankerelement ein Bestandteil des Linkers bzw. der festen Phase gebunden ist, da nach Abspaltung anstelle des Halogenatoms bevorzugt die Funktion - Ar-H auftritt, wobei Ar ein aromatischer Rest ist, der von der festen Phase (dem Arylsiloxyharz) stammt.

Als Ausgangsmolekül für die Synthese der LAK und zur Anbindung an das Harz in einer bevorzugten Ausführungsform, eignen sich kommerziell erhältliche Verbindungen, wie Lysin, Lysinol oder 2,3-Diaminopropionsäure sowie deren Derivate, die entsprechend zweifach orthogonal geschützt erhältlich sind. Zur Anbindung an das Harz sind alle vorhandenen Funktionalitäten möglich. Nach der Abspaltung entsteht Y, je nach Abspaltungsbedingung, bevorzugt als ein Methylamid-, Methylcarboxyl- oder Methylhydroxlyrest.

Die Abspaltung des Ligand-Anker-Könjugates von der festen Phase SP kann auch durch eine intramolekulare Zyklisierung induziert werden. Ist der Linker ein Dipeptid, z.B. Lys-Pro, wobei Prolin C-terminal an das Harz gebunden ist, so entsteht nach der Abspaltung vom Harz ein Diketopiperazin [Anker 3, "DKP-Anker"], z.B. durch Abspaltung einer Schutzgruppe, bevorzugt alpha-tert. Butyloxycarbonyl-geschützt; wodurch die spontane Zyklisierung induziert wird. Es besteht auch die Möglichkeit, nach Abspaltung von SP ein Pyrazolon zu bilden, und zwar dann, wenn der Linker eine β-Ketocarbonsäure umfaßt und zur Abspaltung beispielsweise Phenylhydrazin verwendet wird. Das Prinzip der Abspaltung vom Harz durch spontane Zyklisierung nach Deblockierung ist nicht nur auf Diketopiperazine oder Pyrazolone beschrankt.

Die Aufzählung der Harze und Funktionen ist nicht abschließend und kann mühelos vom Fachmann erweitert werden. Eine Übersicht hierzu liefert "Novabiochem® Combinatorial Chemistry Catalog & Solid Phase Organic Chemistry Handbook" März '98, Calbiochem-Novobiochem AG, Schweiz.

Das oben beschriebene Verfahren bietet die Möglichkeit, kombinatorische Chemie zur Festphasensynthese von Ligand-Anker-Konjugaten einzusetzen. Dies erzeugt eine Reihe vorteilhafter Effekte, wie z.B. die Möglichkeit, eine große Anzahl von verschiedenen Konjugaten herzustellen und diese Später einzeln oder in Kombination zum "Drug-Screening" oder für Bindungsmessungen einzusetzen.

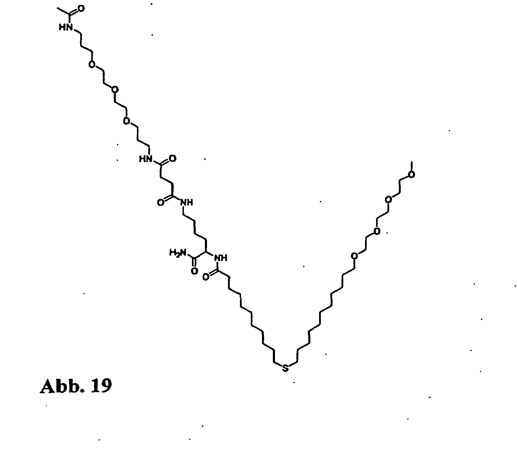

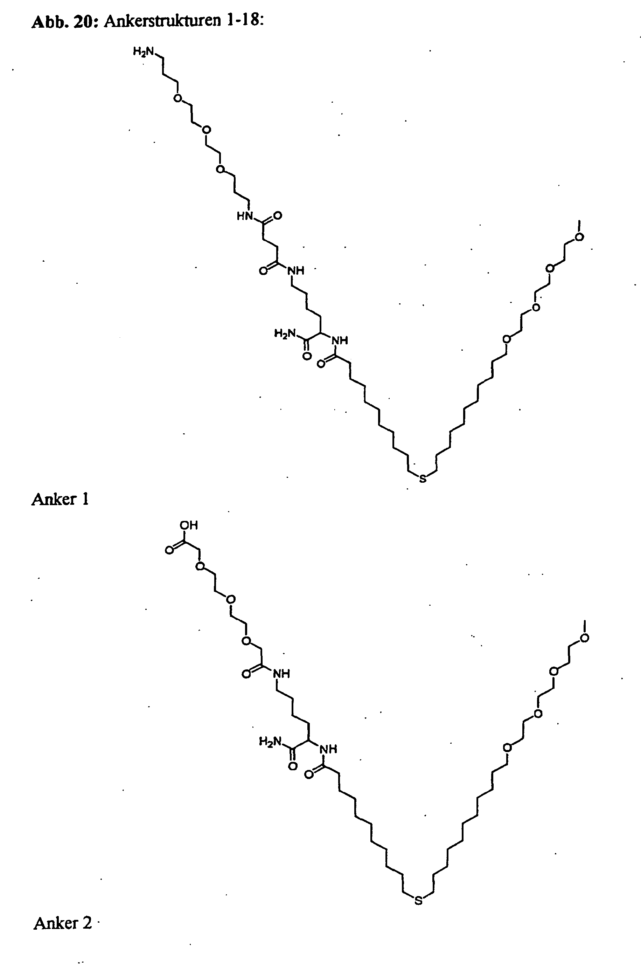

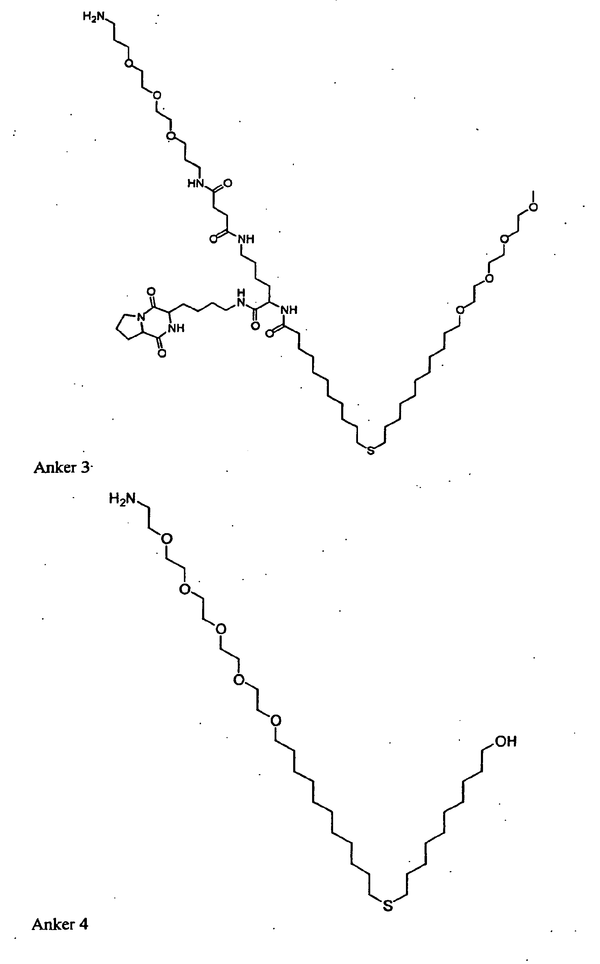

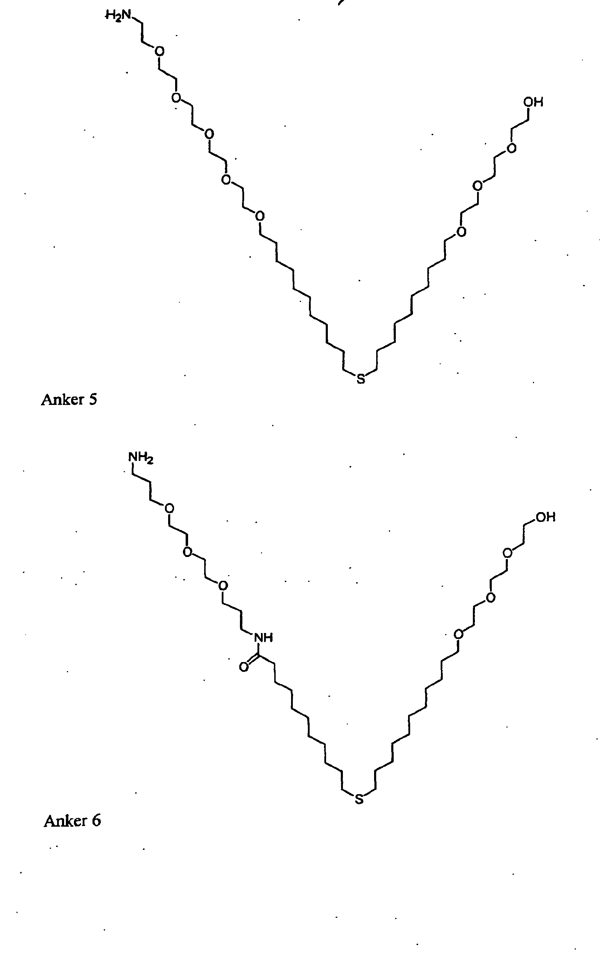

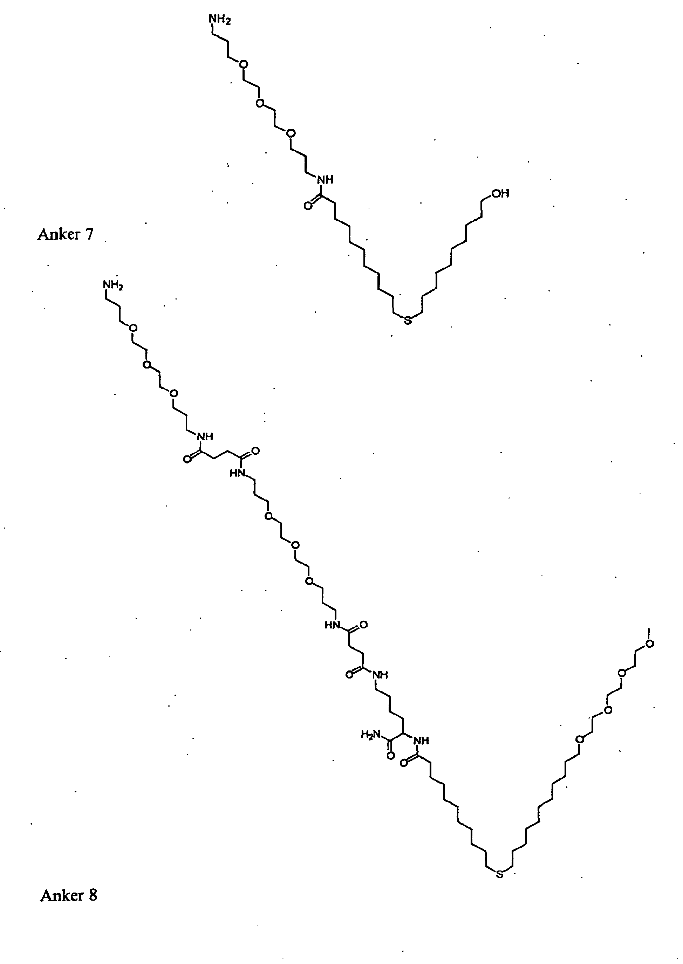

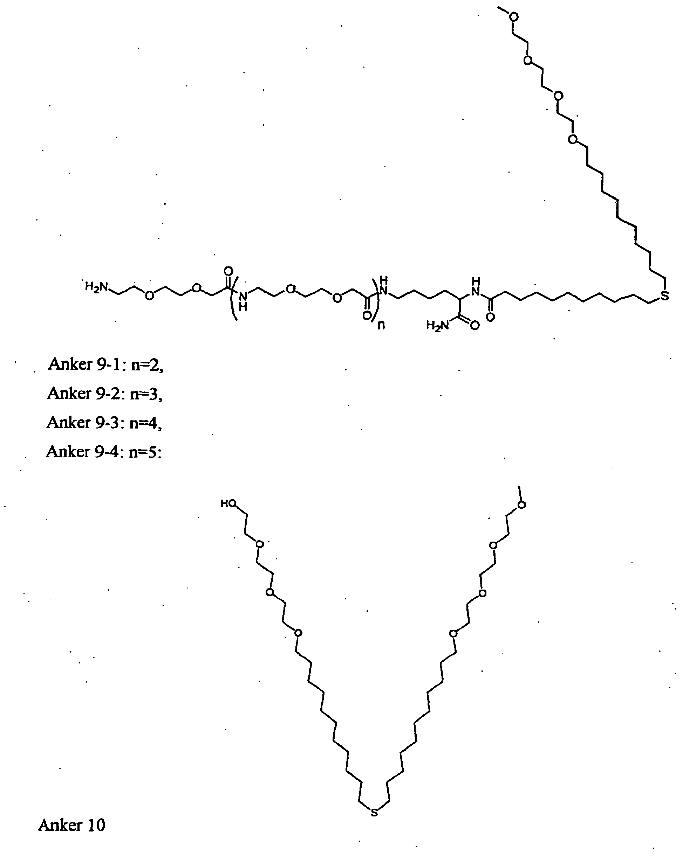

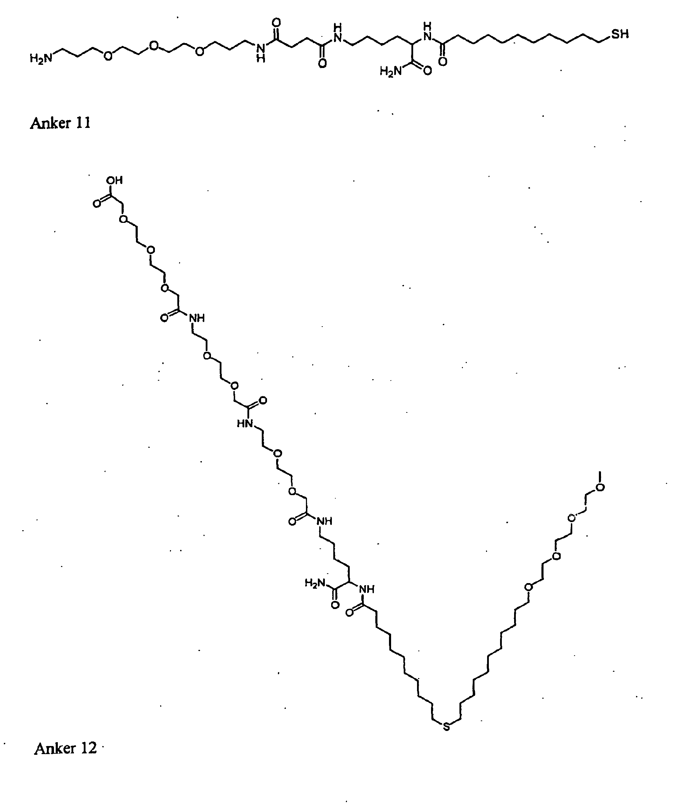

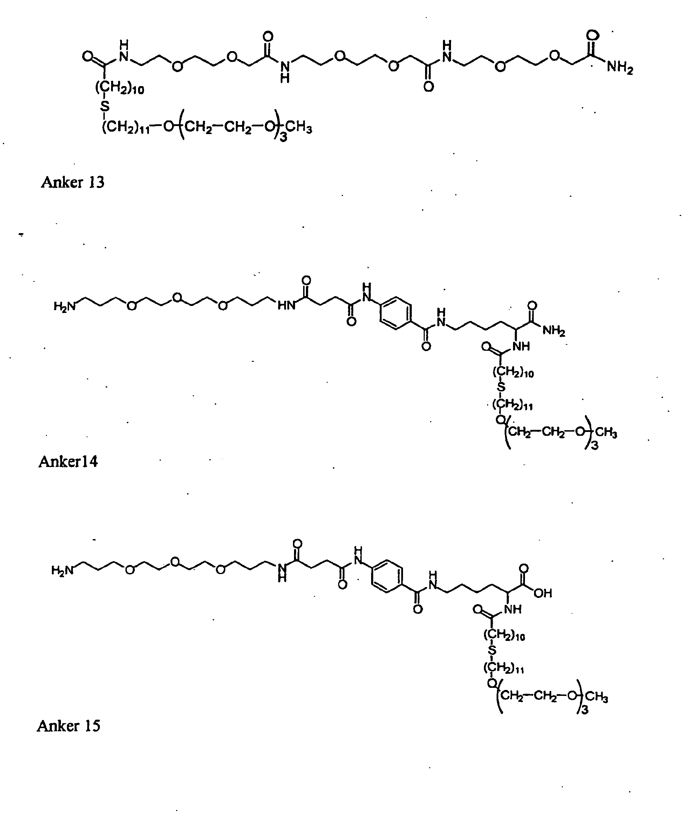

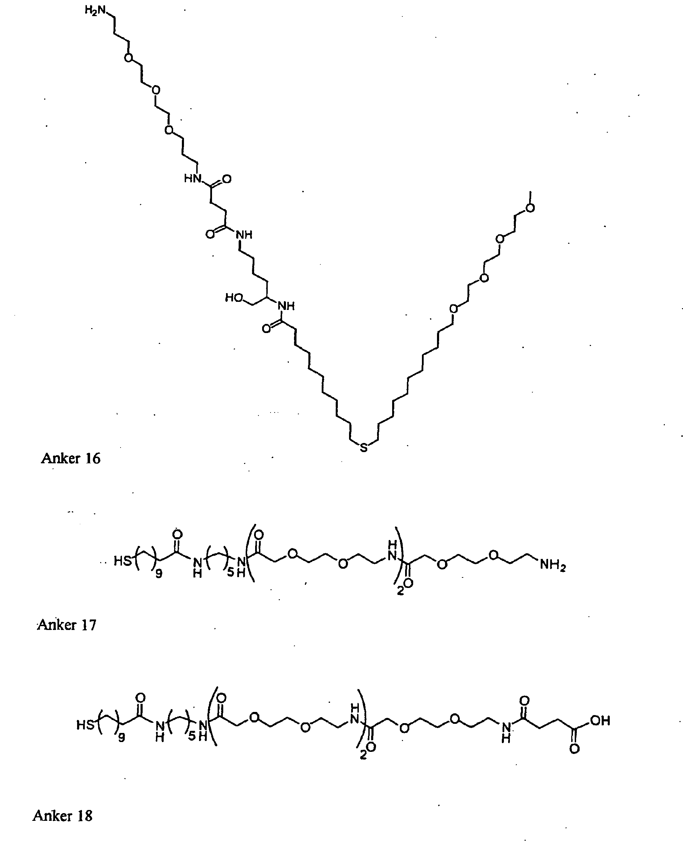

Insbesondere bevorzugt sind Kombinationen von R¹, R^{1a}, R², R^{2a}, X und Y, wie sie in Abb. 20 in den Ankerstrukturen 1-3, 8-10, 12 und 14-16 zu finden sind.

Ein weiterer bevorzugter Aufbau von Ligand-Anker-Konjugaten nach Abb.1 ergibt sich, wenn bei der Synthese eines zweiarmigen LAK die Anknüpfung an die feste Phase an einer Stelle erfolgt, die für den Liganden bzw. Nichtliganden vorgesehen ist. Bei Abspaltung des LAK unter geeigneten Bedingungen wird anschließend die feste Phase direkt durch einen Nichtliganden ersetzt. Der Aufbau des Ankers erfolgt in diesem Fall nicht in einer konvergenten Synthese sondern linear ("straight forward"). Für den Ankeraufbau gelten ebenso die oben genannten Ausführungen zu R¹, R^{1a}, R², R^{2a}, X und Y. Insbesondere bevorzugt sind in diesem Fall Kombinationen von R¹, R^{1a}, R², R^{2a}, X und Y, wie sie in den Ankerstrukturen 4-7 (Abb. 20) in den Beispielen zu finden sind.

Im Rahmen der Festphasensynthese von Verdünnerkomponenten, d.h. Ankern die ausschließlich Nichtliganden tragen, ist es ebenfalls möglich, die Anknüpfung an die feste Phase anstelle eines Nichtliganden durchzuführen. Hierbei handelt es sich dann ebenfalls um eine lineare Synthesestrategie. Für den Ankeraufbau gelten die oben genannten Ausführungen zu R¹, R^{1a}, R², R^{2a}, X und Y. Insbesondere bevorzugt sind in diesem Fall Kombinationen von R¹, R^{1a}, R², R^{2a}, X und Y, wie sie in den Ankerstrukturen 10 und 13 (Abb. 20) zu finden sind.

Unter den Strukturen gemäß Abb. 1 sind mit dem erfindungsgemäßen Verfahren auch Thiolanker darstellbar. Für den Ankeraufbau gelten hier die oben genannten Ausführungen zu R¹, R², X und Y. Insbesondere bevorzugt sind in diesem Fall Kombinationen von R¹, R², X und Y, wie sie in der Ankerstruktur 11 (Abb. 20) zu finden ist. Anker 17 und 18 in dieser Abbildung illustrieren bevorzugte Strukturen für den Fall Y = H.

Die Synthese eines Verdünnners, die nicht nach dem erfindungsgemäBen Verfahren dargestellt wird, ist in Beispiel 2 dargestellt.

Bei Verwendung der oben beschriebenen SAM bildenden Anker, die mit einer geeigneten Gruppe X versehen sind, genügt es, die LAK nach Abspaltung von der festen Phase mit dem Trägermaterial in Kontakt zu bringen, um die einsatzbereite Meßoberfläche zu erhalten. Dabei kann der Träger z.B. in einer wäßrigen Lösung der LAK inkubiert werden, oder solche Lösungen können gezielt auf abgegrenzte Bereiche der Trägeroberflächen aufgebracht werden, z.B. durch Plotting-Verfahren. So wird der parallele Einsatz von LAKs ermöglicht, die unterschiedliche Liganden tragen. Alternativ können auch Gemische verschiedener LAKs eingesetzt werden.

Um gezielt Bereiche auf der Messoberfläche eines Sensors zu erzeugen, die die Bindung eines Rezeptors ermöglichen, während gleichzeitig Bereiche der Oberfläche nicht für die Detektion aktiv sind, können zusätzlich Anker aufgebracht werden, die keine Liganden tragen und ausschließlich mit Resten L^{N} gesättigt sind. Solche sog. "Verdünnerkomponenten" können auch dazu eingesetzt weiden, die Liganden auf der Oberfläche räumlich zu isolieren, um bei Vorliegen eines sterisch ausladenden Rezeptors eine passive Bedeckung immobilisierter Interaktionspartnern zu vermeiden. Wie für Thiole gezeigt wurde, spielt die Ligandendichte (LAK-Dichte) eine wichtige Rolle bei der molekularen Erkennung von Rezeptoren (B.T. Houseman, M. Mrksich, Angew. Chem. 111 (1999), 876-880). Vergleichbares gilt für Sulfide. Um eine optimale Wechselwirkung zwischen Ligand und Rezeptor zu gewährleisten, müssen jeweils angepasste Mischungen von LAK und Verdünner hergestellt und auf der Trägeroberfläche präsentiert werden. Dies ist zuverlässig nur möglich, wenn beide vorher gemischt wurden. Hierin liegt ein weiterer Vorteil des erfindungsgemäßen Verfahrens, da ein Aufbau der LAK auf der Trägeroberfläche keine gleichmäßigen Verdünnungen gewährleisten kann.

In einer bevorzugten Anwendungsform umfasst der Sensor eine Trägerplatte, die eine Vielzahl regelmäßig angeordneter, positionsadressierbarer Felder zur Immobilisierung von LAK aufweist. Werden zum Zweck der Interaktionsanalyse verschiedene Liganden zu Molekülbibliotheken kombiniert, so können jedem Feld der Trägerplatte LAK mindestens eines Ligandentyps zugeordnet werden. Mit Hilfe einer solchen Messanordnung, auf der in wohldefinierter Weise unterschiedliche Liganden immobilisiert wurden, kann dem Analyten eine Vielzahl unterschiedlicher Liganden in Form eines Arrays präsentiert werden. Somit besteht die Möglichkeit, eine große Anzahl an (verschiedenen) Biomolekillen bzw. Rezeptoren gleichzeitig einer Detektion biospezifischen Bindungsverhaltens zu unterziehen. Mit einer solchen Parallelisierung eng verbunden ist die gleichzeitige Miniaturisierung der Testanordnung und Automatisierung des Testablaufs.

Werden ebene Träger verwendet, so besteht zwischen den Feldern keine flüssigkeitsbegrenzende Barriere. Die auf die Goldfelder z.B. mittels herkömmlicher. Microplotting-Verfahren aufgebrachten Flüssigkeitströpfchen oder -filme, die die LAK enthalten, sollten in diesem Fall so dimensioniert sein, daß kein Übersprechen von Flüssigkeit erfolgen kann. Dies muss- auch dann noch berücksichtigt werden, wenn der Träger vorstrukturiert wurde, indem beispielsweise eine Goldschicht mittels Sputtertechnik oder Bedampfen abgeschieden und diese Schicht mittels Photolithographie und Ätztechniken in einzelne Segmente unterteilt wird.

US-A-5,670,322 beschreibt eine Vorrichtung, bei der kleine Kammern mittels üblicher photolithographischer Ätztechniken erzeugt werden, die beispielsweise mit Gold beschichtet sein können. Vorrichtungen dieser Art oder oberflächenbeschichtete Mikrotiterplatten (bestehend aus PP oder PS) weisen einerseits die gewünschten Flüssigkeitsbarrieren auf, haben jedoch andererseits senkrechte Seitenwände, die bei einer Beschichtung mittels Sputter- oder Bedampfungstechilologien nicht vollständig mit Gold versehen werden. Diese unbeschichteten Stellen können dann leicht unspezifisch mit Analyten (Rezeptoren), beispielsweise Prtiteinen/Biomolekülen, belegt werden. Dies sollte jedoch bei der Detektion biospezifischen Bindungsverhaltens weitestgehend vermieden werden, um ein günstiges Signal-Rausch Verhältnis zu erhalten.

Werden in der vorliegenden Erfindung strukturierte Trägerplatten als Sensoroberflächen eingesetzt, so weisen diese daher bevorzugt eine Vielzahl regelmäßig angeordneter, positionsadressierbarer Felder zur Immobilisierung von LAK auf, wobei diese Felder in Kavitäten mit nur geringer Tiefe lokalisiert sind. Damit wird eine Flüssigkeitsbarriere bereitgestellt, gleichzeitig wird die Oberfläche so gering wie möglich gehalten. Weiterhin beinhalten diese Felder eine Schicht des Materials, das die Anbindung der LAK ermöglicht. Vorzugsweise besitzen die Kavitäten eine Tiefe von 20-100 *µ*m, und die LAK werden auf ihrem Boden immobilisiert, der dann z.B. von einem Metall oder Metalloxid, bevorzugt von einem Edelmetall wie Gold gebildet wird.

Mit Hilfe so ausgestalteter Felder gelingt es, Nachteile bezüglich der unspezifischen Bindung sowie des Übersprechens, wie sie in bisher angewendeten Verfahren autftraten, zu vermeiden oder zu minimieren. Zudem kann die Herstellung einer solchen Trägerplatte dadurch kostengünstig gestaltet werden, daß Verfahren und Materialien der Photolithographie und Ätztechniken, wie sie in der Halbleitertechnik angewendet werden, zum Einsatz kommen.

Im folgenden werden bevorzugte Ausfühnmgsformen einer solchen Trägerplatte unter Bezugnahme auf die Abbildungen näher erläutert.
Abb. 21 bis 23 zeigen jeweils einen schematischen Ausschnitt bevorzugter Trägerplatten im Querschnitt.
Abb. 24 bis 26 zeigen CCD-Aufnahmen lumineszenzmarkierter Rezeptoren, die in Wechselwirkung mit auf Trägerplatten immobilisierten Liganden getreten sind.

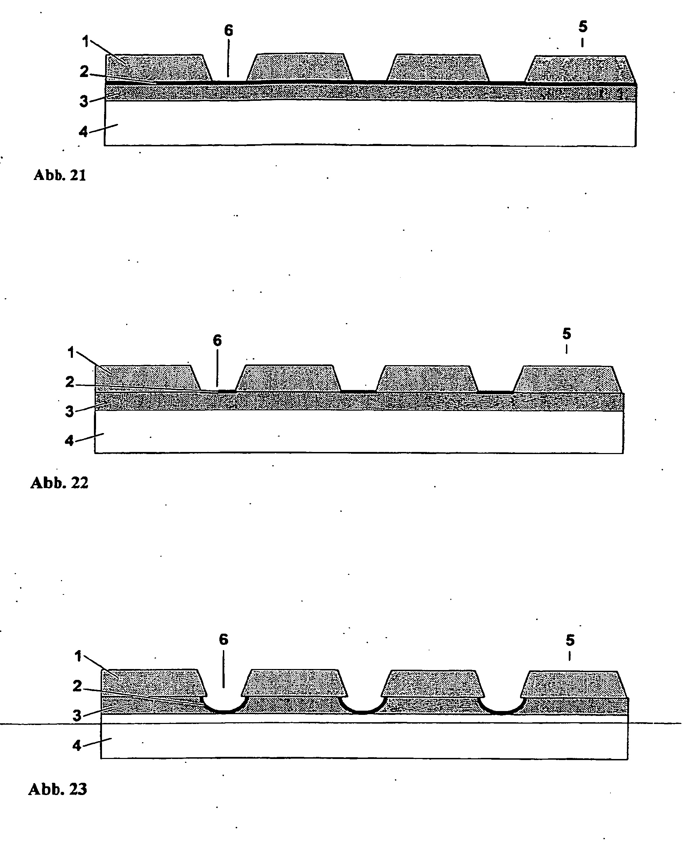

Zur Herstellung einer Trägerplatte (5) nach Abb. 21 kann von einem kupferkaschierten Basismaterial (4) ausgegangen werden, das bevorzugt bereits eine Metallschicht (3), wie Kupfer, trägt und welches in einem galvanischen Abscheideprozeß mit besagter Trägerschicht (2) versehen wird. Die Dicke der Trägerschicht beträgt nur wenige Mikrometer, genau die notwendige Stärke, um eine geschlossene Schicht herzustellen. Nach der Galvanik wird die Platte mit einer UV-belichtbaren Schutzschicht (1) versehen. Dafür können entweder in der Halbleiterfertigung übliche Photoresists oder andere UV-belichtbare und damit strukturierbare Schutzlacke verwendet werden. Die verwendeten Lackschichten weisen vorzugsweise Dicken von 20 *µ*m - 100 *µ*m auf. In einem Belichtungsschritt wird ein Bild einer Maske in die Schutzschicht projiziert. Die Maske weist vorzugsweise runde oder rechteckige/quadratische Muster auf. Nach einem Entwicklungsschritt entstehen in der Schutzschicht definierte Öffnungen, die die darunterliegende Trägerschicht freilegen. Die photostrukturierbare Schutzschicht bildet somit nach der Strukturierung gleichzeitig die Wandung der Kavitäten (6) und somit die Form der Kavität (6) und deren Öffnung.

Wird das Aufbringen und Strukturieren der Schutzschicht (1) dem Aufbringen der Trägerschicht (2) vorangestellt, so erhält man einen Träger (5) gemäß Abb. 22.

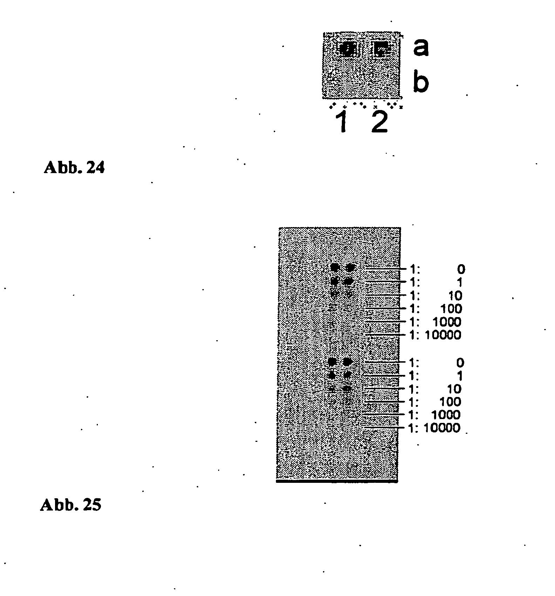

Abb. 23 zeigt eine Trägerplatte-(5) mit tieferen Kavitäten (6), wobei jedoch der Anteil an ungeschützter Wandfläche nicht erhöht wird. Diese Trägerplatte weist bevorzugt ein Basismaterial (4) auf, auf dessen Oberfläche eine metallische Beschichtung (3) vorgesehen ist, die wiederum mit einer Schutzschicht (1) versehen ist, wobei in der Schutzschicht (1) und der Metallschicht (3) mindestens eine Kavität (6) ausgebildet ist, die im Bereich der Metallschicht (3) muldenförmig ist und mit einer Trägerschicht (2) versehen ist und die im Bereich der Schutzschicht (1) in Richtung zur Mulde konisch verjüngend ausgebildet ist, wobei der untere Rand des in der Schutzschicht (1) vorgesehenen Kavitätenabschnitts einen kleineren Durchmesser aufweist als der obere Rand des in der Metallschicht (3) ausgebildeten Kavitätenabschnitts.

Die Herstellung einer Trägerplatte wie sie in Abb. 23 gezeigt ist geht ebenfalls von einer beschichteten Platte aus. In diesem Fall ist jedoch die Dicke der schon vorhandenen Schicht (3) vorzugsweise 100 *µ*m - 150 *µ*m. Mittels eines Photoresists (in der Abbildung nicht dargestellt) wird die Schicht (3) so strukturiert, daß diese bereits Vertiefungen aufweist. Diese Platte wird im Anschluß galvanisch mit der Trägerschicht (2) beschichtet. In einem zweiten Photolithographieschritt wird dann ebenfalls eine Schutzschicht (1) so strukturiert, daß sich über den in Schicht (3) geätzten Kavitäten (6) eine Struktur in der Schutzschicht (1) ausbildet. Die Tiefe der gebildeten Kavität (6) setzt sich hier aus der Tiefe der geätzten Struktur und der Dicke der Schutzschicht (1) zusammen.

Bevorzugt sind die Kavitäten derart angeordnet, daß auf der Trägerplatte ein regelmäßiges, vorzugsweise kartesisches Raster von Spalten und Streifen entsteht. Die Größe und Form der Trägerplatte kann beliebig gewählt und leicht an das verwendete Detekdonssystem angepaßt werden. Bei Verwendung von Tüpfelrobotern für die Immobilisierung der LAK, oder bei Vorliegen der LAK auf Mikrötiterplatten, ist der Abstand der Felder zueinander bevorzugt dem verwendeten Mikrotiterformat bzw. der verwendeten Tüpfeleinrictitung anzupassen. Die Anzahl der Felder kann die Anzahl der Untereinheiten der Mikrotiterplatte auch überschreiten, d.h. es kann ein Vielfaches an Feldern pro Fläche verwendet werden. So kann beispielsweise eine quadratische Trägerplatte mit einer Seitenlänge von ca. 12 cm insgesamt 9216 Felder besitzen, die mittels eines Pipettierroboters aus sechs konventionellen 1536er Mikrotiterplatten belegt werden können.

Ein weiterer Vorteil einer solchen Tragerplatte liegt darin, daß sie durch Zersägen, Zerschneiden oder Stanzen in Segmente zerteilt Werden kann.

Eine strukturierte Präsentation gleicher oder unterschiedlicher LAK kann jedoch auch durch deren Immobilisierung auf einem räumlich abgegrenzten Abschnitt der Sensoroberfläche nach gezieltem Aufbringen eines Flüssigkeitsvolumens erreicht werden, ohne dass dazu eine physikalische Unterteilung eines Trägers in einzelne Kompartimente nötig ist. So lassen sich ebenfalls einzelne Felder der LAK auf der Oberfläche erzeugen indem Lösungen der LAK z.B. mittels Pippetierverfahren, Tüpfelverfahren, Stempelverfahren oder Ink-Jet-Verfahren gezielt aufgebracht werden, Dabei können bevorzugt Techniken, wie sie in der EP-A-0 872 735 zum Aufbringen von Reagenzspots auf Metall oder Metalloxidoberflächen beschrieben sind, in analoger Weise angewendet werden.

Dient die Sensoroberfläche bzw. die Trägerplatte zur Präsentation einer Molekülbibliothek, so werden bevorzugt unterschiedliche Typen von LAK immobilisiert, die sich von Feld zu Feld unterscheiden. Innerhalb eines Feldes können sowohl gleiche LAK als auch Mischungen unterschiedlicher LAK eingesetzt werden. Eine Trägerplatte mit den oben angegebenen Maßen kann somit dem Analyten bis zu 9216 verschiedene Liganden oder Mischungen von Liganden präsentieren.

Die mit Hilfe der erfindungsgemäßen LAK erzeugte Meßoberfläche findet vorzugsweise bei der zur elektrochemischen und spektroskopischen Messung von molekularen Interaktionen zwischen immobilisierten Liganden und nicht immobilisierten Interaktionspartnern, insbesondere Biomolekülen Verwendung. Sie kann damit auch vorteilhaft im medizinisch-diagnostischen Bereich eingesetzt werden.

Aufgrund ihrer vorstehend beschriebenen Vorteile können diese Oberflächen jedoch auch in klassischen Verfahren, wie der Affinitätschromatographie, Anwendung finden.

Als Rezeptor füngierende Moleküle sind bevorzugt in biologischen Systemen auftretende oder mit diesen, interagierende Moleküle, wie Proteine, DNA, RNA, Oligonucleotide, prosthetische Gruppen, Vitamine, Lipide, Mono- Oligo- und Polysaccharide, aber auch synthetische Moleküle wie z.B. Fusionsproteine und synthetisierte Primer.

Zur Detektion einer Bindung des Rezeptors an die Sensoroberfläche stehen bekannte massensensitive und/oder optische Methoden zur Verfügung. Bevorzugt kommen optische Verfahren, wie zum Beispiel die SPR-Spektroskopie oder die Chemolumineszenzmessung zum Einsatz.

### Synthesebeispiele:

### Beispiel 1: 24,27,30,33-Tetraoxa-12-thia-tetratriacontansäure

### 1.1 (rac)-Terahydro-2-pyranyl-(11-brom-1-undecyl)ethr

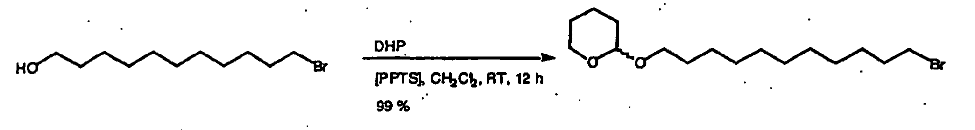

25.1 g (100 mmol) 11-Brom-1-undecanol, 12.6 g (150 mmol) Dihydropyran und 2.5 g (10 mmol) Pyridinium-*p*-toluolsulfonat wurden in 700 ml. Dichlormethan für 12 h bei Raumtemperatur gerührt. Anschließend wurde mit . Diethylether verdünnt und mit halbkonzentrierter Natriumchlorid-Lösung ausgeschüttelt. Nach Trocknen über Natriumsulfat und Entfernen des Lösungsmittels wurden 33.2 g (99 mmol, 99 %) DC-reines Produkt als gelbliches Öl erhalten.
R_{f} = 0.42 (Kieselgel, *c*-Hexan/Essigsäureethylester =9:1)
¹H-NMR (500 MHz, CDCl₃, 303 K): δ = 4.56 (t, 1H), 3.86 (dt, 1H), 3.72 (dt, 1H), 3.47 - 3.51 (m, 1H), 3.36 - 3.40 (m, 3H), 1. 79 -1.88 (m, 3H), 1.68 -1.73 (m, 1H), 1.38 - 1.64 (m, 6H), 1.39 - 1.44 (m, 2H), 1.23 =1.37 (m, 12H).

### 1.2 (rac)-Tetrahydro-2-pyranyl-(12,15,18,21-tetraoxa-1-docosyl)ether

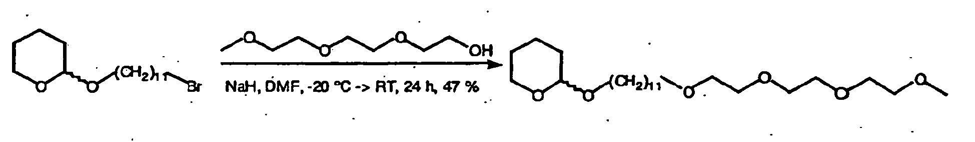

Unter Argon wurden zu einer auf -20 °C gekühlten Suspension von 1.84 g (76.5 mmol) Natriumhydrid in 150 ml *N*,*N*-Dimethylformamid eine Lösung von 12.6 g (76.5 mmol) Triethylenglycolmonomethylether in 50 ml *N,N-*Dimethylformamid getropft. Nach 15 min Rühren bei -20 °C wurde eine Lösung von 25.2 g (75.0 mmol) (*rac*)-Tetrahydro-2-pyranyl-(11-brom-1-undecyl)ether in 50 ml *N*,*N*-Dimethylformamid innerhalb von 45 min zugetropft. Der Reaktionsansatz wurde im Dewar-Gefäß über Nacht ohne weitere Kühlung gerührt und erwärmte sich hierbei auf Raumtemperatur. Das Lösungsmittel wurde anschließend am Rotationsverdampfer entfernt und der Rückstand mit 500 ml Dichlormethan aufgenommen. Nicht gelöste Salze wurden abfiltriert und die Lösung dreimal mit je 150 ml Wasser ausgeschüttelt. Nach Trocknen über Natriumsulfat und Entfernen des Lösungsmittels wurde an 400 g Kieselgel mit c-Hexan/Essigsäureethylester (4:1 → 2:1) chromatographiert. Es konnten 14.7 g (35.0 mmol, 47 %) DC-reines Produkt als gelbliches Öl isoliert werden.
R_{f}= 0.37 (Kieselgel, *c*-Hexan/Essigsäureethylester=1:1)
¹H-NMR (500 MHz, CDCl₃, 303 K): 8 = 4.53 (t, 1H), 3.86 (dt, 1H), 3.68 (dt, 1H), 3.59 - 3.64 (m, 8 H), 3.50 - 3.55 (m, 4H), 3.44 - 3.48 (m, 1H), 3.41 (t, 2H), 3.35 (dd, 1H), 1.76 - 1.85 (m, 1H), 1.63 - 1.70 (m, 1H), 1.45 -1.58 (m, 8H), 1.22 -1.34 (m, 14H).

### 1.3 12,15,18,21-Tetraoxa-1-docosanol

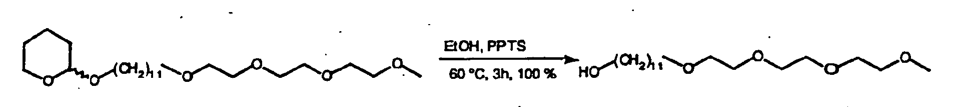

Es wurden 14.7 g (35.0 mmol) (*rac*)-Tetrahydro-2-pyranyl-(12,15,18,21-tetraoxa-1-docosyl)ether in 300 ml Ethanol gelöst, mit 1 g (4 mmol) Pyridinium-*p*-toluolsulfonat versetzt und bei 60 °C für 3 h gerührt. Nach vollständigem Umsatz (DC-Kontrolle) wurde das Lösungsmittel am Rotationsverdampfer entfernt. Der Rückstand wurde in 300 ml Diethylether aufgenommen, der hierin unlösliche Katalysator wurde abfiltriert und das Lösungsmittel am Rotationsverdampfer entfernt. Das Produkt wurde in quantitativer Ausbeute als farbloses Öl erhalten.
R_{f}= 0.14 (Kieselgel, *c*-Hexan/Essigsäureethylester=1:1)
¹H-NMR (500 MHz, CDCl₃, 303 K): δ = 3.55 - 3.60 (m, 8 H), 3.53 (t, 2H), 3.46 - 3.50 (m, 4H), 3.38 (t, 2H), 3.23 (s, 3H), 2.19 (bs, 1H), 1.44 - 1.52 (m, 4H), 1.19 - 1.29 (m, 14H).

### 1.4 12,15.18.21-Tetraoxa-1-docosyl-p-toluolsulfonsäureester

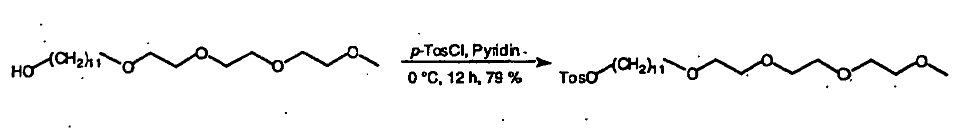

11.7 g (35.0 mmol) 12,15,18,21-Tetraoxa-1-docosanol wurden in 150 ml Pyridin gelöst und auf 0 °C gekühlt. Hierzu wurden langsam 14.3 g (75.0 mmol) *p*-Toluolsulfonylchlorid gegeben. Der Reaktionsansatz wurde über Nacht bei 4 °C stehengelassen, wobei Pyridiniumchlorid in Form langer Nadeln ausfiel. Das Ende der Reaktion wurde mittels DC-Kontrolle ermittelt. Der gesamte Ansatz wurde unter Rühren auf 500 g Eis gegossen und anschließend mehrfach mit Diethylether extrahiert. Die vereinigten organischen Phasen wurden dreimal mit kalter 1 M Salzsäure und dreimal mit kaltem Wasser gewaschen. Nach Trocknen über Kaliumcarbonat/Natriumsulfat wurde das Lösungsmittel auf ca. 50 ml eingeengt und diese Lösung über 100 g Kieselgel mit Dichlormethan als Eluens filtriert. Nach Entfernen des Lösungsmittels wurden 13.5 g (27.6 mmol, 79 %) Produkt als farbloses Öl erhalten.
R_{f} = 0.39 (Kieselgel, *c*-Hexan/Essigsäureethylester = 1:1)
¹H-NMR (500 MHz, CDCl₃, 303 K): δ = 7.77 (d, 2H), 7.32 (d, 2H), 4.00 (t, 2H), 3.51 - 3.68 (m, 12 H), 3.42 (t, 2H), 3.36 (s, 3H), 2.42 (s, 3H), 1.59 - 1.65 (m, 2H), 1.51 - 1.59 (m, 2H), 1.19 - 1.31 (m, 14H).

### 1.5 24,27,30,33-Tetraoxa-12-thia-tetratriacontansäure

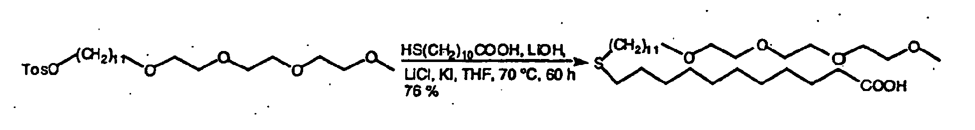

Es wurden 10.7 g (450 mmol) Lithiumhydroxid und 18.9 g (450 mmol) . Lithiumchlorid in 450 ml Tetrahydrofuran suspendiert und 15 min bei Raumtemperatur gerührt. Hierzu wurden zuerst 17.6 g (80.6 mmol) 11-Mercaptoundecansäure gegeben und nach weiteren 15 min Rühren noch 18.0 g (110 mmol) Kaliumiodid. Zu diesem Gemisch wurden eine Lösung von 10.0 g (20.5 mmol) 12,15,18,21-Tetraoxa-1-docosyl-p-toluolsulfonsäuieester in 50 ml Tetrahydrofuran gegeben. Der. Reaktionsansatz wurde unter DC-Kontrolle bis zum vollständigen Umsatz (ca. 60 h) unter Rückfluß erhitzt. Nach Abkühlen auf Raumtemperatur wurde mit ca. 40 ml 32 %iger Salzsäure auf pH = 2 angesäuert. Anschließend wurde das Lösungsmittel am Rotationsverdampfer entfernt, der Rückstand mit Dichlormethan aufgenommen und nicht gelöste Salze abfiltriert. Das hierbei erhaltene Rohprodukt wurde auf 50 g Kieselgel aufgezogen und mit *c*-Hexan/Essigsäureethylester (1:1) an 500 g Kieselgel chromatographiert. Das erhaltene Produkt wurde nochmals aus *n*-Pentan umkristallisiert. Es wurden 9.09 g (15.5 mmol, 76 %) eines weißen, feinkristallinen Pulvers erhalten.
R_{f} = 0.26 (Kieselgel, *c*-Hexan/Essigsäureethylester = 2:3)
¹H-NMR (500 MHz, CDCl₃, 303 K): δ = 3.63 - 3.67 (m, 8H), 3.54 - 3.59 (m, 4H), 3.45 (t, 2H), 3.38 (s, 3H), 2.50 (t, 4H), 2.34 (t, 2H), 1.55-1.67 (m, 8H), 1.26 - 1.41 (m, 26H).

### Beispiel 2: Bis-(12,15,18,21-tetraoxa-1-docosyl)sulfid

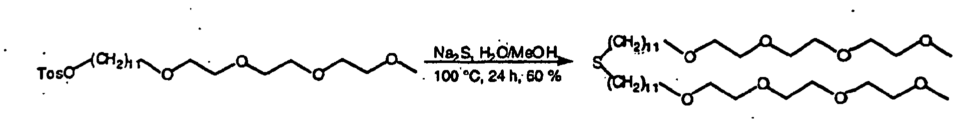

Es wurden 2.8 g (5.0 mmol) 12,15,18,21-Tetraoxa-1-docosyl-*p*-toluolsulfonsäureester und 680 mg (ca. 2.80 mmol) Natriumsulfid-Hydrat in einem Gemisch aus 40 ml Wasser und 20 ml Methanol für 24 h unter Rückfluß erhitzt. Nach vollständiger Umsetzung (DC-Kontrolle) und Abkühlen auf Raumtemperatur wurde mit Dichlormethan extrahiert, die vereinigten organischen Phasen über Natriumsulfat getrocknet und das Lösungsmittel am Rotationsverdampfer entfernt. Zur Reinigung wurde das Rohprodukt auf 15 g Kieselgel aufgezogen und mit *c*-Hexan/Essigsäureethylester (2:3) als Eluens an 180 g Kieselgel chromatographiert. Das erhaltene Produkt wurde nochmals aus *n*-Pentan bei -20 °C umkristallisiert, wobei 950 mg (1.48 mmol, 60 %) reinweißes, kristallines Produkt erhalten wurden.
R_{f} = 0.21 (Kieselgel, c-Hexan/Essigsäureethylester = 2:3)
¹H-NMR (500 MHz, CDCl₃, 303 K): δ = 3.62 - 3.66 (m, 8H), 3.54 - 3.58 (m, 4H), 3.44 (t, 2H), 3.38 (s, 3H), 2.49 (t, 2H), 1.53 -1.60 (m, 4H), 1.26 - 1.40 (m, 14H).

### Beispiel 3: Synthese von Ligand-Anker- Konjugaten (LAK) basierend auf Anker1

Die Synthese der LAK basierend auf Anker1 wurde in einer Polypropylen-Spritze mit einer Polypropylen-Fritte an TentaGel-RAM® Harz durchgeführt.
Standardzyklus für die Kupplung von N-Fmoc-geschützten Aminosäuren und von 24, 27, 30, 33-Tetraoxa-12-thia-tetratriacontansäure aus Beispiel 1:
Zur Abspaltung der Fmoc-Gruppe wurden 500 mg (0,24 mmol/g) TentaGel-RAM® Harz für 20 min mit 5 ml 20 % Piperidin in DMF geschüttelt. Anschließend wurde das Harz fünfmal mit DMF gewaschen. 5 Äquivalente der Fmoc-Aminosäure (0,48 mmol) und 5 Äquivalente (75 mg, 0,48 mmol) 1-Hydroxy-1H-benzotriazol (HOBt) wurden in 1,5 ml DMF gelöst und mit 5 Äquivalenten (72 *µ*l, 0,48 mmol) N,N'-Diisopropylcarbodiimid (DIC) versetzt. Diese Lösung wurde zum Harz gegeben und die Suspension für 60 min geschüttelt. Anschließend wurde das Harz fünfmal mit DMF gewaschen.

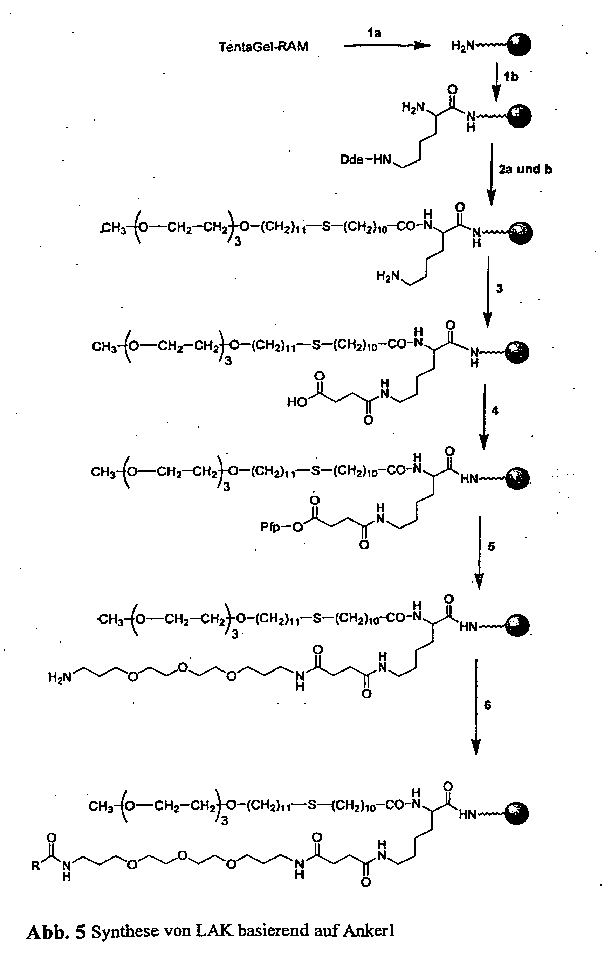

### Synthese von Anker 1(siehe Abb.5):

1a, 1b) Die Kupplung von Fmoc-Lys(Dde)-OH erfolgte analog dem Standardzyklus.
2a) Die Kupplung von 24,27,30,33-Tetraoxa-12-thia-tetratriacontansäure erfolgte analog dem Standardzyklus mit einer Kupplungszeit von 6h.
2b) Für die Abspaltung der Dde-Schutzgruppe wurde das Harz viermal für jeweils 3 min mit 2,5 % Hydrazin in DMF inkubiert. Anschließend wurde das Harz fünfmal mit DMF gewaschen.
3) Die Kupplung der Bernsteinsäure erfolgte durch Inkubation des Harzes mit 2/1/17 (w/v/v) Bernsteinsäureanhydrid/Pyridin/DMF für 60 min. Anschließend wurde das Harz fünfmal mit DMF gewaschen.
4) Die Herstellung des Pentafluorphenyl-Esters (Pfp-Ester) erfolgte durch Inkubation des Harzes mit einer Lösung von 200 µl (1,16 mmol) Trifluoressigsäure-pentafluorphenylester und 100 µl (1,24 mmol) Pyridin in 500 µl DMF für 2 h. Anschließend wurde das Harz fünfmal mit DMF gewaschen.
5) Die Kupplung von 1,13-Diamino-4,7,10-trioxatridecan erfolgte durch Inkubation des Harzes mit einer Lösung von 500 µl 1,13-Diamino-4,7,10-trioxatridecan und 50 mg HOBt in 500 µl DMF für 90 min. Anschließend wurde das Harz fünfmal mit DMF gewaschen.

### 6) Kupplung der Liganden

### 6a) Acetyl-Ankerl 1

Die Amino-Gruppe des Anker1 wurde durch Inkubation von 50 mg Harz mit einer Lösung von 50 µl Essigsäureanhydrid und 25 µl Pyridin in 300 µl DMF acetyliert. Anschließend wurde das LAK vom Harz abgespalten.

Allgemeine Vorschrift für die Abspaltung des Ligand-Anker- Konjugats vom TentaGel-RAM Harz:
Nach erfolgter Synthese wurde das Harz (50 mg) fünfmal mit DMF und dreimal mit DCM gewaschen. Dann wurde das Harz für 2 h mit 1 ml 92/4/4 (v/v/v) TFA/Triethylsilan/Wasser inkubiert und gelegentlich geschüttelt. Die Lösung wurde vom Harz abgetrennt und das Harz zweimal mit 250 µl TFA gewaschen. Die vereinigten Lösungen wurden im Stickstoffstrom eingeblasen und der Rückstand durch RP-HPLC gereinigt.
   Charakterisierung 6a):
   ESI-MS (berechnet): (M+H)⁺ 1007,1 (1006,7); (M+2H)²⁺ 504,6 (504,2)

### 6b) Acetyl-O-Phospho-tyrosyl-Anker1

Fmoc-Tyr(PO₃H₂)-OH wurde analog der Standardvorschrift unter Zugabe von 2 Äquivalenten Ethyl-Diisopropylamin gekuppelt. Nach Abspaltung der Fmoc-Schutzgruppe wurde die freie Aminogruppe analog 6a) acetyliert. Die Abspaltung des LAK erfolgte analog der Standardvorschrift.
Charakterisierung:
ESI-MS (berechnet): (M+2H)²⁺ 625,6 (625,8)

### 6c) Acetyl-Gly-Arg-Gly-Asp-Ser-Pro-Lys-Ankerl

Die Kupplung der L-Aminosäuren erfolgte nach dem Standardzyklus unter Verwendung der folgenden Aminosäurederivate: Fmoc-Gly-OH, Fmoc-Lys(Boc)-OH, Fmoc-Ser(tBu)-OH, Fmoc-Asp(OtBu)-OH, Fmoc-Arg(Pbf)-OH. Die Acetylierung erfolgte analog 6a. Die Abspaltung des LAK erfolgte analog der Standärdvorschrift. Charakterisierung:
ESI-MS (berechnet): (M+2H)²⁺ 853,2 (853,1); (M+3H)³⁺ 569,0 (569,1)

### 6d) Acetyl-(D-Phe)-Pro-Arg-Pro-Gly-Ankerl

Die Kupplung der Aminosäuren erfolgte nach dem Standardzyklus. Die Acetylierung erfolgte analog 6a. Die Abspaltung des LAK vom Harz erfolgte nach der Standardvorschrift. Charakterisierung:
ESI-MS (berechnet): (Ni+2H)²⁺782,3 (781,6); (M+2H+NH₄)³⁺ 527,1 (527,0)

### 6e) γ-Glu-Cys(StBu)-Gly-Ankerl

Die Kupplung der Aminosäuren erfolgte nach dem Standardzyklus unter Verwendung der folgenden Aminosäurederivate: Fmoc-Glu(OH)-OtBu, Fmoc-Cys(StBu)-OH und Fmoc-Gly-OH. Die Abspaltung des LAK vom Harz erfolgte nach der Standardvorschrift.
Charakterisierung:
ESI-MS (berechnet): (M+2H)²⁺ 672,0 (672,0)

Bei einem Derivat von 6e wurde vor der Abspaltung des LAK vom Harz die StBu-Schutzgruppe des Cysteins entfernt. Dazu wurde das Harz (50 mg) mit 1ml 200 mM Dithiothreitol in 3/2 (v/v) Natrium-Phosphat-Puffer, pH 7,5/Acetonitril für 2 h unter Stickstoff geschüttelt. Anschließend wurde das Harz fünfmal mit Wasser und fünfmal mit DMF gewaschen. Die Abspaltung des LAK vom Harz erfolgte nach der Standardvorschrift. Charakterisierung:
ESI-MS (berechnet): (M+2H)⁺627,8 (627,9)

### 6f) (2-Trimethylammonium-ethyl)-succinyl-Anker1 (Bernsteinsäure-Cholinester-Anker 1)

Die Kupplung der Bernsteinsäure erfolgte analog 3). Der Cholinester wurde durch Schütteln des Harzes (50 mg) mit einer Lösung von 100 mg (0,4 mmol) (2-Bromethyl)-trimethylammonium-bromid, 20 µl Ethyl-diisopropylamin in 1,2 ml DMSO für 5 h bei Raumtemperatur hergestellt. Die Abspaltung des LAK vom Harz erfolgte nach der Standardvorschrift.
Charakterisierung:
ESI-MS (berechnet): M⁺ 1149,9 (1149,8); (M+H)²⁺ 575,8 (575,8)

### Beispiel 4: Synthese von Ligand-Anker-Konjugaten (LAK) basierend auf Anker2

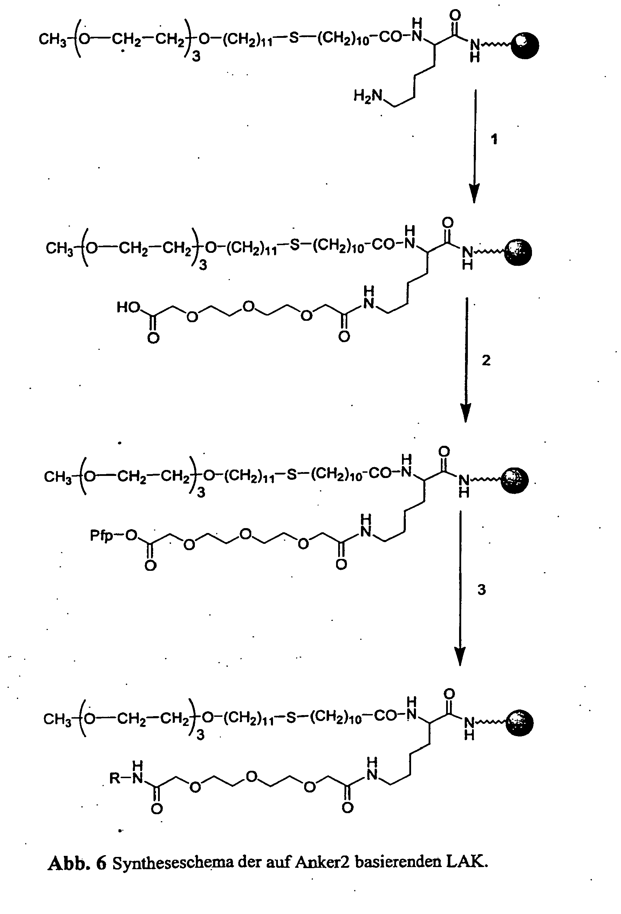

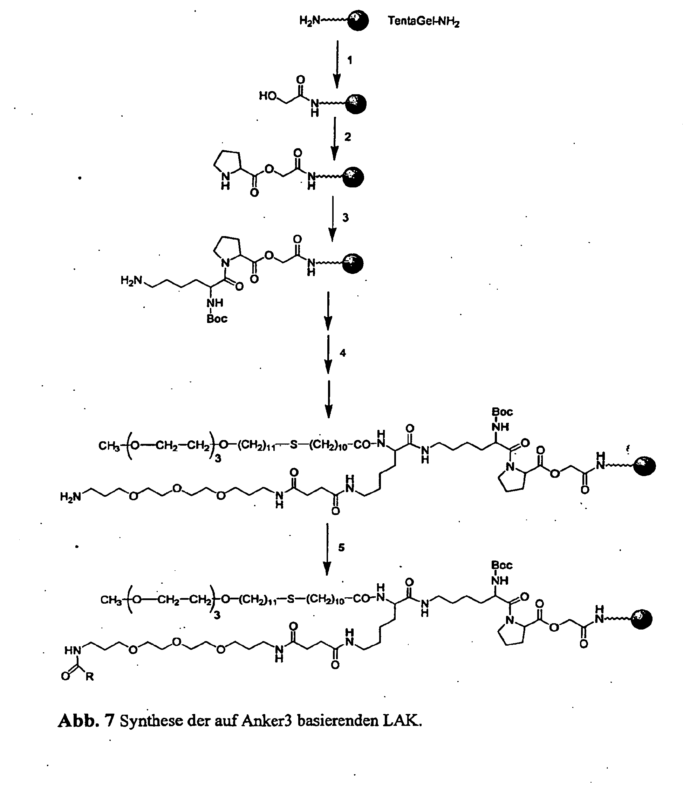

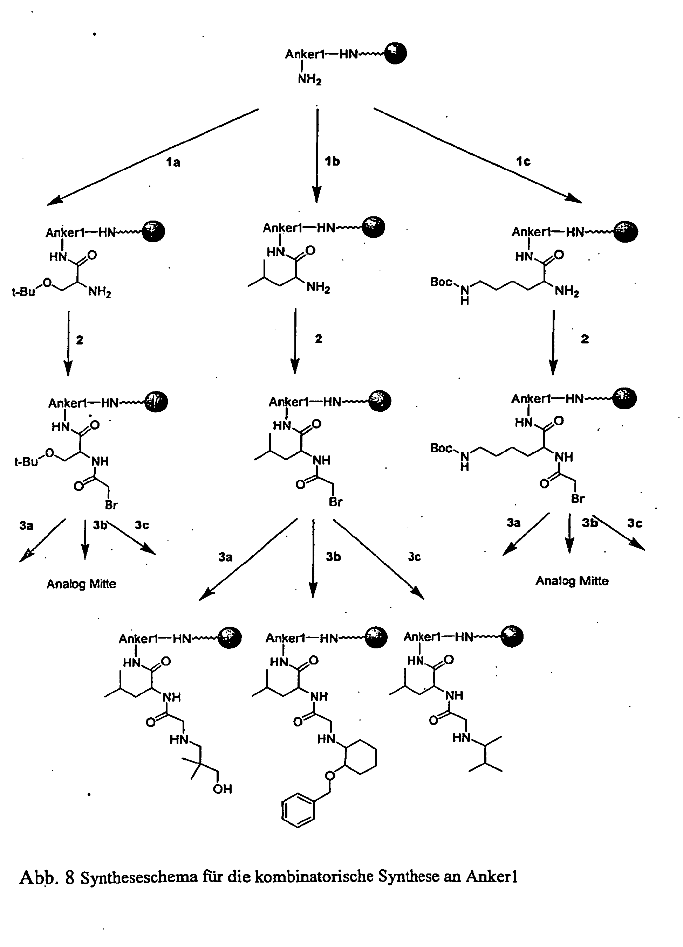

Die Synthese der auf Anker2 basierenden LAK erfolgte bis Reaktionsschritt 2b aus Beispiel 3 (Abb. 5) analog Anker1. Das Syntheseschema ist in Abbildung 6 dargestellt.
1) Die Kupplung von 3,6,9-Trioxaundecandisäure erfolgte durch Inkubation von 500 mg Harz mit 200 mg (0,9 mmol) Trioxaundecandisäure und 134 *µ*l (0,9 mmol) Diisopropylcarbodiimid in 2 ml DMF für 2 h. Anschließend wurde das Harz fünfmal mit DMF gewaschen.
2) Die Herstellung des Pfp-Esters erfolgte analog Schritt 4) aus Beispiel 3.

### 3) Kupplung der Liganden

### 3a) 2-Acetamido-1-Amino-1,2-Dideoxy-β-D-Glucopyranosyl-Anker2

50 mg Harz wurden für 1 h mit einer Lösung von 20 mg (0,09 mmol) 2=Acetamido-1-Amino-1,2-Dideoxy-β-D-Glucopyranose, 14 mg (0,09 mmol) HOBt und 16 *µ*l (0,09 mmol) Ethyldüsopropylamin in 300 *µ*l DMSO geschüttelt. Anschließend wurde das Harz dreimal mit DMSO und dreimal mit DMF gewaschen. Die Abspaltung des LAK vom Harz erfolgte analog der Standardvorschrift.
Charakterisierung:
ESI-MS (berechnet): (M+H)⁺1069,0 (1068,7); (M+H+NH₄)²⁺543,8 (543,7)

### 3b) Nα,Nα-bis(carboxymethyl)-L-εLys-Anker2

50 mg Harz wurden für 1 h mit einer Lösung von 23 mg (0,06 mmol) Nα,Nα-bis(carboxymethyl)-L-Lysin-Trifluoressigsäure-Salz, 9,36 mg (0,06 mmol) HOBt und 52 µl (0,3 mmol) Ethyldüsopropylamin in 500 µl DMSO geschüttelt. Anschließend wurde das Harz dreimal mit DMSO und dreimal mit DMF gewaschen. Die Abspaltung des LAK vom Harz erfolgte analog der Standardvorschrift.
Charakterisierung:
ESI-MS (berechnet): (M+H)⁺ 1110,1 (1110,7); (M+2H)²⁺ 556,2 (556,7)

### 3c) N⁶-Aminohexyl-adenin-Anker2

N⁶-Aminohexyladenin wurde nach D.B. Craven et al., Eur. J. Biochem. (1974), 41, 329-333 hergestellt und durch RP-HPLC gereinigt.

Die Kupplung von Aminohexyladenin erfolgte durch Inkubation des Harzes (40 mg) mit 5 mg (0,014 mmol) Aminohexyladenin-Trifluoressigsäure-Salz, 4 mg HOBt und 5 *µ*l DIEA in 200 *µ*l DMF für 2 h. Anschließend wurde das Harz fünfmal mit DMF gewaschen.
Charakterisierung:
ESI-MS (berechnet): (M+2H)²⁺ 542,2 (542,3)

### 3d) N⁶-Aminohexyl-adenosin-5'-monophosphat-Anker2

Die Synthese erfolgte analog 3c)
Charakterisierung:
ESI-MS (berechnet): (M+2H)²⁺ 648,2 (648,3)

### Beispiel 5: Synthese von Ligand-Anker- Konjugaten (LAK) basierend auf Anker3

Die Synthese der auf Anker3 basierenden -LAK erfolgte an TentaGel-NH₂® Harz.
1) Die Kupplung der Glykolsäure erfolgte durch Inkubation des Harzes (500 mg, 0,31 mmol/g) mit einer Lösung von 80 mg (1,05 mmol) Glykolsäure, 164 mg (1,05 mmol) HOBt und 157 *µ*l DIC in 2 ml DMF für 30 min. Anschließend wurde das Harz fünfmal mit DMF gewaschen.
2) Die Veresterung der Harz-gebundenen Glykolsäure mit Fmoc-Pro-OH erfolgte durch Inkubation des Harzes mit 210 mg (0,623 mmol) Fmoc-L-Pro-OH, 50 µl (0,63 mmol) N-Methylimidazol und 94 µl (0,63 mmol) DIC für 2 h. Anschließend wurde das Harz fünfmal mit DMF gewaschen, die Fmoc-Gruppe abgespalten (Siehe Beispiel 3 - Standardzyklus) und das Harz wiederum fünfmal mit DMF gewaschen.
3) Die Kupplung von Boc-L-Lys(Fmoc)-OH erfolgte nach dem Standardzyklus.
4) Die folgenden Schritte erfolgten analog der Synthese von Anker1 Schritt 1 bis 5.

### 5a) Acetyl-Anker3

Die Acetylierung erfolgte analog Schritt 6a aus Beispiel 3.

Allgemeine Vorschrift für die Abspaltung der LAK basierend auf Anker3 unter Bildung eines Diketopiperazins:
Das Harz (50 mg) mit den LAK wurde dreimal mit Dichlormethan gewaschen. Zur Abspaltung der Nα-Boc-Schutzgruppe wurde das Harz für 30 min mit 2 ml 1/1 (v/v) Trifluoressigsäure/Dichlormethan inkubiert. Anschließend wurde das Harz fünfmal mit Dichlormethan gewaschen und im Vakuum getrocknet. Dann wurde das Harz einmal mit Wasser gewaschen und anschließend für 12 h in 3/2 (v/v) 0,1 M NH₄HCO₃/Acetonitril geschüttelt. Die Lösung wurde vom Harz abgetrennt und lyophilisiert. Das LAK wurde in 1/1 Wasser/Acetonitril aufgenommen und vor der HPLC-Reinigung mit Trifluoressigsäure angesäuert.
Charakterisierung:
ESI-MS (berechnet): (M+2H)²⁺ 608,4 (608,4)

### 5b) Acetyl-O-Phosphotyrosyl-Anker3

Die Synthese erfolgte analog Schritt 6b) aus Beispiel 3.
Charakterisierung:
ESI-MS (berechnet): (M+2H)²⁺ 730,0 (729,9)

### Beispiel 6: Kombinatorische Synthese an Anker1

Als Beispiel für eine kombinatorische Synthese an Anker1 wurden, 9 LAK aus der Kombination von drei Aminosäuren (Ser, Lys, Leu) mit drei Aminen (3-Amino-2,2-dimethyl-1-propanol = Amin1; (1S,2S)-2-Benzyloxycyclohexylamin = Amin2; (S)-3-Methyl-2-butylamin = Amin3), wie in Abb. 4 gezeigt, hergestellt.
1) Die Aminosäuren a) Fmoc-L-Ser(tBu)-OH. b) Fmoc-L-Leu-OH und c) Fmoc-L-Lys(Boc)-OH wurden an jeweils 90 mg Harz analog dem Standardzyklus gekuppelt und jeweils die Fmoc-Gruppe abgespalten.
2) Die Kupplung von Bromessigsäure erfolgte durch Inkubation der drei Harze mit jeweils 41,4 mg (0,3 mmol) Bromessigsäure und 45 µl (0;3 mmol) DIC in 1 ml DMF für 30 min. Anschließend wurde das Harz fünfmal mit DMF und dreimal mit DMSO gewaschen.
3) Die drei Harze wurden jeweils in drei gleiche Portionen geteilt und die resultierenden: neun . Harzportionen wie in Abb. 4. gezeigt mit 300 µl 2 M Lösungen der drei Amine a) 3-Amino-2,2-dimethyl-1-propanol = Amin1; b) (1S,2S)-2-Benzyloxycyclohexylamin = Amin2 und c) (S)-3-Methyl-2-butylamin = Amin3 in DMSO für 2 h inkubiert. Anschließend wurden die Harze jeweils fünfmal mit DMSO und dreimal mit DMF gewaschen. Die Abspaltung der LAK erfolgte nach der Standardvorschrift wie in Schritt 6a aus Beispiel 3 beschrieben.
   Charakterisierung: ESI-MS. (berechnet)
   Amin1-Acety1-Ser-Anker1: (M+H)⁺ 1195,1 (1194,8); (M+2H)²⁺ 598,6 (598,4)
   Amin2-Acety1-Ser-Anker1: (M+H)⁺ 1296,9 (1296,9); (M+2H)²⁺ 649,7 (649,4)
   Amin3-Acetyl-Ser-Anker1: (M+H)⁺ 1179,1 (1178,8); (M+2H)²⁺ 590,6 (590,4)
   Amin1-Acetyl-Leu-Anker1: (M+H)⁺ 1221,2 (1220,9); (M+2H)²⁺ 611,7 (611,4)
   Amin2-Acetyl-Leu-Anker1: (M+H)⁺ 1322,8 (1322,9); (M+2H)²⁺ 662,7 (662,5)
   Amin3-Acetyl-Leu-Anker1: (M+H)⁺ 1205,2 (1204,9); (M+2H)²⁺ 603,6 (603,4)
   Amin1-Acetyl-Lys-Anker1: (M+H)⁺ 1236,2 (1235,9); (M+2H)²⁺ 619,2 (618,9)
   Amin2-Acetyl-Lys-Anker1: (M+H)⁺ 1337,9 (1337,9); (M+2H)²⁺ 670,3 (670,0)
   Amin3-Acetyl-Lys-Anker1: (M+H)⁺ 1220,3 (1219,9); (M+2H)²⁺ 611,2 (610,9)

### Beispiel 7: Synthese von Ligand-Anker- Konjugaten basierend auf Anker6

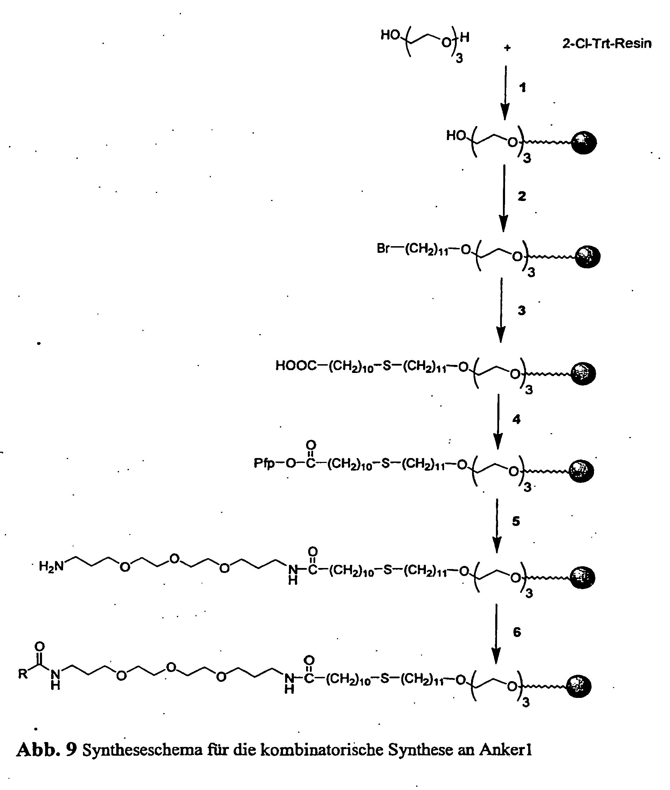

Die Synthese der LAK basierend auf Anker6 erfolgte an 2-Chlortrityl-chlorid Harz (Abb.9).
1) 500 mg (1,35 mmol/g) des 2-Chlortrityl-chlorid-Harzes wurden in einem Rundkolben mit Rückflußkühler in 500 mg (3,33 mmol) Triethylenglykol und 540 *µ*l (6,67 mmol) Pyridin in 5 ml Tetrahydrofuran suspendiert und für 6 h bei 60 °C gerührt. Anschließend wurde das Harz in eine Fritte überführt und fünfmal mit Tetrahydrofuran gewaschen.
2) 318 µl (1,35 mmol) 1,11-Dibromundecan wurden in 3 ml Tetrahydrofuran gelöst und 142 mg (0,54 mmol)18-Krone-6 und 30 mg (0,54 mmol) KOH zugegeben. 200 mg Harz wurden zugegeben und die Suspension für 16 h bei Raumtemperatur gerührt. Anschließend wurde das Harz fünfmal mit Tetrahydrofuran, fünfmal mit Wasser, dreimal mit DMF und dreimal mit Tetrahydrofuran gewaschen.
3) 235 mg (1,08 mmol) 11-Mercaptoundecansäure wurde in 4 ml Tetrahydrofuran gelöst und 78 mg (3,24 mmol) LiOH, 137,4 mg (3,24 mmol) LiCl und 170 mg (1,02 mmol) KI zugegeben. Nach Zugabe des Harzes wurde die Suspension für 16 h unter Rückfluss gerührt. Anschließend wurde das Harz fünfmal mit Tetrahydrofuran, fünfmal mit Wasser, dreimal mit DMF, dreimal mit Dichlormethan, zweimal mit Hexan gewaschen und im Vakuum getrocknet.
4) Die Aktivierung der Carbonsäure unter Ausbildung des Pentafluorphenylesters erfolgte analog Schritt 4) aus Beispiel 3.
5) Die Kupplung von 1,13-Diamino-4,7,10-trioxatridecan erfolgte analog Schritt 5) aus Beispiel 3.
6) Die Acetylierung erfolgte analog Schritt 6a) aus Beispiel 3.

Allgemeine Vorschrift für die Abspaltung des LAK vom 2-Chlortrityl-Harz:
Das Harz wurde fünfmal mit Dichlormethan gewaschen und anschließend mit 1 ml / (100 mg Harz) 46/46/4/4 (v/v/v/v) Trifluoressigsäure/Dichlormethan/Wasser/Triethylsilan für 20 min inkubiert. Die Lösung wurde vom Harz abgetrennt und das Harz zweimal mit der Abspaltlösung gewaschen. Die vereinigten Lösungen wurden zur Trockene eingeengt und der Rückstand durch HPLC gereinigt
Charakterisierung:
ESI-MS (berechnet): (M+H)⁺ 765,8 (765,6)

### Beispiel 8: Synthese von Ligand-Anker- Konjugaten basierend auf Anker7

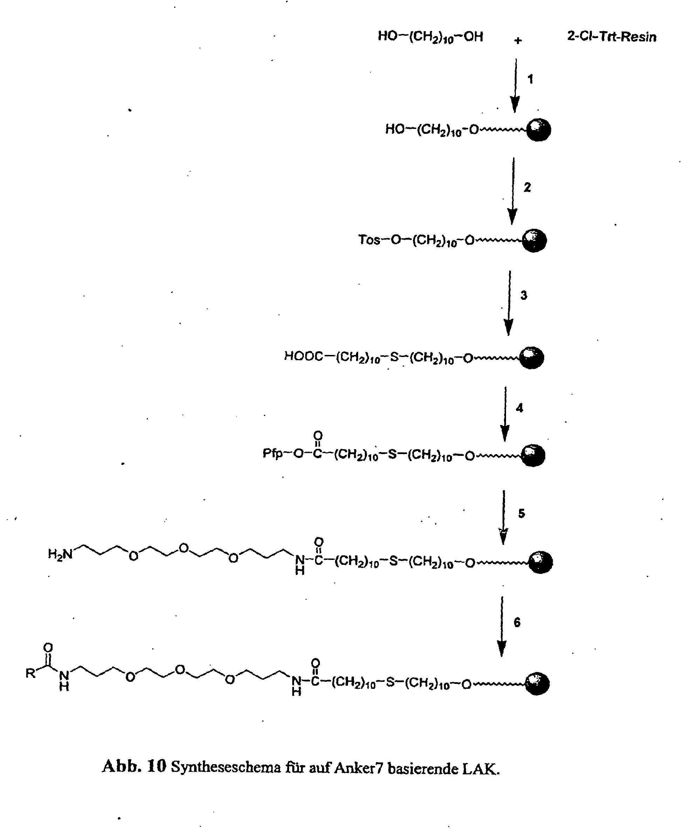

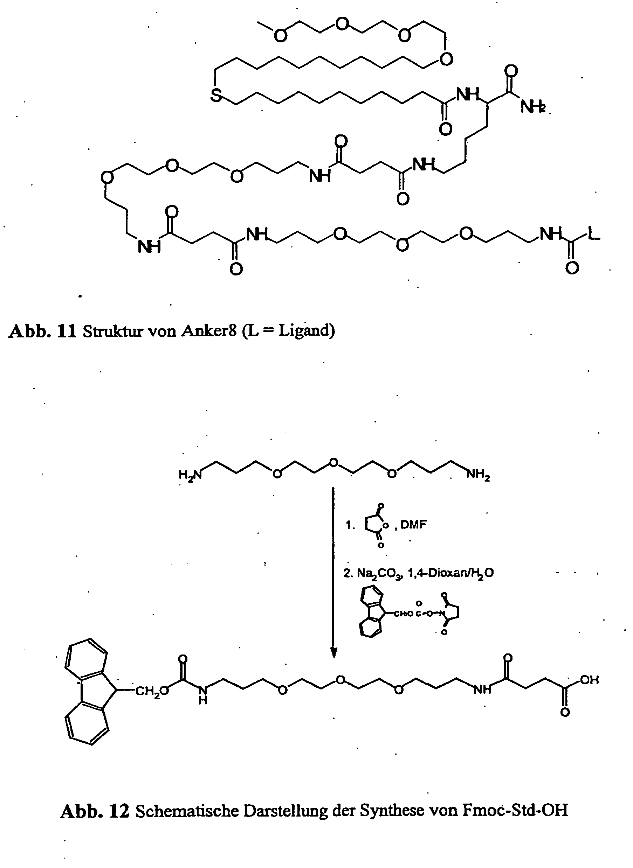

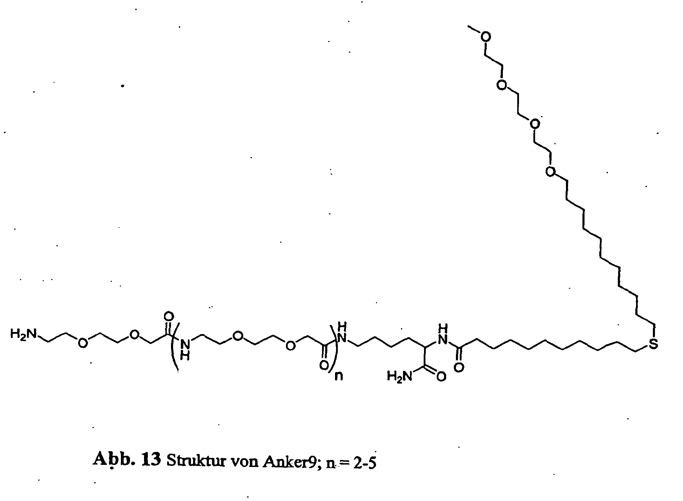

Das Syntheseschema für LAK basierend auf Anker7 ist in Abbildung 10 gezeigt.
1a) Die Beladung des 2-Chlortrityl-chlorid-Harzes mit 1,10-Decandiol erfolgte analog Schritt 1) aus Beispiel 7.
2) Die freie Hydroxylgruppe wurde durch Inkubation von 500 mg Harz mit 515 mg (2,7 mmol) p-Toluolsulfonylchlorid und 425 µl (5,4 mmol) Pyridin in 4 ml DCM tosyliert. Anschließend wurde das Harz fünfmal mit Dichlormethan und dreimal mit Tetrahydrofuran gewaschen.
   Die folgenden Schritte erfolgten analog zu den Schritten 3) bis 6) aus Beispiel 7.
   Charakterisierung:
   ESI-MS (berechnet): (M+H)⁺ 619,8 (619,5)

### Beispiel 9: Synthese von Ligand-Anker-Konjugaten basierend auf Anker8

Die Synthese der LAK basierend auf Anker8 wurde in einer Polypropylen-Spritze mit einer Polypropylen-Fritte an TentaGel-RAM® Harz durchgeführt.
Standardzyklus für die Kupplung von N-Fmoc-geschtitzten Aminosäuren und von 24,27,30, 33-Tetraoxa-12-thia-tetratriacontansäure aus Beispiel 1.

Zur Abspaltung der Fmoc-Gruppe wurden 50 mg TentaGel-RAM® (0,24 mmol/g) Harz für 20 min mit 0,5 ml 20 % Piperidin in DMF geschüttelt. Anschließend wurde das Harz fünfmal mit DMF gewaschen.
5 Äquivalente der Fmoc-Aminosäure (0,06 mmol) und 5 Äquivalente (9 mg, 0,06 mmol) 1-Hydroxy-1H-benzotriazol (HOBt) wurden in 0,15 ml DMF gelöst und mit 5 Äquivalenten (11 µl, 0,07 mmol) N,N'-DÜsopropylcarbodümid (DIC) versetzt. Diese Lösung wurde zum Harz gegeben und die Suspension für 60 min geschüttelt. Anschließend wurde das Harz fünfmal mit DMF gewaschen.

Synthese von Anker8 (analog der Synthese von Anker1):
Die Kupplung von Fmoc-Lys(Dde)-OH erfolgte analog dem Stahdardzyklus. Nach Fmoc-Abspaltung erfolgte die Kupplung von 24,27,30,33-Tetraoxa-12-thiatetratriacontansäure entsprechend dem Standardzyklus mit einer Kupplungszeit von 6 h. Für die Abspaltung der Dde-Schutzgruppe wurde das Harz viermal für jeweils 3 min mit 2,5 % Hydrazin in DMF inkubiert. Anschließend wurde das Harz fünfinal mit DMF gewaschen.

Nach der Dde-Abspaltung wurde N-Fluorenylniethoxycarbonyl-N-Succinyl-4,7, 10-Trioxatridecan-1,13-diamin (Fmoc-Std-OH), das in Lösung unter Verwendung von Bernsteinsäureanhydrid, 4,7,10-Trioxa-1,13-diaminotridecan und 9-Fluorenylmethyloxy carbonyl-N-hydroxysuccinimid hergestellt wurde, entsprechend dem Standardzyklus für Fmoc-Aminosäuren gekuppelt (2 h). Nach Fmoc-Abspaltung wurde diese Kupplung wiederholt.

### Kupplung der Liganden

### a)Acetyl-Anker8

Die Amino-Gruppe des Anker8 wurde durch Inkubation von 50 mg Harz mit einer Lösung von 50 µl Essigsäureanhydrid und 25 µl Pyridin in 300 µl DMF acetyliert. Anschließend wurde das LAK vom Harz abgespalten.
Charakterisierung:
ESI-MS (berechnet): (M+2H)²⁺655,0 (655,6)

### b) Acetyl-O-Phospho-tyrosyl-Anker8

Fmoc-Tyr(PO₃H₂)-OH wurde analog der Standardvorschrift unter Zugabe von 2 Äquivalenten Ethyl-Diisopropylamin gekuppelt. Nach Abspaltung der Fmoc-Schutzgruppe wurde die freie Aminogruppe analog a) acetyliert. Die Abspaltung des LAK erfolgte analog der Standardvorschrift.
Charakterisierung:
ESI-MS (berechnet): (M+2H)²⁺ 776,9 (776.5)

### c) Acetyl-Gly-Arg-Gly-Asp-Ser-Anker8

Die Kupplung der L-Aminosäuren erfolgte nach dem Standardzyklus unter Verwendung der folgenden Aminosäurederivate: Fmoc-Gly-OH, Fmoc-Ser(tBu)-OH, Fmoc-Asp(OtBu)-OH. und Fmoc-Arg(Pbf)-OH. Die Acetylierung erfolgte analog a). Die Abspaltung des LAK erfolgte analog der Standardvorschrift.
Charakterisierung:
ESI-MS (berechnet): (M+2H)²⁺892,1 (892,1)

### Beispiel 10: Synthese von Ligand-Anker-Konjugaten basierend auf Anker9

### Synthese von Anker9-1 (n=2) bis 9-4 (n=5):

Die Synthese der Anker9-1 bis 9-4 erfolgte analog der Synthese von Anker8 mit dem einzigen Unterschied, daß anstelle der zwei aufeinanderfolgenden Fmoc-Std-OH Kupplungen (n + 1) aufeinanderfolgende Kupplungen von Fmoc-8-amino-3,6-dioxaoctansäure (Neosystem, Strasbourg) erfolgten.

### 4) Kupplung der Liganden

### a) Acetyl-Anker9-1

Die Amino-Gruppe des Anker9-1 wurde durch Inkubation von 50 mg Harz mit einer Lösung von 50 µl Essigsäureanhydrid und 25 µl Pyridin in 300 µl DMF acetyliert. Anschließend wurde das LAK nach Standardvorschrift vom Harz abgespalten.
Charakterisierung:
ESI-MS (berechnet): (M+2H)²⁺ 570,9 (570,4)

### b) Acetyl-O-Phospho-tyrosyl-Anket9-1

Fmoc-Tyr(PO₃H₂)-OH wurde analog der Standardvorschrift unter Zugabe von 2 Äquivalenten Ethyl-Diisopropylamin gekuppelt. Nach Abspaltung der Fmoc-Schutzgruppe wurde die freie Aminogruppe analog a) acetyliert. Die Abspaltung des LAK erfolgte analog der Standardvorschrift.
Charakterisierung:
ESI-MS (berechnet): (M+2H)²⁺692,2 (691,9)

### c) Acetyl-Gly-Arg-Gly-Asp-Ser-Anker9-1

Die Kupplung der L-Aminosäuren erfolgte nach dem Standardzyklus unter Verwendung der folgenden Aminosäurederivate: Fmoc-Gly-OH, Fmoc-Ser(tBu)-OH, Fmoc-Asp(OtBu)-OH und Fmoc-Arg(Pbf)-OH. Die Acetylierung erfolgte analog a). Die Abspaltung des LAK erfolgte analog der Standardvorschrift.
Charakterisierung:
ESI-MS (berechnet): (M+2H)²⁺ 807,1 (806,5)

### d) Acetyl-Anker9-2

Die Acetylierung erfolgte analog a). Die Abspaltung vom Harz erfolgte analog der Ständardvorschrift.
Charakterisierung:
ESI-MS (berechnet): (M+2H)²⁺ 643,9 (642,9)

### e) Acetyl-Anker9-3

Die Acetylierung erfolgte analog a). Die Abspaltung vom Harz erfolgte analog der Standardvorschrift.
Charakterisierung:
ESI-MS (berechnet): (M+2H)²⁺ 715,6 (715,5)

### f) Acetyl-Anker9-4

Die Acetylierung erfolgte analog a). Die Abspaltung vom Harz erfolgte analog der Standardvorschrift.
Charakterisierung:
ESI-MS (berechnet): (M+2H)²⁺ 789,5 (788,0)

### Beispiel 11: Synthese von Ligand-Anker- Konjugaten basierend auf Anker11 (Thiolanker)

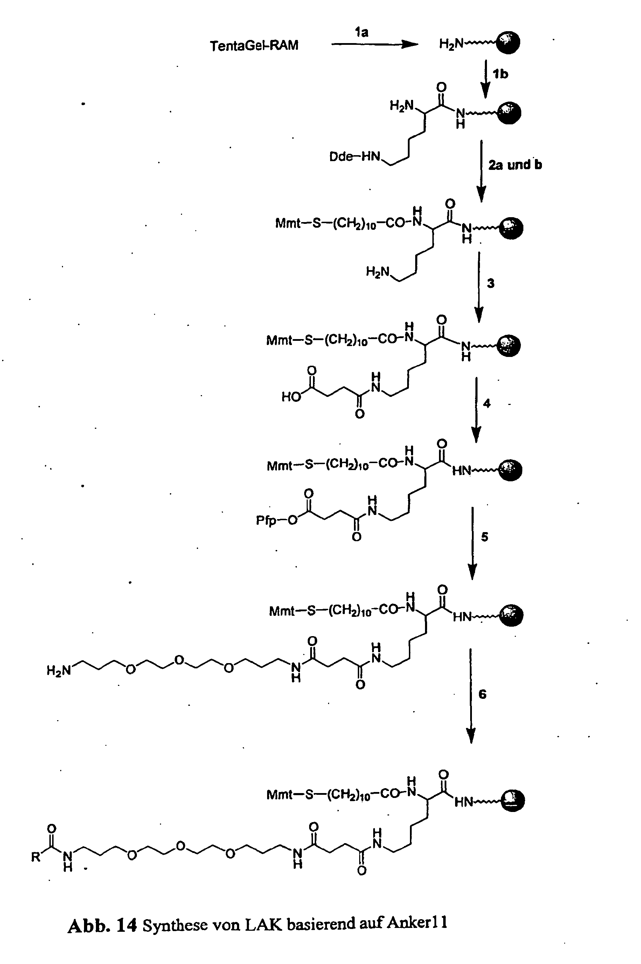

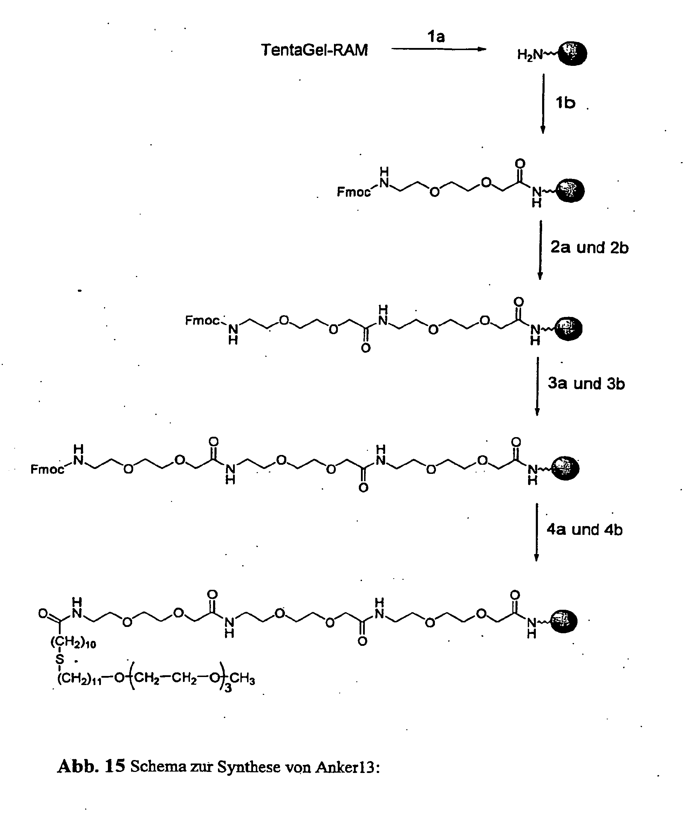

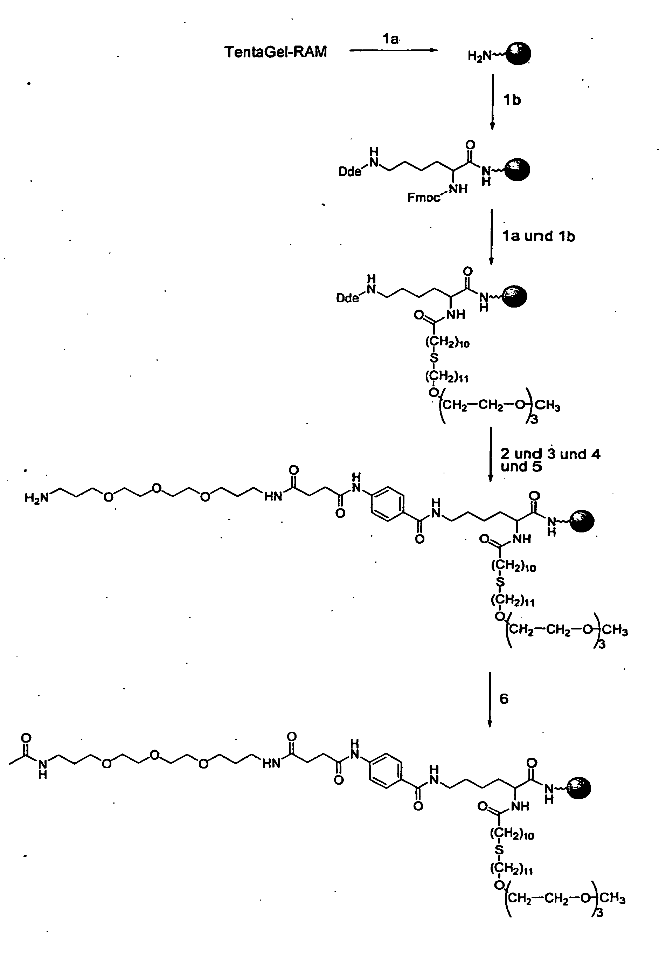

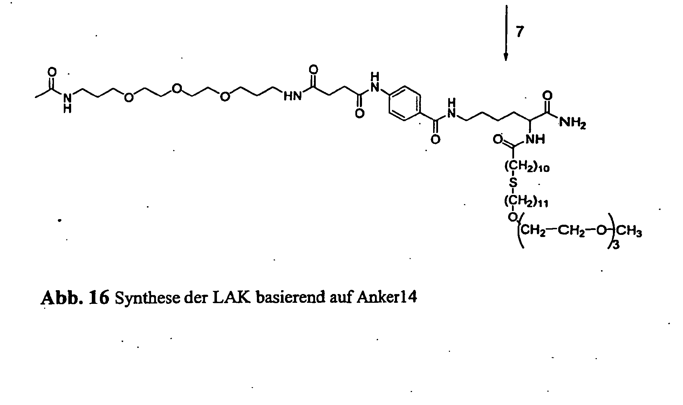

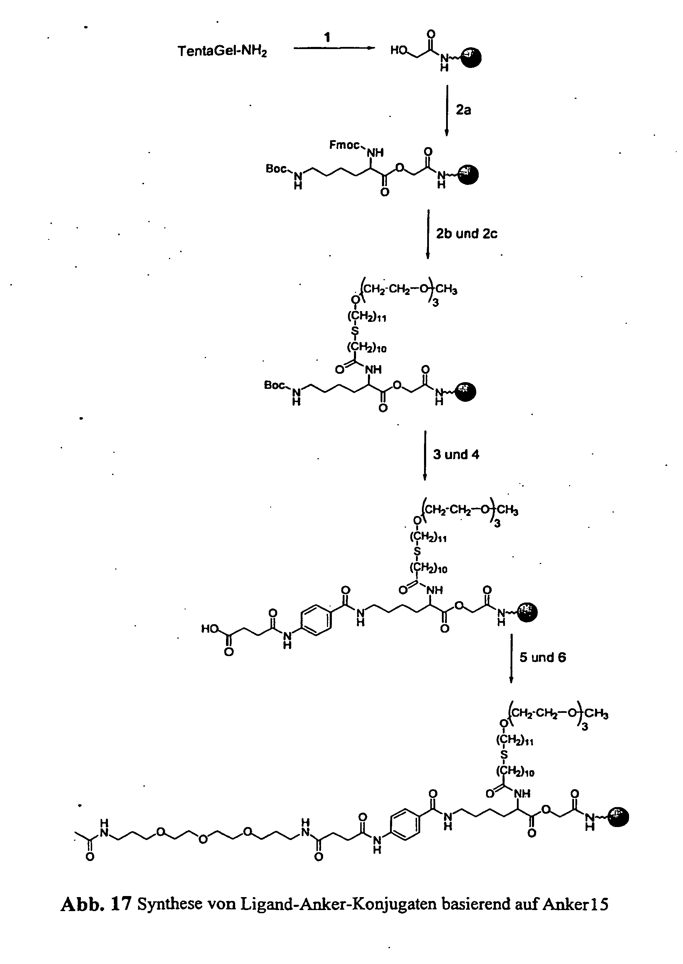

Die Synthese der LAK basierend auf Anker11 erfolgte bis Schritt 1b und ab Schritt 2b in Abbildung 14 analog den Syntheseschritten 1a bis 1b und 2b bis 6b in Beispiel 3.

### 2a) Die Kupplung von S-Mmt-11-Mercaptoundecansäure erfolgte analog dem Standardzyklus aus Beispiel 3.

11-Mercaptoundecansäure wurde mit der Mmt-Schutzgruppe analog M. Bodanszky, A. Bodanszky, The Practice of Peptide Synthesis, Springer Verlag, Berlin, 2. Auflage, 1994, S.68 geschützt.

### 6a) Acetyl-Ankerl 11

Charakterisierung:
ESI-MS (berechnet): (M+H)⁺ 690,7 (690,4).

### 6b) Acetyl-O-Phospho-tyrosyl-Anker11

Charakterisierung:
ESI-MS (berechnet): (M+H)⁺ 933,8 (933,5).

### Beispiel 12: Synthese von Ligand-Anker-Konjugaten basierend auf Anker12

### Synthese von Ankerl2:

Die Synthese von Anker12 erfolgte analog der Synthese von Anker10 bis zur 2. Kupplung von Fmoc-8-amino-3,6-dioxaoctansäure. Nach Abspaltung der Fmoc-Schutzgruppe wurde 3,6,9-Trioxaundecandisäure gekuppelt. Dies erfolgte durch Inkubation von 100 mg Harz mit 30 mg 3,6,9-Trioxaundecandisäure, 23 µl Düsopropylcarbodümid und 25 µl Ethyl-diisopropylamin in 300 µl DMF für 90 min. Anschließend wurde das Harz fünfmal mit DMF gewaschen.

### a)2,4-Diamino-6-(hydroxymethyl)-pteridin- Anker12

Die freie Carboxylat-Gruppe wurde analog Beispiel 3, 4) in den Pfp-Ester Überführt. Die Kupplung des Liganden erfolgte durch Schütteln von 50 mg Harz mit 12 mg 2,4-Diamino-6-(hydroxymethyl)-pteridin, 15 µl N-Methylimidazol in 250 µl DMF für 2 h bei Raumtemperatur. Die Abspaltung des Ligand-Anker-Konjugats erfolgte analog der Standardvorschrift.
Charakterisierung:
LC-MS (erwartet): [M+2]²⁺ 666,6 (666,4)

### Beispiel 13: Synthese des Ankers13 als Verdünnerkomponente

Die Synthese der LAK wurde in einer Polypropylen-Spritze mit einer Polypropylen-Fritte an 50mg (0,26mmol/g) TentaGel-RAM® Harz durchgeführt.
Die Abspaltung der Fmoc-Gruppe und die Kupplung von Fmoc-8-amino-3,6-dioxaoctansäure erfolgten analog dem Standardzyklus (siehe Beispiel 3).
Die Kupplung von 24,27,30,33-Tetraoxa-12-thia-tetratriacontansäure erfolgte analog dem Standardzyklus mit einer Kupplungszeit von 90 Minuten.
Die Abspaltung des Ankers erfolgte analog der Standardvorschrift zur Abspaltung von LAK (siehe Beispiel 3).
Charakterisierung:
ESI-MS (berechnet): (M+H)⁺ 969,8 (968,6)

### Beispiel 14: Synthese von Ligand-Anker-Konjugaten basierend auf Anker14

Die Synthese der LAK wurde in einer Polypropylen-Spritze mit einer Polypropylen-Fritte an 100mg (0,26mmol/g) TentaGel-RAM® Harz durchgeführt.
1a) Die Abspaltung der Fmoc-Gruppe erfolgte analog dem Standardzyklus (siehe Beispiel 3).
1b) Die Kupplung von Fmoc-Lys(Dde)-OH und von 24,27,30,33-Tetraoxa-12-thiatetratriacontansäure erfolgte ebenfalls analog den Standardvorschriften (siehe Beispiel 3) mit einer Kupplungszeit von 90min. Für die Kupplung von Fmoc-Lys(Dde)-OH wurden noch zusätzlich 5 Äquivalente 1-Hydroxy-1H-benzotriazol hinzugegeben,
2) Für die Abspaltung der Dde-Schutzgruppe wurde das Harz viermal für jeweils 3min mit 2% Hydrazin in DMF inkubiert. Anschließend wurde das Harz fünfmal mit DMF gewaschen.
3) 6 Äquivalente der 4-Aminobenzoesäure (0,156mmol) und 6 Äquivalente (24mg, 0,156mmol) 1-Hydroxy-1H-benzotriazol (HOBt) wurden in 500µl DMF gelöst und mit 6 Äquivalenten (25µl, 0,156mmol) N.N'-Düsopropylcarbodiimid (DIC) versetzt. Diese Lösung wurde zum Harz gegeben und die Suspension für 90min geschüttelt.
   Anschließend wurde das Harz zweimal mit DMF gewaschen und die Kupplung wurde einmal wiederhoh.
   Anschließend wurde das Harz fünfmal mit DMF gewaschen.
4) Die Kupplung der Bernsteinsäure erfolgte durch Inkubation des Harzes mit 5 Äquivalenten Bernsteinsäureanhydrid , und mit 5 Äquivalenten HOBt in 750µl DMF über Nacht. Anschließend wurde das Harz fünfmal mit DMF gewaschen.
5) Für die Kupplung von 1,13-Diamino-4,7,10-trioxatridecan wurde zuerst der Pentafluorphenyl-Ester hergestellt. Dies erfolgte durch Inkubation des Harzes mit einer Lösung von 200µl (1,16mmol) Trifluoressigsäure-pentafluorphenylester und 100 µl (1,24mmol) Pyridin in 500µl DMF für 2 h. Anschließend wurde das Harz fünfmal mit DMF gewaschen.
   Die Kupplung von 1,13-Diamino-4,7,10-trioxatridecan erfolgte durch Inkubation des Harzes mit einer Lösung von 500µl 1,13-Diamino-4,7,10-trioxatridecan und 50mg HOBt in 500µl DMF über Nacht. Anschließend wurde das Harz fünfmal mit DMF gewaschen.
6) Die Amino-Gruppe von 1,13-Diamino-4,7,10-trioxatridecan wurde durch Inkubation des Harzes mit einer Lösung von 50µl Essigsäureanhydrid und 100µl Pyridin in 150µl DMF acetyliert.
7) Die Abspaltung des LAK erfolgte analog der Standardvorschrift (siehe Beispiel 3).
Charakterisierung:
ESI-MS (berechnet): (M+2H)²⁺ 564,2 (563,8)

### Beispiel 15: Synthese von Ligand-Anker-Konjugaten basierend auf Anker 15

Die Synthese der LAK wurde in einer Polypropylen-Spritze mit einer Polypropylen-Fritte an 200mg (0,25mmol/g) TentaGel-NH₂® Harz durchgeführt.
1) Die Kupplung der Glykolsäure erfolgte durch Inkubation des Harzes mit einer Lösung von 20mg (0,25mmol) Glykolsäure, 39mg (0,25 mmol) HOBt und 40µl DIC in 750µl DMF für 2 h. Anschließend wurde das Harz fünfmal mit DMF gewaschen.
2a) Für die Kupplung von Fmoc-Lys(Boc)-OH wurden 5 Äquivalente von Fmoc-Lys(Boc)-OH, 5. Äquivalente 1-Hydroxy-1H-benzotriazol (HOBt), 5 Äquivalente N,N'-Diisopropylcarbodiimid (DIC) und 5 Äquivalente N-Methylimidazol (NMI) in 750µl DMF gelöst. Diese Lösung wurde zum Harz gegeben und die Suspension für 90min geschüttelt.
2b) Die Abspaltung der Fmoc-Gruppe erfolgte analog dem Standardzyklus (siehe Beispiel 3).
2c) Die Abspaltung der Fmoc-Gruppe erfolgte analog dem Standardzyklus (siehe Beispiel 3). Die Kupplung von 24,27,30,33-Tetraoxa-12-thia-tetratriacontansäure erfolgte analog den Standardvorschriften (siehe Beispiel 3) mit einer Kupplungszeit von 90min.
2d) Das Harz wurde dreimal mit Dichlormethan (DCM) gewaschen. Für die Abspaltung der - Boc-Schutzgruppe wurde das Harz für 30min mit 750µl 50% TFA in DCM inkubiert. Anschließend wurde das Harz dreimal mit DCM und fünfmal mit DMF gewaschen.
3) 6 Äquivalente der 4-Aminobenzoesäure (0,156mmol) und 6 Äquivalente (24mg, 0,156nunol) 1-Hydroxy-1H-benzotriazol (HOBt) wurden in 750µl DMF gelöst und mit 6 Äquivalenten (25µl, 0,156mmol) N,N'-Diisopropylcarbodiimid (DIC) versetzt. Diese Lösung wurde zum Harz gegeben und die Suspension für 90min geschüttelt.
   Anschließend wurde das Harz zweimal mit DMF gewaschen und die Kupplung wurde einmal wiederholt.
   Anschließend wurde das Harz fünfmal mit DMF gewaschen.
4) Die Kupplung der Bernsteinsäure erfolgte durch Inkubation des Harzes mit 5 Äquivalenten Bernsteinsäureanhydrid und mit 5 Äquivalenten HOBt in 750 µl DMF über Nacht. Anschließend wurde das Harz fünfinal mit DMF gewaschen.
5) Für die Kupplung von 1,13-Diamino-4,7,10-tnoxatridecan wurde zuerst der Pentafluorphenyl-Ester hergestellt. Dies erfolgte durch Inkubation des Harzes mit einer Lösung von 200µl (1,16mmol) Trifluoressigsäure-pentafluorphenylester und 100µl (1,24mmol) Pyridin in 500µl DMF für 2 h. Anschließend wurde das Harz fünfinal mit DMF gewaschen.
   Die Kupplung von 1,13-Diamino-4,7,10-trioxatridecan erfolgte durch Inkubation des Harzes mit einer Lösung von 500µl 1,13-Diamino-4,7,10-trioxatridecan und 50mg HOBt in 500µl DMF über Nacht. Anschließend wurde das Harz fünfmal mit DMF gewaschen.
6) Die Amino-Gruppe von 1,13-Diamino-4,7,10-trioxatridecan wurde durch Inkubation des Harzes mit einer Lösung von 50µl Essigsäureanhydrid und 100µl Pyridin in 150µl DMF acetyliert.
7) Für die Abspaltung des LAK vom Harz wurde das Harz dreimal mit Ethanol gewaschen und anschließend mit 1ml 0,085M KOH in Wasser für 1 h inkubiert.

Die Lösung wurde vom Harz abgetrennt und das Harz wurde mit 850µl 0,1M HCl in Wasser gewaschen. Die vereinigten Lösungen wurden lyophilisiert. Das LAK wurde in 1/1 Wasser/Acetonitril aufgenommen und durch HPLC gereinigt.
Charakterisierung :
ESI-MS (berechnet) : (M+2H)²⁺ 564,7 (564,3)

### Beispiel 16: Synthese von Anker-Ligand Konjugaten basierend auf Anker 17 und 18

### 1) Immobilisierung von N-(N⁵-Fmoc-5-aminopentyl)-11-mercaptoundecanamid an Chlortrityl-Harz

600 mg (1,15 mmol) N-(N⁵-Fmoc-aminopentyl)-11-mercaptoundecanamid, erhältlich aus S-geschütztem 11-Mercaptoundecanamid und Fmoc-1,5-Diaminopentanhydrochlorid, wurden in 15 ml DMF gelöst und mit 2 g Methoxytrityl-chlorid-Harz (1,6 mmol) (Novabiochem) versetzt. Die Suspension wurde 1 h vorsichtig geschüttelt. Anschliessend wurden 500 µl Pyridin zugegeben und die Suspension weitere 3 h geschüttelt. Das Harz wurde anschliessend 5 x mit DMF, 3 x mit DCM 2 x mit Hexan gewaschen und im Vakuum getrocknet. Die Beladung des Harzes mit N-(N⁵-Fmoc-5-aminopentyl)-11-mercaptoundecanamid wurde über Fmoc-Analytik zu 0,35 mmol/g bestimmt (Ausbeute 60 % d.Th.).

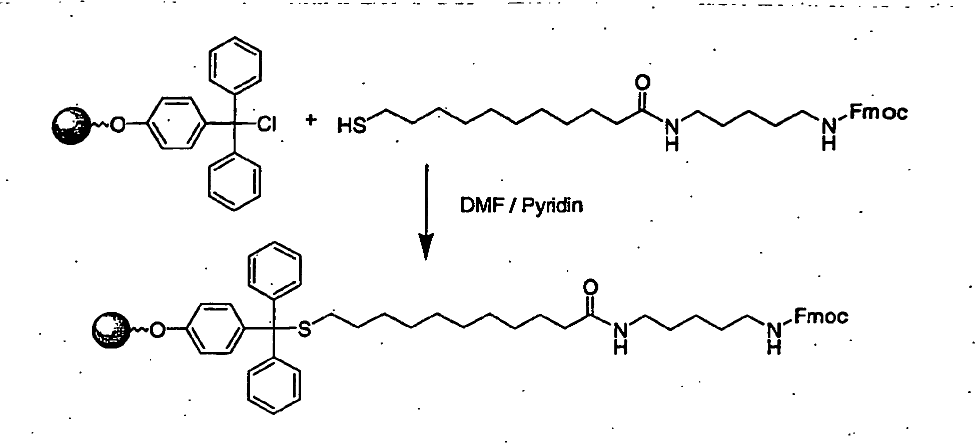

### 2) Allgemeine Vorschrift für die Kupplung von Fmoc-8-Amino-3,6-dioxa-octansäure (Fmoc-Ado)

Für die Abspaltung der Fmoc-Schutzgruppe wurde 1 g des beladenen Harzes (0,35 mmol) 20 min in 15 ml 1/3 (v/v) Piperidin/DMF vorsichtig gerührt und anschliessend 6 mal mit DMF gewaschen. Die Kupplung von Fmoc-8-amino-3,6-dioxa-octansäure erfolgte durch 4 h Inkubation des Harzes mit einer Lösung von 270 mg (0,70 mmol) Fmoc-8-aminti-3,6-dioxaoctansäure, 270 mg (0,71 mmol) HATU und 250 µl (1,44 mmol) Ethyl-diisopropylamin in 7 ml DMF. Anschliessend wurde das Harz 5 mal mit DMF, 3 mal mit Dichlormethan und 2 mal mit Hexan gewaschen und getrocknet.

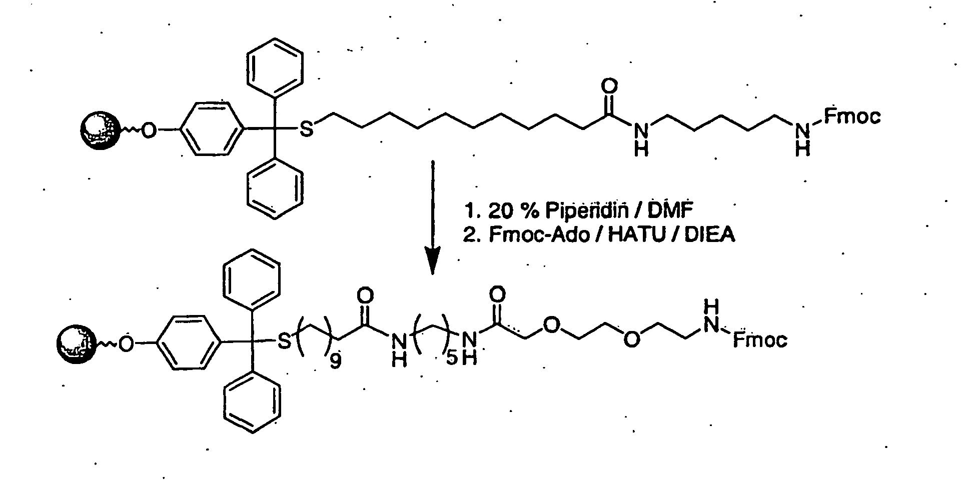

### 3) Synthese einer Verdünnerkomponente

An 500 mg Harz aus 2) (0,175 mmol) wurde wie unter Stufe 2) beschrieben Fmoc-8-amino-3,6-dioxa-octansäure gekuppelt und anschliessend die Fmoc-Schutzgruppe wie unter Stufe 2) beschrieben abgespalten. Anschliessend wurden die freien Aminogruppen durch 30 min Inkubation des Harzes mit 10 ml 1/1/2 (v/v/v) Essigsäureanhydrid/Pyridin/DMF acetyliert. Dann wurde das Harz 5 mal mit DMF und 3 mal mit Dichlormethan gewaschen. Die Abspaltung des Produktes vom Harz erfolgte mit 2/18/1 (v/v/v) Trifluoressigsäure / Dichlormethan / Triethylsilan. Das Produkt wurde durch präparative RP-HPLC gereinigt und mittels LC/MS analysiert.
LC-MS (ber.): [M+H]⁺ 635,5 (635,4), [M+Na]⁺ 657,5 (657,4)

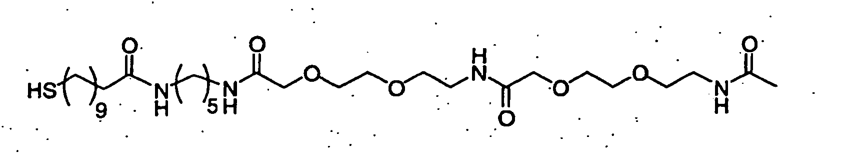

### 4) Synthese des Ankers 17

Für die Synthese des Ankers wurde an das Harz aus 2) zweimal wie unter 2) beschrieben Fmoc-8-amino-3,6-dioxa-octansäure gekuppelt und anschliessend die Fmoc-Schutzgruppe abgespalten. Anschliessend wurde das Harz 3 mal mit Dichlormethan und 2 x mit Hexan gewaschen und im Vakuum getrocknet.

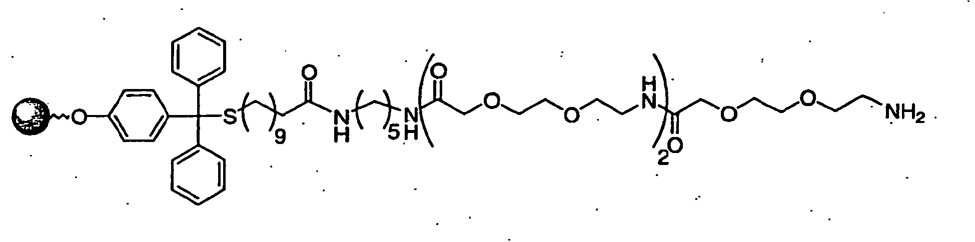

### 5) Allgemeine Vorschrift für die Kupplung von Carbonsäure-Liganden an den Anker 17 am Beispiel p-Aminobenzoesäure

Für die Kupplung einer Carbonsäure an den Anker wurde das Harz, aus 4) 1 h mit einer Lösung von 4 eq. Carbonsäure, 4 eq. Diisopropylcarbodiimid und 4 eq. 1-Hydroxybenzotriazol in DMF (c = 0,15 M) inkubiert. Dann wurde das Harz 5 mal mit DMF und 3 mal mit Dichlormethan gewaschen. Die Abspaltung des Produktes vom Harz erfolgte durch 1 h Inkubation des. Harzes mit 18/1/1 (v/v/v) Trifluoressigsäure / Wasser / Triethylsilan. Das Produkt wurde durch präparative RP-HPLC gereinigt und mittels LC/MS analysiert. Beispiel: p-Aminobenzoesäure
LC-MS (ber.): [M+H]⁺ 872,3 (872,2) [(M+2H)/2]²⁺ 436,3 (436,6)

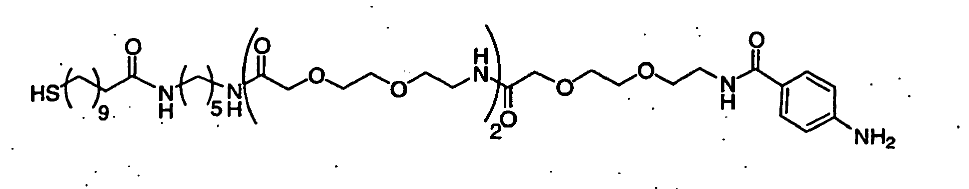

### 6) Kupplung von Aminen an den Anker am Beispiel von N⁶-(6-Aminohexyl)adenosin-2',5'-diphosphat

Anker 18 wurde erhalten durch Inkubation und 60 min Kupplung des Harzes aus 4) mit 1ml/(100 mg Harz) 2/1/17 (w/v/v) Bernsteinsäureanhydrid/Pyridin/DMF Anschließend wurde das Harz fünfmal mit DMF gewaschen.
Dann wurde der Pentafluorphenyl-Ester der freien Carbonsäure durch 1 h Inkubation von 100 mg Harz mit einer Lösung von 100 µl (0,58 mmol) Trifluoressigsäure-pentafluorphenylester und 50 µl (0,62 mmol) Pyridin in 500 µl DMF hergestellt. Anschließend wurde das Harz fünfmal mit DMF gewaschen.
Die Kupplung des Amins an den Anker erfolgte durch Inkubation des Harzes mit 500 *µ*l/ (100 mg Harz) einer Lösung von 0,1 M Amin, 0,1 M Ethyl-diisopropylamin und 0,1 M 1-Hydroxy-benzotriazol in DMSO.
Die Abspaltung des Produktes vom Harz erfolgte durch 1 h Inkubation des Harzes mit 18/1/1 (v/v/v) Trifluoressigsäure / Wasser / Triethylsilan. Das Produkt wurde durch präparative RP-HPLC gereinigt und mittels LC/MS analysiert.
Beispiel: N⁶-(6-Aminohexyl)adenosin-2',5'-diphosphat
LC-MS (ber.): [(M+2H)/2]²⁺ 674,0 (674,2)

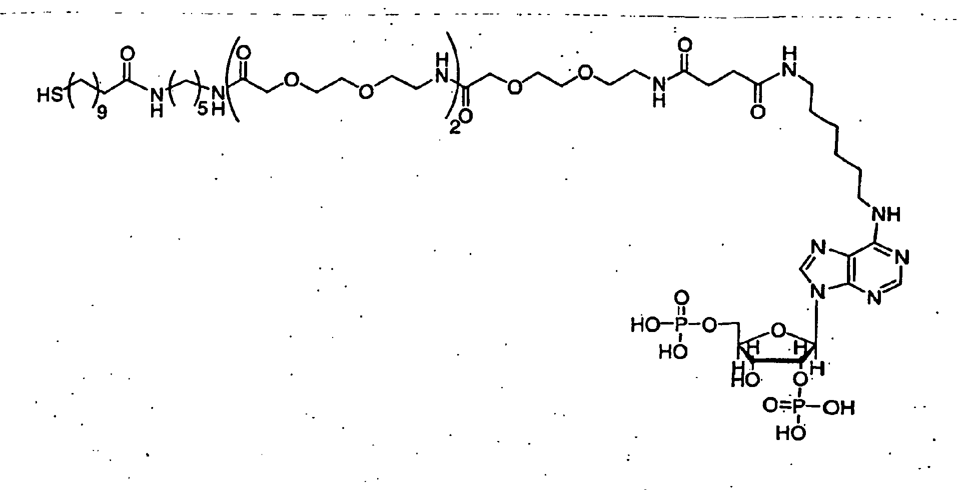

### Anwendungsbeispiele:

### Beispiel 17

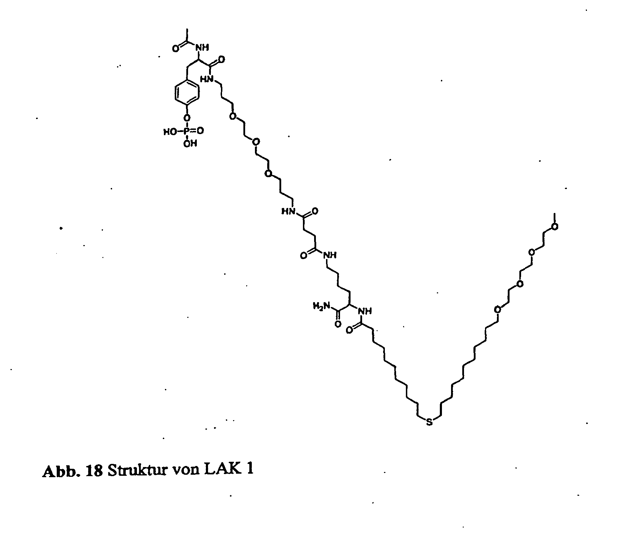

Abb. 24 zeigt die CCD-Aufnahme eines Ausschnittes von vier Feldern einer goldbeschichteten Trägerplatte als Sensoroberfläche, wie sie in Abb. 21 schematisch dargestellt ist. Die Trägerplatte umfasst insgesamt 9216 Felder. Eine Chemolumineszenzreaktion dient zur Detektion von Feldern; auf welchen eine spezifische Ligand-Rezeptor Bindung stattgefunden hat. Die Deckschicht (1) ist hierbei nicht zu erkennen.
Zwei 500x500 µm große Felder (Feld 1a und 2a in Abb. 24) der Trägerplatte (5) werden mit je 0,1 µl einer Lösung von N-Acetylphosphotyrosin, das unter Ausbildung einer Amidbindung kovalent an einen Sulfidanker gekoppelt ist (LAK 1, Abb. 18) belegt.

Auf zwei weitere Felder (1b und 2b) wird 0,1 µl einer Lösung eines identischen Ankermoleküls gegeben, welches am Amino-N-Atöm mit einem Acetylrest (Nichtligand) versehen ist und dadurch nicht an Proteine binden sollte (Verdünner 1, Abb, 19).

Die Konzentration des Anker-Ligand Konjugats bzw. des Ankermoleküls ist 1 mM in 20% HBS, 30% Ethylenglycol, 50% Acetonitril pH 7,2. Die Lösungen trocknen auf dem Goldfeld ein. Anschließend wird die Platte zur Absättigung evtl. noch freier Goldflächen in eine Lösung aus 150 mM NaCl, 5 mg/ml BSA 0,5% (w/v) Tween-20 und 50 mM Tris/HCl pH 7,3 gegeben und für 10 h bei 4°C inkubiert.
Anschließend wird die Platte in einer Lösung aus 8.6 nM anti-Phosphotyrosin (Sigma) Antikörper in 0,5% (w/v) Tween-20 und 50 mM Tris/HCl pH 7,3 gegeben und für 4 h bei 4°C inkubiert. Der anti-Phosphotyrosin Antikörper dient hier als Rezeptor im Sinne der Erfindung. Nach einem anschließenden kurzen Waschschritt in 0,5% (w/v) Tween-20 und 50 mM Tris/HCl pH 7,3 wird die Platte in eine Lösung von 0.04 U/ml anti-mouse-Fab-Fragment-Alkalische Phosphatase Konjugat (Boehringer Mannheim) gegeben und für weitere 4 h bei 4°C inkubiert. Anschließend wird die Platte in TBS gewaschen und die Detektion der Bindung wird die Platte in das ELISA-Substrat BM Chemiluminescence Elisa Substrate AP gegeben und die einsetzende Lumineszenzreaktion auf den einzelnen Meßfelden mit einem CCD basierten Lumi-Imagers (Boehringer Mannheim) verfolgt. Fig. 24 zeigt, daß die mit einem ligandentragenden Anker beschichteten Goldfelder anti-phosphotyrosin Antikörper binden. Die Spezifität der Reaktion ist daran zu erkennen, daß die Felder, welche nur mit acetylierten Anker-Molekülen beschichtet wurden, keine Antikörper binden.

### Beispiel 18

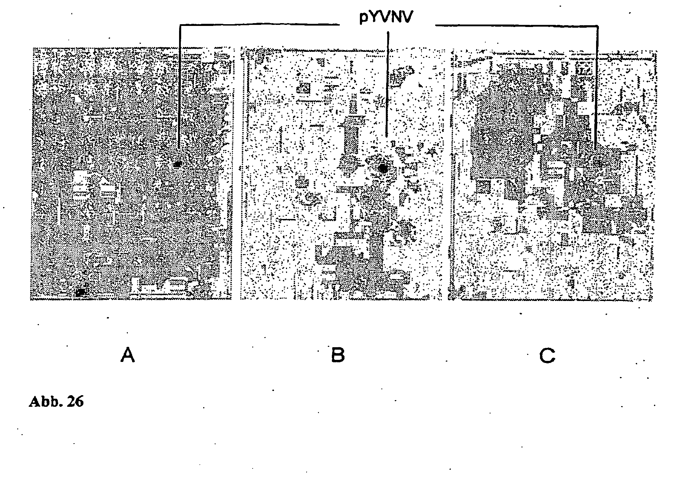

Auf zwölf Feldern einer Trägerplatte, wie sie in Beispiel 17 verwendet wurde, sind Mischungen unterschiedlicher Verhältnisse von Phosphotyrosin-Anker-Konjugat und acetyliertem Anker (vgl. Bsp. 17) aufgetragen. Die Gesamtkonzentration an ankertragenden Molekülen betrug dabei stets 1mM. In Fig. 25 ist die CCD-Aufnahme dieser Felder während der Lumineszenzreaktion zu sehen. Von oben nach unten wurde dabei das Verhältnis von ligandtragendem Anker zu acetyliertem Anker variiert. Es wurden die Mischungsverhältnisse 1:0, 1:1, 1:10, 1:100, 1:1000 und 1:10000 aufgetragen. Die Trägerplatte ist danach analog zu der in Beispiel 1 behandelt worden. Es ist zu erkennen, daß die Signalintensität mit steigendem Anteil an Phosphotyrosin-Anker Konjugat zunimmt. Die hohe Sensitivität wird dadurch deutlich, daß auch ein Verhältnis von 1:10000 des Phosphotyrosin Anker-Konjugates zu acetyliertem Anker noch ein Signal liefert, welches sich von einer Acetyl-Anker-Oberfläche unterscheidet. Der Resist 1 der Trägerplatte weist die vorteilhafte Eigenschaft auf, daß dort unter den Versuchsbedingungen weder Ankermoleküle noch unspezifische Proteinbindung auftritt und somit die Nachweisfelder deutlich getrennt voneinander vorliegen.

### Beispiel 19

Es wurde auf eine Trägerplatte, wie sie in Beispiel 17 verwendet wurde, mit 24 x 32 = 768 Feldern entsprechend den oben beschriebenen Bedingungen 487 unterschiedliche Ligand-Anker-Konjugate aufgebracht:

Fig. 26 A-C zeigt die parallele Messung der Lumineszenz mittels einer CCD-Kamera in unterschiedlichen Konzentrationen nachdem die Platte folgendermaßen behandelt wurde: Nach der Absättigüng der Trägerplatte, wie in Beispiel 17 beschrieben, wird die Trägerplatte für 4 Stunden in einer 10 nM Lösung eines Grb2-SH2-Protein A Fusionproteins (Sigma) in 150 mM NaCl, 5 mg/ml BSA, 0,5% (w/v) Tween-20 und 50 mM Tris/HCl pH 7,3 inkubiert. Nach einem kurzen Waschschritt in 150 mM NaCl, 5 mg/ml BSA, 0,5%. (w/v) Tween-20 und 50 mM Tris/HCl pH 7,3 wird die Trägerplatte in einer 1:5000 verdünnten anti goat-AP konjugierten Antikörper-Lösung (Sigma) mit 150 mM NaCl, 5 mg/ml BSA, 0,5% (w/v) Tween-20 und 50 mM Tris/HCl pH 7,3 für 90 min inkubiert. Nach zweimaligem Wäschen in TBS werden Bindungsereignisse auf der Trägerplatte durch eine im Lumi-Imager (Boehringer Mannheim) verfolgte Chemilumineszenzreaktion in BMChemiluminescence Elisa Substrate AP detektiert. Die Konzentration der Anker-Moleküle war stets bei 1 mM und das Verhältnis von ligandtragendem Anker zu acetyliertem Anker betrug in Fig. 26A 1:1, 26B 1:5 und 26C 1:10. Das markierte Feld in den Fig. 26 A-C zeigt die starke Ligand-Rezeptor-Wechselwirkung des Liganden pYVNV mit dem Enzym. Daneben sind einige andere auch spezifisch an das Protein bindende Liganden zu erkennen, welche aber eine deutlich schwächere Wechselwirkung zeigen.

Entsprechend Beispiel 18 ist die Intensität der Schwärzung auf die Konzentration der Liganden und Rezeptoren sowie auf die Stärke der Ligand-Reieptor-Wechselwirkung zurückzuführen. Es wurden folgende Liganden (Aminocarbonsäuren, Carbonsäuren oder Amine) auf den Feldern der Trägerplatte präsentiert:
Propargylamin, Cyclopropylamin, Propylamin, Ethylendiamin, Ethanolamin, Imidazol, 3-Aminopropionitril, Pyrrolidin, Glyoxylsäure-Monohydrat, Essigsäurehydrazid, L-Glycin, Glykolsäure, Pyridin, 1-Methylimidazol, Cyanessigsäure, Cyclopropancarbonsäure, (S)-(+)-3-Methyl-2-butylamin, Brenztraubensäure, N,N-Dimethylethylendiamin, N,N'-Dimethylethylendiamin, L-Alanin, Beta-L-Alanin, D-Alanin, beta-Alanin, Sarcosin, (R)-2-Amino-1-butanol, 2-Amino-1,3-propandiol, Anilin, 3-Aminopyridin, 4-Pentinsäure, 4-Pentensäure, alpha-beta-Dehydro-2-aminobuttersäure, Aminocyclopropylcarbonsäure, 3-Amino-1-propanolvinylether, (R)-(-)-Tetrahydrofurfurylamin, (S)-(+)-Prolinol, (R)-3,3-Dimethyl-2-butylamin, 1,5-Diaminopentan, gamma-Aminobuttersäure, 2-Aminobuttersäure, 2-Aminoisobuttersäure, 3-Ammo-2,2-dimethyl-1-propanol, Thiomorpholin, L-2,3-Diaminopropionsäure, D-Serin, L-Serin, 2-(2-Aminoethoxy)ethanol, (Methylthio)essigsäure; Benzylamin, 3-Chlorpropionsäure, 4-Aminophenol, Histamin, Chinuclidin, Exo-2-aminonorbornan, Cyclopentancarbonsäure, trans-1,4-Diaminocyclohexan, L-Prolin, D-Prolin, L-Allylglycin, 1-Amino-1-cyclopentanmethanol, Tetrahydro-2-furoesäure, 3,3-Dimethylbuttersäure, Succinamidsäure, L-Valin, L-Leucinol, Hydantoinsäure, L-Threonin, D-Threonin, (S)-(-)-alpha-Methylbenzylamin, 2-(2-Aminoethyl)pyridin, 5-Amino-o-cresol, p-Anisidin, Pyrazincarbonsäure, 1-(3-Aminopropyl)-imidazol, Tropan, Cyclooctylamin, L-alpha-Aminocaprolactam, 5-Oxo-L-prolin, Isonipecotinsäure, L-Pipecolinsäure, 1,4,7-Triazacyclononan, Octylamin, Dibutylamin, 4-Methyl-2-oxovaleriansäure, L-Asparaginsäure, L-Asparagin, L-Leucin, 6-Aminöcapronsäure, L-Isoleucin, L-alpha-t-Butylglycin; D-Leucin, Z-beta-Alanin, L-Ornithin, 5-Aminoindol, D-Asparaginsäure, L-Thiazolidin-4-carbonsäure, 4-Aminobenzoesäure, 3-(2-Furyl)acrylsäure, 3-Thiophenessigsäure, Cycloheptancarbonsäure, 3,5-Difluorbenzylamin, 1,4-Dioxa-8-azaspiro[4,5]-decan, N-Cyclohexylethanolamin, Caprylsäure, L-Glutamin, D-Glutamin, L-Lysin, D-Glutaminsäure, L-Glutaminsäure, 4-Cyanobenzoesäure, (S)-1,2,3,4-Tetrahydro-1-naphthylamin, 2,2,3,3,3-Pentafluorpropylamin, (1S,2R)-(-)-cis-1-Amino-2-indanol, L-Methionin, D-Methitinin, 4-Carboxybenzaldehyd, 3-Phenylpropionsäure, 4'-Aminoacetanilid, Piperonylamin, L-Phenylglycin, D-Phenylglycin, 4-(Aminomethyl)benzoesäure, 1-Adamantanamin, 4-(Hydroxymethyl)benzoesäure, (-)-cis-Myrtanylamin, (1R,2R,3R,SS)-(-)-Isopinocampheylamin, (R)-(+)-Bornylamin, 1,3,3-Trimethyl-6-Azabicyclo[3,2,1]octan, 3,5-Dihydroxybenzoesäure, 2-Norbornanessigsäure, L-2-Furylalanin, L-Histidin, D-Histidin, L-Cyclohexylglycin, Ethylpipecolinat, 5-Amino-1-naphthol, Tryptamin, 4-Aminobutyraldehyddiethylacetal, 2-Benzofurancarbonsäure, L-Indolin-2-carbonsäure, D-Phenylalanin, L-Phenylalanin, 4-Dimethylaminobenzoesäure, L-Methioninsulfoxid, 3-(4-Hydroxyphenyl)propionsäure, DL-Atrolactinsäure-Hemihydrat, 4-Sulfamoylbuttersäure, Vanillinsäure, 4-Aminobiphenyl, (R)-(+)-Citronellsäure, 4-Chlorphenylessigsäure, L-3-Thienylalanin, L-Cyclohexylalanin, D-Cyclohexylalanin, (S)-(-)-1-(1-Naphthyl)-ethylamin, 2-Chlor-6-methylnicotinsäure, L-Arginin, D-Arginin, L-4-Thiazolylalanin, 3-Pyridylessigsäure-Hydrochlorid, 3-Indolylessigsäure, 7-Amino-4-methylcumarin, L-Citrullin, 4-Benzylpiperidin, 2,4-Dichlorbenzylamin, 4-Amino-N-methylphthalimid, (-)-Cotinin, L-Tetrahydroisochinolincarbonsäure, 4-Acetamidobenzoesäure, (R)-(-)-2-Benzylamino-1-butanol, 4-Pentyloxyanilin, o-Acetylsalicylsäure, 4-Nitrophenylessigsäure, 2-Nitrophenylessigsäure, 2-Methyl-6-nitrobenzoesäure, L-Tyrosin; D-Tyrosin, L-Methionin(O2), 3-(Diethylamino)piopionsäure-Hydrochlorid, 4-Nitroanthranilsäure. 2,6-Dimethoxybenzoesäure, 3,5-Dimethoxybenzoesäure, 3,4-Dihydroxyhydrozimtsäure, 2-(4-Hydroxyphenoxy)-propionsäure, 2-Methoxyphenoxyessigsäure, 4-Hydroxy-3-methoxyphenylessigsäure, 4-.(Ethylthio)benzoesäure, S-Benzylthioglykolsäure, 4-(Methylthio)phenylessigsäure, 2-Chlorzimtsäure, 3-Chlorzimtsäure, gamma-Maleimidobuttersäure, 2,6-Dimethoxynicotinsäüre, L-4-Fluorphenylalanin, L-2-Fluorphenylalanin, (R)-(-)-Epinephrin, Cyclododecylamin, trans-2,5-Diffüorzimtsäure, DL-3,4-Dihydroxymandelsäure, Thymin-1-essigsäure, cis-Pinonsäure, 1,2-Bis(4-pyridyl)ethan, 4-tert-Butylcyclohexancarbonsäure, N,N-Diethylnipecotamid, 3,4-Difluorhydrozimtsäure, 2-Naphthylessigsäure, 3-Carboxyproxyl, 4-Chlor-o-anissäure, 4-Chlotphehoxyessigsäüre, 3-Chlor-4-hydroxyphenylessigsäure, 5-Chlor-2-methöxybenzoesäure, 4-Chlor-DL-mandelsäure, 4-(Pyrrol-1-yl)benzoesäure, 4-(Difluormethoxy)benzoesäure, Gallussäure-Monohydrat, 2,4,6-Trihydroxybenzoesäure-Monohydrat, 6-Hydroxy-2-naphthoesäure, Süberinsäure-Monomethylester, -2-Hydroxydecansäure, 2-Chlor-6-fluorphenylessigsäure, alpha-Cyano43-hydroxyzimtsäure, Indol-3-glyoxylsäüre, 8-Hydroxychinolin-2-carbonsäure, 2-Methyl-3-indolessigsäure, 4-(Trifluormethyl)benzoesäure, Cumarin-3-carbonsäure, 3-Hydroxy-2-chinoxalincarbonsäute, 4-Fluor-1-naphthoesäure, 1-Phenyl-1-cyclopentancarbonsäure, p-Toluolsulfonylchlorid, 5-Brom-2-furoesäüre, 2,5-Dichlorbenzoesäure, 3,4-Dichlorbenzoesäure, 5-Methöxyindol-2-carbonsäure, Isochinolin-3-carbonsäure-Hydrat; L-Styrylalanin, 4-(Dimethylamino)zimtsäure, 4-Oxo-2-thioxo-3-thiazolidinylessigsäure, 1-Ethyl-3-(3-dimethylaminopropyl)cärbodiimid-Hydrochlorid, 5,6-Dichlornicotinsäure, 2,6-Dichlornicotinsäure, 2,6-Dichlorpyridin-4-carbonsäure, Trimellith-Anhydrid, D-(-)-Chininsäure, trans-3,4-Methylendioxyzimtsäure, 7-Methoxybenzofuran-2-carbonsäure, trans-5-Acetoxy-1,3-oxathiolait-2-carbonsäure, 4-Benzoylbuttersäure, 4-Pentylbenzoesäure, 6-Phenylhexansäure, 2-Chlor-4,5-difluorbenzoesäure, 4-Chlor-2,5-difluorbenzoesäure, 5-Fluorindol-3-essigsäure, N-Formyl-DL-phenylalanin, 4-Diethylaminobenzoesäüre, 2-Aminoanthracen, D-Glucuronsäure, trans-Ferulasäure, (S)-(+)-o-Acetylmandelsäure, 4-Aminohippursäure, 1-Adamantanessigsäure, 6-Bromhexansäure, alpha-Hydroxyhippursäure, N-[3-(2-Furylacryloyl)]-glycin, 1-Methyl 2-aminoterephthalat, L-Serin(Bzl), 3,3,3-Trifluor-2-(Trifluormethyl)propionsäure, Diethylphosphönoessigsäure, D-Glucönsäure, 3-(4-Fluorbenzoyl)propionsäure, 2,5-Dimethoxyphenylessigsäure, Monomethyl-cis-5-norbornen-endo-2,3-dicarboxylat, 4-Hydroxy-3-nitrophenylessigsäure, 3-Methoxy-4-nitrobenzoesäure, 5-Methoxy-2-nitrobenzoesäure, 3,4,5-Trimethoxybenzylamin, DL-Hydroxy-3-methoxymandelsäüre, (-)-Camphansäure, (1R)-(+)-Camphansäure, 2-Methoxy-4-(methylthio)benzoesäure, cis-5-Dodecansäure, 4-Amino-5-carboxy-2-ethyl-mercaptopyrimidin, 4-Aminozimtsäure-Hydröchlorid, DL-3-(4-Hydroxyphenyl)milchsäure-Hydrat 4-(Methylsulfonyl)benzoesäure, 4-Carboxy-2,2,6,6-tetramethylpiperidin-1-oxyl, 2-Butylöctansäure, trans-2-Chlor-6-fluorzimtsäure, 4-Chlor-o-tolyoxyessigsäure, 2-Bromtienzoesäure, 4-Carboxybenzolsulfonamid, 2-(2-Aminothiazol-4-yl)-2-methoxyiminoessigsäure, 1-(N-t-Amino)cyclopropancarbonsäure, 2-Chlor-3-nitrobenzoesäure, 4-Chlor-3-nitröbenzoesäure, 2-Chlor-4-nitrobenzoesäure, 4-Chlor-2-nitrobenzoesäure, 4-Amino-5-chlor-2-methoxybenzoesäure, 5-Bromnicotinsäure, 6-Brompicolinsäure, 2-Methyl-5-phenylfuran-3-carbonsäure, Tributylphosphin, 2-Chlor-5-(methylthio)benzoesäure, 4,5-Difluor-2-nitrobenzoesäure, 2-Hydroxy-5-(pyrrol-1-yl)benzoesäure, Indol-3-buttersäure, 2-(Trifluormethyl)phenylessigsäure, 3-(Trifluormethyl)phenylessigsäure, 4-(Trifluormethyl)phenylessigsäure, 3,7-Dihydroxy-2-naphthoesäure, 6-methylchromon-2-carbonsäure, DL-Tryptophan, D-Tryptophan, 2,6-Dichlorphenylessigsäure, 3,4-Dichlorphenylessigsäure, 3-(Trifluormethyl)anthranilsäure, alpha-Acetamidozimtsäure, 5-Methoxyindol-3-essigsäure, DL-Indol-3-milchsäure, (1S,2S)-(-)-2-Benzyloxycycloliexylamin, 3,5-Dichloranthranilsäure,Chloramben, S-(+)-Ibüprofen, DL-Thioctansäure, 3,5-Dichlor-4-hydroxybenzoesäure, 5-Bromthiophen-2-carbonsäure, 2,3,5,6-Tetrafluor-p-toluylsäure, 2-Fluor-3-(triflüormethyl)benzoesäure, 3-Fluor-4-(trifluormethyl)benzoesäure, 5-Azido-2-nitrobenzoesäure, trans-2,3-Dimethoxyzimtsäure, N-(4-Aminobenzoyl)-beta-alanin, 4-Butoxyphenylessigsäure, 2-(2-Aminophenyl)indol, 2-Amino-3,4,5,6-tetrafluorbenzoesäure, 2-Nitrophenylbernsteinsäure, Z-Glycin, 4-(4-Nitrophenyl)buttersäure, S-(-)-2-[(Phenylamino)carbonyloxy]propionsäure, L-Threonin(Bzl), 2,6-Dichlor-5-fluor-3-pyridincarbonsäure, Trimesinsäure, (4-Formyl-3-methoxy-phenoxy)essigsäure, (E)-5-(2-Carboxyvinyl)-2,4-dimethoxypyrimidin, L-Phenylalanin(4-NO2), 2-Oxo-6-pentyl-2H-pyran-3-carbonsäure, N,N-Bis(2-hydroxyethyl)isonicotinamid; (+/-)-Jasmonsäure, epsilon-Maleimidocapronsäure, (S)-(-)-N-Benzyl-1-phenylethylamin, 2,4-Dinitrobenzoesäure, 2,4,5-Trimethoxybenzoesäure, 3,4,5-Trimethoxybenzoesäure, S-(Thiobenzoyl)thioglykolsäure, 4-Iodbuttersäure, 3-Phenoxybenzoesäure, 4-(4-Hydroxyphenyl)benzoesäure, D-Desthiobiotin, (-)-Methoxyessigsäure, 2-(o- Chlorphenoxy)-2-methylpropionsäure, 4-Bromphenylessigsäure, 3-Brom-4-methylbenzoesäure, 3-Bromphenylessigsäure, [1R-(1alpha,2beta,3alpha)]-(+)-3-Methyl-2-(nitromethyl)-5-oxocyclopentanessigsäure, L-Asparaginsäure(OCHX), L-1=Naphthylalanin, 2-(Trifluormethyl)zimtsäure, Monomethylsebacat, 5-Aminovaleriansäure, o-Carboxyphenylphosphat, 4-(Trifluormethyl)hydrozimtsäure, Monoethyl-(R)-3-acetoxyglutarat, beta-(Naphthylmercapto)-essigsäure, 3-Brom-4-flüorbenzoesäure, 3-Phthalimidopropionsäure, L-Arginin(NO2), cis-(1S,2R)-(-)-2-Benzylaininocyclohexanmethanol, 7-Hydroxycumarin-4-essigsäure, 2-Sulfobeozoesäure-Hydrat, 5-Methoxy-1-indanon-3-essigsäure, 4,7,10-Trioxa-1,13-tridecandiamin, 2,4-Dichlorphenoxyessigsäure, (S)-(+)-2-Oxo-4-phenyl-3-oxazolidinessigsäure, (S)-(-)-N-(1-Phenylethyl)succinamidsäure, 3-(Trifluormethylthio)benzoesäure, 5-(4-Chlorphenyl)-2-furoesäure, 8-Bromoctansäure, L-Asparaginsäure(OBzl), N-Acetyl-L-tyrosin, 2-Nitro-5-thiocyanatobenzoesäure, 9-Flnorenon-4-carbonsäure, Fluoren-9-essigsäure, 2-Chlor-5-(trifluormethyl)benzoesäure, 1-(4-Chlorphenyl)-1-cyclopentancarbonsäure, 3,5-DiaminobenzoesäureDihydrochlorid, N-Acetyl-4-fluor-DL-phenylalanin, 2,4,6-Trichlorbenzoesäure, 2,3,4,5,6-Pentafluorphenylessigsäure, 2,4-Dinitrophenylessigsäure, 3,4,5-Trimethoxyphenylessigsäure, Xanthen-9-carbonsäure, (R)-(+)-3-Hydroxy-5-oxo-1-cyclopenten-1-tieptansäure, 2-Bibenzylcarbonsäure, 2,2-Diphenylpropionsäure, 4-Bromzimtsäure, 4-Carboxybenzolsulfonazid, 3-Benzoyl-2-pyridincarbonsäure, trans-4-Chlor-3-nitrozimtsäure, 2,3,5,6-Tetrafluor-4-hydroxybenzoesäure-Hydrat, 3,5-Dinitrosalicylsäure, (Z)-(2-(Fortnarnido)thiazol-4-yl)(methoxyimino)essigsäure, L-Glutaminsäure-gamma-cyclohexylester, Mono-2-(methacryloyloxy)ethylsuccinat, Naproxen, L-Lysin-(ALLOC)-OH, -4-Bromrnandelsäure, 2-Brom-5-methoxybenzoesäure, L-Hydioxyprolin, 6-(Amino)hexansäure, N-tert-Butoxycarbonyl-L-leucin, 4-Brom-3,5-dihydroxybenzoesäure, N-(4-Carboxy-3-hydroxyphenyl)maleimid, 5-(2-Nitrophenyl)-2-furoesäure, 5-(3-Nitrophenyl)-2-furoesäure, N-Phthaloyl-DL-alpha-aminobuttersäure, L-Thiazolidin-4-carbonsäure, (S)-(-)-alpha-Methoxy-alphä-(trifluormethyl) phenylessigsäure, 7-Carboxymethoxy-4-methylcumarin, 3,5-Di-tert-butylbenzoesäure, 2-(2-Chloracetamido)4-thiazolessigsäure, 5-Bromorotsäure, 2-Nitro-alpha,alpha,alpha-trifluor-p-toluylsäure, Benzoyl-DL-leucin, L-Glutaminsäure(OBzl), N,N'-Dibenzylethylendiamin, L-Biphenylalanin, Diphensäure, L-4-Bromphenylalanin, Pindolol, L-Leucin-4-nitroanilid, alpha, alpha-Diphenyl-L-prolinol, L-Pentafluorphenylalanin, L-Phosphotyrosin, 4-Iodphenylessigsäure, L-Benzoylphenylalanin, Methylrot, L-Tyrosin(Bzl), Pentafluorphenyltrifluoracetat, L-Lysin(Z), R-(+)-1,1'-Binaphthyl-2,2'-diamin, (+)-Dehydroabietylamin, N-(4-Amino-2-methylphenyl)-4-chlorphthalimid, 1-Pyrenbuttersäure, Atropin, L-Phenylalanin(4-I), 4-(2,4-Di-tert-amylphenoxy)butylamin, L-Diaminopropionsäure(IVDDE), L-Lysin(DDE), L-Lysin(2-Cl-Z)-OH, L- Tyrosin(2,6-C12-BzI), 4,4'-(9-Fluorenyliden)dianilin, L-Hydroxyprolin, 4'-Carboxybenzo-18-krone-6, Cholsäure sowie Verbindungen mit folgender Struktur:

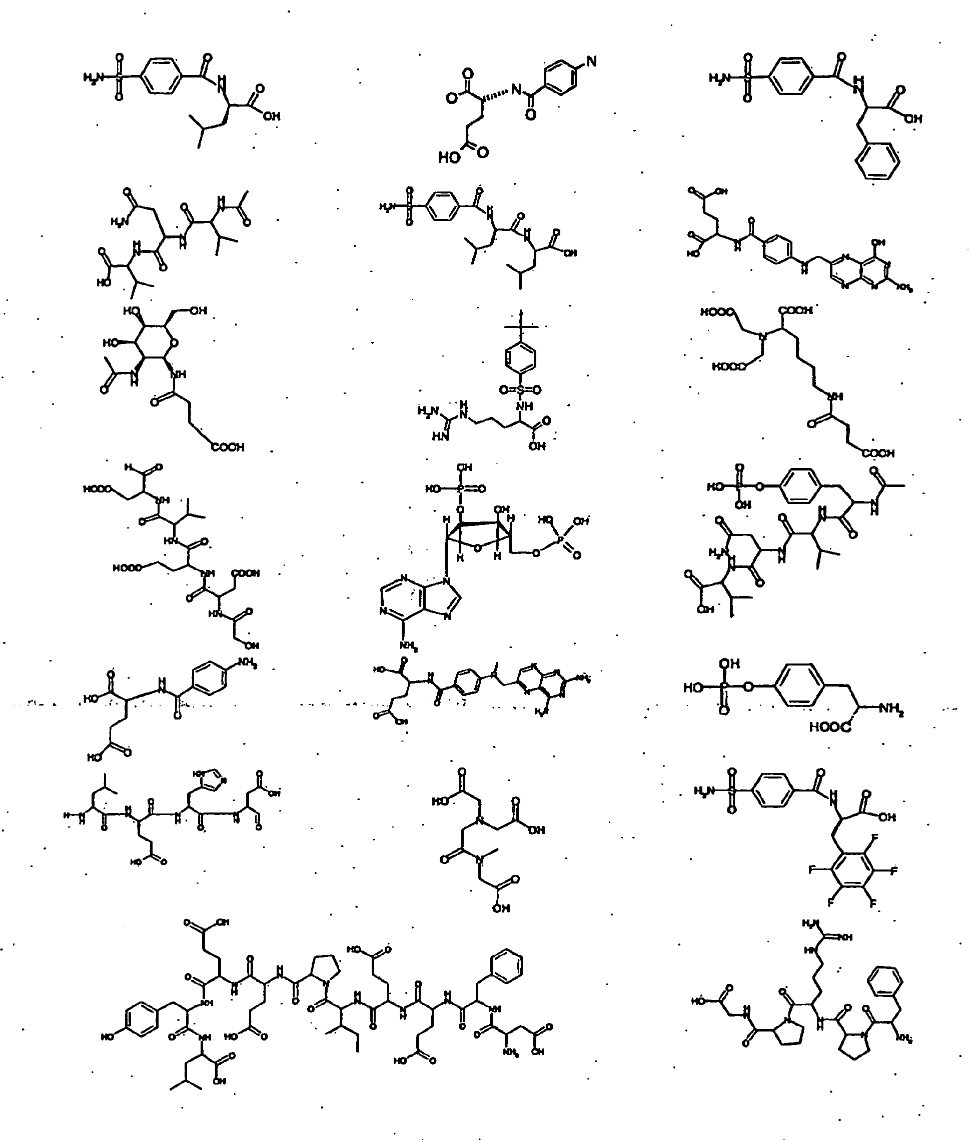

## Patentansprüche

1. Ligand-Anker-Konjugat, umfassend ein Ankermolekül zur Generierung einer biospezifischen Grenzschicht auf einer Oberfläche, umfassend mindestens eine Struktureinheit X, die zur Anbindung des Ankers an die Oberfläche geeignet ist, sowie mindestens eine Struktureinheit R, die die Ausbildung einer selbstassemblierten Monolage auf der Oberfläche ermöglicht und die zur Bildung einer Bindung mit einem Liganden oder einem Nichtliganden durch eine Gruppe A terminal funktionalisiert ist, wobei das Ankermolekül terminal mit mindestens einem Liganden verknüpft ist, der zu spezifischen Wechselwirkungen mit einem Rezeptor fähig ist

2. Ligand-Anker-Konjugat nach Anspruch 1, wobei R eine verzweigte oder unverzweigte, gegebenenfalls substituierte, gesättigte oder ungesättigte Kohlenwasserstoffkette ist, die durch Heteroatome, Aromaten und heterozyklische Verbindungen unterbrochen sein kann und 2-2000 Atome umfasst.

3. Ligand-Anker-Konjugat nach Anspruch 1 oder 2, wobei R eine hydrophobe Struktureinheit R¹ umfasst, die von einer verzweigten oder unverzweigten Kohlenwasserstoffkette von 1 bis 50 Kohlenstoffatomen, die gesättigt oder teilweise ungesättigt sein kann, gebildet wird.

4. Ligand-Anker-Konjugat nach einem der Ansprüche 1 bis 3, wobei R einen verzweigten oder unverzweigten hydrophilen Spacer R² umfasst, der durch eine mit Heteroatomen unterbrochene Kohlenwasserstoffkette von 2 bis 1000 Kohlenstoffatomen gebildet wird.

5. Ligand-Anker-Konjugat nach einem der Ansprüche 1 bis 4, wobei das Strukturelement X mindestens ein Element aus der V. oder VI. Hauptgruppe des Periodensystems umfasst.

6. Ligand-Anker-Konjugat nach Anspruch 5, wobei X eine Disulfid- eine Thiol- oder eine Sulfidgruppe ist

7. Ligand-Anker-Konjugat nach einem der Ansprüche 1 bis 6, wobei A eine Hydroxyl-, Amino- oder Carboxylgruppe ist.

8. Ligand-Anker-Konjugat nach einem der Ansprüche 1 bis 7, wobei das Ankermolekül folgende allgemeine Struktur aufweist: 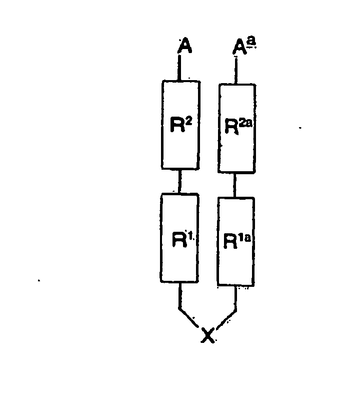 wobei die Reste R¹ und R^{1a} unabhängig voneinander wie R¹ in Anspruch 3 definiert sind, die Reste R² und R^{2a} unabhängig voneinander wie R² in Anspruch 4 definiert sind, die Gruppen A und A^{a} jeweils unabhängig voneinander wie A in Anspruch 7 definiert sind und X wie in Anspruch 6 definiert ist, und wobei eine oder zwei beliebige Struktureinheiten ausgewählt aus R^{1a}, R^{2a} und A^{a} gegebenenfalls nicht vorhanden sind oder die Kombination aus R^{1a}, R^{2a} und A^{a} vollständig durch ein Wasserstoffatom ersetzt sein kann.

9. Ligand-Anker-Konjugat nach einem der Ansprüche 3 bis 8, wobei R¹ und gegebenenfalls R^{1a} die Struktur -(CH₂)ₙ- aufweist und n eine natürliche Zahl von 1 bis 50 darstellt.

10. Ligand-Anker-Konjugat nach einem der Ansprüche 4 bis 9, wobei R² und gegebenenfalls R unabhängig voneinander einen Oligoamid- und/oder Oligoetherrest darstellen.

11. Ligand-Anker-Konjugat nach einem der Ansprüche 1 bis 10, wobei das Ankermolekül zusätzlich eine funktionelle Gruppe Y umfasst, die von der Immobilisation des Ankermoleküls an einer festen Phase herrührt.

12. Ligand-Anker-Konjugat nach Anspruch 11, wobei Y ein Carbonsäure-, Carbonsäureester-, Carbonsäureamid-, Aldehyd-, Hydrazid-, Hydroxamsäure-, Hydroxy, Hydroxyalkyl- oder Diketopiperazylrest ist

13. Ligand-Anker-Konjugat nach einem der Ansprüche 1 bis 12, wobei der Ligand ausgewählt ist aus Proteinen, Peptiden, Oligonukleotiden, Kohlehydraten, Isoprenoiden, Enzymen, Lipidstrukturen, Sacchariden, Antikörpern, Peptidhormonen, Zytokinen, Antibiotika oder organischen Molekülen mit einem Molekulargewicht ≥ 50 g/mol.

14. Ligänd-Anker-Konjugat nach einem der Ansprüche 1 bis 13, wobei das Ankermolekül zusätzlich mit einem Nichtliganden verknüpft ist.

15. Verfahren zur Herstellung eines Ligand-Anker-Konjugats, umfassend :
a) Immobilisation oder Synthese eines Ankermoleküls an einer für die chemische Synthese geeigneten festen Phase,
b) Synthese eines Liganden am Ankermolekül oder Bindung eines Liganden an das Ankermolekül und
c) Trennen des entstandenen Ligand-Anker-Konjugats von der festen Phase, wobei das Ankermolekül eine Struktureinheit aufweist, die zur Anbindung des Ligand-Anker-Konjugat an eine Oberfläche geeignet ist, sowie eine Struktureinheit R, die die Ausbildung einer selbstassemblierten Monolage auf der Oberfläche ermöglicht und die zur Bildung einer Bindung mit dem Liganden terminal funktionalisiert ist, und wobei der Ligand spezifische Wechselwirkungen der Oberfläche mit einem Rezeptor ermöglichen soll.

16. Verfahren nach Anspruch 15, wobei eine Vielzahl unterschiedlicher Ligand-Anker-Konjugate durch Verwendung kombinatorischer Methoden bei der Synthese der Liganden erzeugt wird.

17. Verfahren nach Anspruch 15 oder 16, wobei die zur Synthese eingesetzte feste Phase ein Syntheseharz, ein Synthesepolymerfilm oder eine Silizium-oder Silikatoberfläche ist.

18. Verfahren nach Anspruch 17, wobei die feste Phase ein Syntheseharz, ausgewählt aus einem Hydroxyharz, einem Aminoharz, einem Tritylharz, einem Dihydropyranharz, einem Carboxyharz oder einem Arylsiloxyharz ist.

19. Verfahren zur Bereitstellung einer biospezifischen Grenzschicht auf einer Oberfläche, umfassend die Herstellung von Ligand-Anker-Konjugaten nach einem der Ansprüche 15 bis 18, und zusätzlich das Inkontaktbringen der erhaltenen Ligand-Anker-Konjugate mit der Oberfläche.

20. Verfahren nach Anspruch 19, wobei die Oberfläche vollständig oder teilweise von Gold, Silber, Palladium oder Platin gebildet wird.

21. Verfahren nach Anspruch 19 oder 20, wobei die Oberfläche eine Vielzahl ptisitionsadressierbarer Felder aufweist, auf denen die Ligand-Anker-Konjugate immobilisiert werden.

22. Verfahren nach Anspruch 21, wobei die Ligand-Anker-Konjugate eine Molekülbibliothek bilden, bei der sich die eingesetzten Liganden von Feld zu Feld unterscheiden.

23. Verwendung von Ligand-Anker-Konjugaten nach einem der Ansprüche 1 bis 14 zur Bereitstellung eines Biosensors.

## Claims

1. A ligand-anchor conjugate, comprising an anchor molecule for generating a biospecific boundary layer on a surface, comprising at least one structural unit X, which is capable of immobilizing the anchor on the surface, as well as at least one structural unit R, which enables formation of a self-assembled monolayer on the surface and is terminally functionalized by a group A for binding to a ligand or a non-ligand, wherein the anchor molecule is terminally bonded to at least one ligand which is capable of specific interaction with a receptor.

2. The ligand-anchor conjugate according to claim 1, wherein R is a branched or unbranched, optionally substituted, saturated or unsaturated hydrocarbon chain which may be interrupted by heteroatoms, aromatics or heterocyclic compounds and comprises 2 to 2000 atoms.

3. The ligand-anchor conjugate according to claim 1 or 2, wherein R comprises a hydrophobic structural unit R¹ which is formed by a branched or unbranched hydrocarbon chain of 1 to 50 carbon atoms which may be saturated or partially unsaturated.

4. The ligand-anchor conjugate according to any of claims 1 to 3, wherein R comprises a branched or unbranched hydrophilic spacer R² which is formed by a hydrocarbon chain, which is interrupted by heteroatoms and comprises 2 to 1000 carbon atoms.

5. The ligand-anchor conjugate according to any of claims 1 to 4, wherein the structural element X comprises at least one element of main group V or VI of the periodic table.

6. The ligand-anchor conjugate according to claim 5, wherein X is a disulfide, thiol or sulphide group.

7. The ligand-anchor conjugate according to any of claims 1 to 6, wherein A is a hydroxyl, amino or carboxyl group.

8. The ligand-anchor conjugate according to any of claims 1 to 7, wherein the anchor molecule has the following general structure 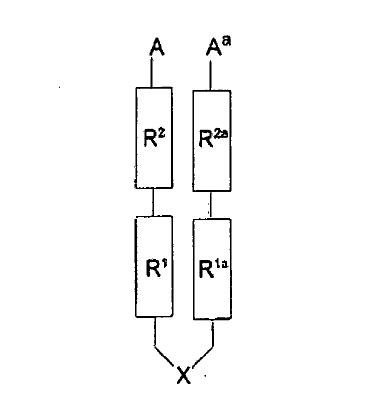 wherein R¹ and R^{1a} are independently defined as R¹ in claim 3;
R² and R^{2a} are independently defined as R² in claim 4;
the groups A and A^{a} are independently defined as A in claim 7; and
X is defined as in claim 6;
and wherein one or two structural units arbitrarily selected from R^{1a}, R^{2a} and A are optionally not present or in the combination of R^{1a}, R^{2a} and A^{a} may completely be replaced by a hydrogen atom.

9. The ligand-anchor conjugate according to any of claims 3 to 8, wherein R¹ and optionally R^{1a} have the structure -(CH₂)ₙ-, n being an integer from 1 to 50.

10. The ligand-anchor conjugate according to any of claims 4 to 9, wherein R² and optionally R^{2a} are independently an oligoamide and/or oligoether group.

11. The ligand-anchor conjugate according to any of claims 1 to 10, wherein the anchor molecule additionally comprises a functional group Y, which results from the immobilization of the anchor molecule on a solid phase.

12. The ligand-anchor conjugate according to claim 11, wherein Y is a carboxylic acid, carboxylic ester, carboxamide, aldehyde, hydrazide, hydroxamic acid, hydroxy, hydroxyalkyl or diketopiperazyl group.

13. The ligand-anchor conjugate according to any of claims 1 to 12, wherein the ligand is selected from proteins, peptides, oligonucleotides, carbohydrates, isoprenoids, enzymes, lipid structures, saccharides, antibodies, peptide hormones, cytokines, antibiotics or organic molecules having a molecular weight of ≥ 50 g/mol.

14. The ligand-anchor conjugate according to any of claims 1 to 13, wherein the anchor molecule is additionally bound to a non-ligand.

15. A method for the production of a ligand-anchor conjugate, comprising:
a) immobilization or synthesis of an anchor molecule on a solid phase which is suitable for chemical synthesis;
b) synthesis of a ligand on an anchor molecule or binding of a ligand to the anchor molecule; and
c) cleavage of the formed ligand-anchor conjugate form the solid phase,
wherein the anchor molecule comprises a structural unit which is capable of immobilizing the ligand-anchor conjugate on a surface, as well as a structural unit R which enables the formation of a self-assembled monolayer on the surface, and which is terminally functionalized for binding with the ligand,
and wherein the ligand should allow specific interaction of the surface with a receptor.

16. The method according to claim 15, wherein a multitude of different ligand-anchor conjugates is generated using combinatorial methods in the synthesis of the ligand.

17. The method according to claim 15 or 16, wherein the solid phase used for synthesis is a synthesis resin, a synthesis polymer film or a silicon or silicate surface.

18. The method according to claim 17, wherein the solid phase is a synthesis resin, selected from hydroxy resin, an amino resin, a trityl resin, a dihydropyrane resin, a carboxy resin or an arylsiloxy resin.

19. A method for providing a biospecific boundary layer on a surface, comprising the production of ligand-anchor conjugates according to any of claim 15 to 18 and additionally contacting the obtained ligand-anchor conjugates with the surface.

20. The method according to claim 19, wherein the surface is completely or partly formed of gold, silver, palladium or platin.

21. The method according to claim 19 or 20, wherein the surface has a multitude of position-addressable areas on which the ligand-anchor conjugates are immobilized.

22. The method according to claim 21, wherein the ligand-anchor conjugates form a molecular library in which the ligands used are different from area to area.

23. The use of ligand-anchor conjugates according to any of claims 1 to 14 for the provision of a biosensor.

## Revendications

1. Conjugué ligand-ancre, comprenant une molécule d'ancrage pour générer une couche limite biospécifique sur une surface, comprenant au moins une unité structurelle X, qui convient à la fixation de l'ancre sur la surface, ainsi qu'au moins une unité structurelle R, qui permet la formation d'une monocouche auto-assemblée sur la surface et qui est fonctionnalisée en position terminale par un groupement A pour former une liaison avec un ligand ou un non ligand, la molécule d'ancrage étant liée en position terminale à au moins un ligand qui est capable d'interactions spécifiques avec un récepteur.

2. Conjugué ligand-ancre selon la revendication 1, dans lequel R est une chaîne hydrocarbonée ramifiée ou non ramifiée, éventuellement substituée, saturée ou insaturée, qui peut être interrompue par des hétéroatomes, des aromatiques et des composés hétérocycliques et comprend 2 à 2000 atomes.

3. Conjugué ligand-ancre selon la revendication 1 ou 2, dans lequel R comprend une unité structurelle hydrophobe R¹, qui est formé par une chaîne hydrocarbonée, ramifiée ou non ramifiée, de 1 à 50 atomes de carbone, qui peut être saturée ou en partie insaturée.

4. Conjugué ligand-ancre selon l'une quelconque des revendications 1 à 3, dans lequel R comprend un espaceur hydrophile ramifié ou non ramifié R², qui est formé par une chaîne hydrocarbonée avec 2 à 1000 atomes de carbone interrompue par des hétéroatomes.

5. Conjugué ligand-ancre selon l'une quelconque des revendications 1 à 4, dans lequel l'élément structurel X comprend au moins un élément du groupe principal V ou VI du système périodique.

6. Conjugué ligand-ancre selon la revendication 5, dans lequel X est un groupement disulfure, thiol ou sulfure.

7. Conjugué ligand-ancre selon l'une quelconque des revendications 1 à 6, dans lequel A est un groupement hydroxyle, amino ou carboxyle.

8. Conjugué ligand-ancre selon l'une quelconque des revendications 1 à 7, dans lequel la molécule d'ancrage présente la structure générale suivante : 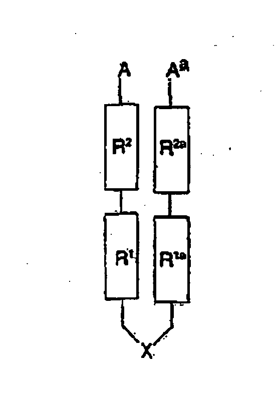 dans laquelle les radicaux R¹ et R^{1a} sont définis, indépendamment l'un de l'autre, comme R¹ dans la revendication 3, les radicaux R² et R^{2a} sont définis, indépendamment l'un de l'autre, comme R² dans la revendication 4, les groupements A et A^{a} sont définis respectivement, indépendamment l'un de l'autre, comme A dans la revendication 7 et X est défini comme dans la revendication 6, et dans laquelle une ou deux unités structurelles quelconques choisies parmi R^{1a}, R^{2a} et A^{a} n'est ou ne sont éventuellement pas présentes ou la combinaison de R^{1a}, R^{2a} et A^{a} peut être complètement substituée par un atome d'hydrogène.

9. Conjugué ligand-ancre selon l'une quelconque des revendications 3 à 8, dans lequel R¹ et éventuellement R^{1a} présentent la structure - (CH₂)ₙ- et n représente un nombre naturel de 1 à 50.

10. Conjugué ligand-ancre selon l'une quelconque des revendications 4 à 9, dans lequel R² et éventuellement R^{2A} représentent, indépendamment l'un de l'autre, un radical oligoamide et/ou oligoéther.

11. Conjugué ligand-ancre selon l'une quelconque des revendications 1 à 10, dans lequel la molécule d'ancrage comprend en plus un groupe fonctionnel Y qui provient de l'immobilisation de la molécule d'ancrage sur une phase solide.

12. Conjugué ligand-ancre selon la revendication 11, dans lequel Y est un radical acide carboxylique, ester d'acide carboxylique, amide d'acide carboxylique, aldéhyde, hydrazide, acide hydroxamique, hydroxyle, hydroxyalkyle ou dicétopipérazyle.

13. Conjugué ligand-ancre selon l'une quelconque des revendications 1 à 12, dans lequel le ligand est choisi parmi les protéines, les peptides, les oligonucléotides, les hydrates de carbone, les isoprénoïdes, les enzymes, les structures lipidiques, les saccharides, les anticorps, les hormones peptidiques, les cytokines, les antibiotiques ou les molécules organiques ayant un poids moléculaire ≥ 50 g/mole.

14. Conjugué ligand-ancre selon l'une quelconque des revendications 1 à 13, dans lequel la molécule d'ancrage est liée en plus à un non ligand.

15. Procédé de fabrication d'un conjugué ligand-ancre, comprenant :
a) l'immobilisation ou la synthèse d'une molécule d'ancrage sur une phase solide convenant à la synthèse chimique,
b) la synthèse d'un ligand sur la molécule d'ancrage ou la liaison d'un ligand sur la molécule d'ancrage, et
c) la séparation du conjugué ligand-ancre produit de la phase solide, la molécule d'ancrage présentant une unité structurelle qui convient à la fixation du conjugué ligand-ancre sur une surface, ainsi qu'une unité structurelle R, qui permet la formation d'une monocouche autoassemblée sur la surface et est fonctionnalisée en position terminale pour former une liaison avec le ligand, le ligand permettant des interactions spécifiques de la surface avec un récepteur.

16. Procédé selon la revendication 15, dans lequel une pluralité de différents conjugués ligand-ancre est produite par utilisation de procédés combinatoires lors de la synthèse du ligand.

17. Procédé selon la revendication 15 ou 16, dans lequel la phase solide utilisée pour la synthèse est une résine de synthèse, un film polymère de synthèse ou une surface de silicium ou de silicate.

18. Procédé selon la revendication 17, dans lequel la phase solide est une résine de synthèse, choisie parmi une résine hydroxy, une résine amino, une résine trityle, une résine dihydropyranne, une résine carboxyle ou une résine arylsiloxy.

19. Procédé de préparation d'une couche limite biospécifique sur une surface, comprenant la fabrication de conjugués ligand-ancre selon l'une quelconque des revendications 15 à 18 et, en plus, la mise en contact avec la surface des conjugués ligand-ancre obtenus.

20. Procédé selon la revendication 19, dans lequel la surface est formée complètement ou partiellement d'or, d'argent, de palladium ou de platine.

21. Procédé selon la revendication 19 ou 20, dans lequel la surface présente une pluralité de champs adressables en position, sur lesquels les conjugués ligand-ancre sont immobilisés.

22. Procédé selon la revendication 21, dans lequel les conjugués ligand-ancre forment une bibliothèque de molécules, dans laquelle les ligands utilisés se différencient de champ à champ.

23. Utilisation de conjugués ligand-ancre selon l'une quelconque des revendications 1 à 14 pour préparer un biocapteur.
